# EUROPÄISCHE PATENTSCHRIFT

(11) **EP 0 968 193 B1**
(45) Veröffentlichungstag und Bekanntmachung des Hinweises auf die Patenterteilung: **21.02.2007**
(21) Anmeldenummer: 98904053.0
(22) Anmeldetag: 10.01.1998
(51) Int. Cl.: C07D 239/54, C07D 239/47, C07D 239/42, C07D 251/46, C07D 251/52, C07D 251/42, C07D 403/12, C07D 401/12, C07D 417/12, C07D 405/12, A01N 47/36, C07C 311/47

(54) **PHENYLSULFONYLHARNSTOFFE, VERFAHREN ZU IHRER HERSTELLUNG UND IHRE VERWENDUNG ALS HERBIZIDE UND PFLANZENWACHSTUMSREGULATOREN**
PHENYLSULPHONYL UREAS, PROCESSES FOR THEIR PREPARATION AND THEIR USE AS HERBICIDES AND PLANT-GROWTH REGULATORS
UREES PHENYLSULFONIQUES, PROCEDES PERMETTANT DE LES PREPARER ET LEUR UTILISATION EN TANT QUE LES HERBICIDES ET LES REGULATEURS DE CROISSANCE VEGETALE

(30) Priorität: 23.01.1997 DE 19702200
(43) Veröffentlichungstag der Anmeldung: 05.01.2000
(73) Patentinhaber: Bayer CropScience GmbH, 65929 Frankfurt/Main (DE)
(72) Erfinder: LORENZ, Klaus, D-64331 Weiterstadt (DE); WILLMS, Lothar, D-65719 Hofheim (DE); BAUER, Klaus, D-63456 Hanau (DE); BIERINGER, Hermann, D-65817 Eppstein (DE); ROSINGER, Christopher, D-65719 Hofheim (DE)
(86) Internationale Anmeldenummer: PCT/EP1998/000116
(87) Internationale Veröffentlichungsnummer: WO 1998/032743

(56) Entgegenhaltungen:
- EP-A- 0 496 701
- WO-A-89/10921
- WO-A-94/10154
- DE-A- 4 335 297
- DE-A- 4 439 675
- DE-A- 19 616 445

## Beschreibung

Es ist bekannt, daß einige Phenylsulfonylharnstoffe herbizide und pflanzenwachstumsregulierende Eigenschaften besitzen; vgl. DE-A 4439675, EP-A 0496701, US-A-4,786,314, US-A-4,927,453, WO 89/10921, WO 94/10154, WO 95/10507 (= ZA 94/8063) und WO 97/40690. Diese weisen jedoch bei ihrer Anwendung zum Teil Nachteile auf, wie beispielsweise eine hohe Persistenz oder Unzureichende Selektivität in wichtigen Nutzpflanzenkulturen.

Es wurden nun neue Phenysulfonylharnstoffe mit speziellen Resten am Phenylring gefunden, die mit Vorteilen als Herbizide und Pflanzenwachstumsregulatoren eingesetzt werden können.

Gegenstand der vorliegenden Erfindung sind Verbindungen der Formel (I) oder deren Salze, 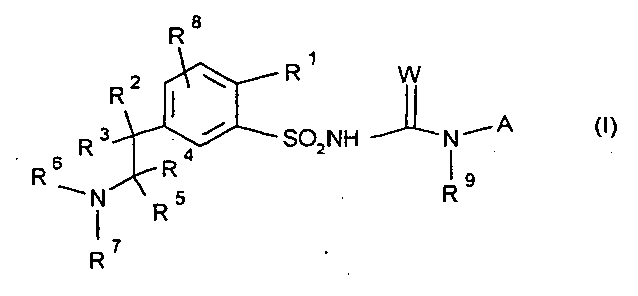 worin
- R¹: S(O)ₙ-R¹⁰ oder COQR¹¹,
- R², R³, R⁴; R⁵: unabhängig voneinander H oder (1-4)Alkyl,
- R⁶: H, OH, Formyl, (1-6)Alkyl, (2-6)Alkenyl, (2-4)Alkinyl, (1-6)Alkoxy, (2-6)Alkenyloxy, (2-6)Alkinyloxy, [(1-6)Alkyl]-carbonyl, [(2-6)Alkenyl]-carbonyl, [(2-6)Alkinyl]-carbonyl), (1-4)Alkyl-sulfonyl, (2-6)Alkenylsulfonyl, (2-6)Alkinylsulfonyl, (3-6)Cycloalkyl, (5-6)Cycloalkenyl, [(3-6)Cycloalkyl]-carbonyl, [(5-6)Cycloalkenyl]-carbonyl, [(3-6)Cycloalkyl]-sulfonyl, [(5-6)Cycloalkenyl]-sulfonyl, wobei jeder der 18 letztgenannten Reste unsubstituiert oder durch einen oder mehrere Reste aus der Gruppe Halogen, (1-4)Alkoxy, (1-4)Alkylthio, (1-4) Alkylsulfinyl, (1-4)Alkylsulfonyl, [(1-4)Alkoxy]carbonyl, [(1-4)Alkyl]carbonyl, [(1-4)Alkyl]carbonyloxy und CN und im Falle cyclischer Reste auch (1-4)Alkyl und (1-4)Haloalkyl substituiert ist,
oder
Phenylcarbonyl oder Phenylsulfonyl, wobei jeder der beiden letztgenannten Reste im Phenylring unsubstituiert oder durch einen oder mehrere Reste aus der Gruppe Halogen, CN, NO₂, (1-4)Alkyl, (1-4) Haloalkyl, (1-4)Alkoxy und (1-4)Haloalkoxy substituiert ist,
- R⁷: CHO, [(1-6)Alkyl]-carbonyl, [(2-6)Alkenyl]-carbonyl, [(2-6)Alkinyl]-carbonyl, (1-6)Alkylsulfonyl, (2-6)Alkenylsulfonyl, (2-6)Alkinylsulfonyl, [(3-6)Cycloalkyl]-carbonyl, [(5-6)Cycloalkenyl]-carbonyl, [(3-6)Cycloalkyl]suffonyl, (5-6)Cycloalkenylsulfonyl, wobei jeder der 10 letztgenannten Reste unsubstituiert oder durch einen oder mehrere Reste aus der Gruppe Halogen, (1-4)Alkoxy, (1-4)Alkylthio, (1-4)Alkylsulfonyl, (1-4)Alkylsulfinyl, (1-4)Alkylcarbonyl, [(1-4)Alkoxy]-carbonyl, [(1-4)Alkyl]-carbonyloxy und CN und im Falle cyclischer Reste auch (1-4)Alkyl und (1-4)Haloalkyl substituiert ist,
oder
Phenylcarbonyl oder Phenylsulfonyl, wobei jeder der beiden letztgenannten Reste im Phenylring unsubstituiert oder durch einen oder mehrere Reste aus der Gruppe Halogen, CN, NO₂, (1-4)Alkyl, (1-4)Haloalkyl, (1-4)Alkoxy und (1-4)Haloalkoxy substituiert ist,
oder
Mono- oder Di-[(1-4)alkyl]aminosulfonyl, das im Alkylteil unsubstituiert oder durch einen oder mehrere Reste aus der Gruppe Halogen, (1-4)Alkoxy, (1-4)Alkylthio, (1-4)Alkylsulfinyl, (1-4)Alkylsulfonyl, [(1 1-4)Alkyl]-carbonyl, [(1-4) Alkyl]-carbonyloxy, [(1-4)Alkoxy]-carbonyl und CN substituiert ist, oder eine Gruppe der Formel COCOR', worin R' = H, OH, (1-4)Alkoxy oder (1-4)Alkyl ist, oder eine Gruppe der Formel 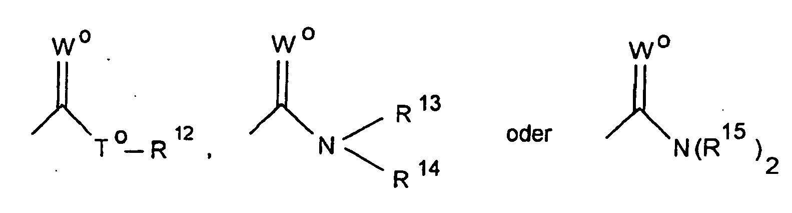 oder
- R⁶ und R⁷: gemeinsam eine Kette der Formel (-CH₂₂)ₘ₁ B¹- oder -B¹-(CH₂)ₘ₂B²-, wobei die Kette unsubstituiert oder durch einen oder mehrere (1-3)Alkylreste oder Halogen substituiert ist und m1 = 3, 4 oder 5 bzw. m2 = 2, 3 oder 4 sind, sowie
- W,W^{o}: ein Sauerstoffatom oder ein Schwefelatom,
- B¹,B²: unabhängig voneinander SO₂ oder CO,
- Q: O, S oder NR¹⁶,
- T^{o}: ein Sauerstoffatom oder ein Schwefelatom,
- R⁸: H, (1-4)Alkyl, (1-4)Alkoxy, (1-4)Alkylthio, [(1-4)Alkyl]carbonyl oder [(1-4)-Alkoxy]carbonyl, wobei jeder der letztgenannten 5 Reste unsubstituiert oder im Alkylteil durch ein oder mehrere Halogenatome substituiert ist, oder Halogen, NO₂, CN oder Mono- oder Di(1-4)alkylamino,
- R⁹: H oder CH₃,
- R¹⁰: NR¹⁷R¹⁸, (1-6)Alkyl, (2-6)Alkenyl, (2-6)Alkinyl, (3-6)Cycloalkyl, (5-6)Cycloalkenyl oder Phenyl, wobei jeder der letztgenannten 6 Reste unsubstituiert oder durch einen oder mehrere Reste aus der Gruppe Halogen, CN, (1-4)Alkoxy, (1-4)Alkylthio, (1-4)Alkylsulfinyl, (1-4)Alkylsulfonyl, [(1-4)Alkyl]-carbonyl, [(1-4)Alkoxy]-carbonyl und [(1-4)Alkyll-carbonyloxy substituiert ist,
- n: die Zahl 0, 1 oder 2, außer für R¹⁰ = NR¹⁷R¹⁸, wo n = 2 ist, sowie
- R¹¹: H, (1-6)Alkyl, (2-6)Alkenyl, (2-6)Alkinyl, (3-6)Cycloalkyl, (5-6)Cycloalkenyl oder Phenyl, wobei jeder der letztgenannten 6 Reste unsubstituiert oder durch einen oder mehrere Reste aus der Gruppe Halogen, CN, (1-4)Alkoxy, (1-4)Alkylthio, (1-4)Alkylsulfinyl, (1-4)Alkylsulfonyl, [(1-4)Alkyl] carbonyl, [(1-4)Alkoxy] carbonyl und [(1-4)Alkyl]carbonyloxy und im Falle cyclischer Reste auch (1-4)Alkyl und (1-4)Haloalkyl substituiert ist, oder einen Rest des Typs Heterocyclyl oder Heterocyclyl(1-4)alkyl mit 3-7 Ringatomen, vorzugsweise mit 1 bis 3 Heteroringatomen aus der Gruppe N, O und S, insbesondere einen Rest der Formel A-1 bis A-6, 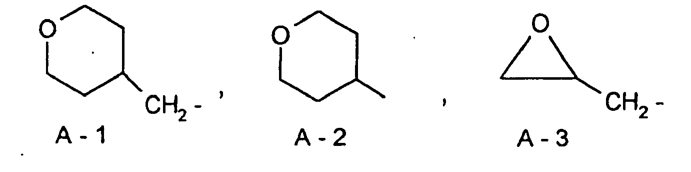 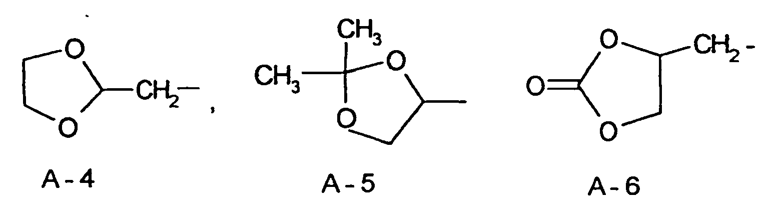
- R¹²: (1-4)Alkyl, (3-4)Alkenyl oder (3-4)Alkinyl, wobei jeder der drei letztgenannten Reste unsubstituiert oder durch einen oder mehrere Reste aus der Gruppe-Halogen, (1-4)Alkoxy, (1-4)Alkylthio, [(1-4)Alkyl]-carbonyl und [(1-4)Alkoxy]-carbonyl substituiert ist,
- R¹³, R¹⁴: unabhängig voneinander H, (1-4)Alkyl, (3-4)Alkenyl oder (3-4)Alkinyl, wobei jeder drei letztgenannten Reste unsubstituiert oder durch einen oder mehrere der Reste aus der Gruppe Halogen, (1-4)Alkoxy, (1-4)Alkylthio, [(1-4)Alkyl]-carbonyl und [(1-4)Alkoxy]-carbonyl substituiert ist,
- die Reste R¹⁵: gemeinsam mit dem N-Atom einen heterocyclischen Ring mit 5 oder 6 Ringgliedern, der weitere Heteroatome aus der Gruppe N, O und S in den möglichen Oxidationsstufen enthalten kann und unsubstituiert oder durch (1-4)-Alkyl oder die Oxogruppe substituiert ist oder benzokondensiert ist,
- R¹⁶: H, (1-4)Alkyl, (3-4)Alkenyl oder (3-4)Alkinyl, wobei jeder der drei letztgenannten Reste unsubstituiert oder durch einen oder mehrere Reste aus der Gruppe Halogen, (1-4)Alkoxy und (1-4)Alkylthio substituiert ist,
- R¹⁷: H, (1-4)Alkyl oder (1-4)Alkoxy,
- R¹⁸: H oder (1-4)Alkyl
- A: einen Rest der Formel 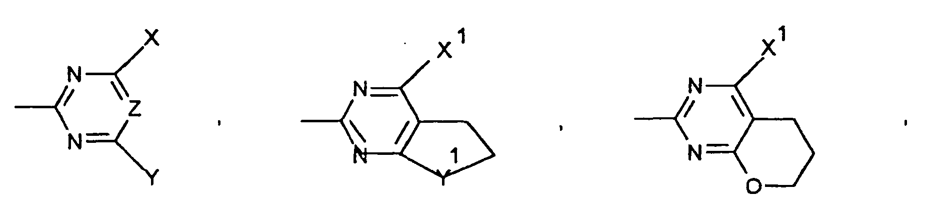 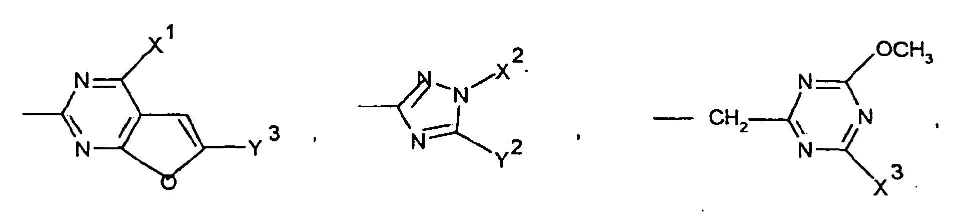 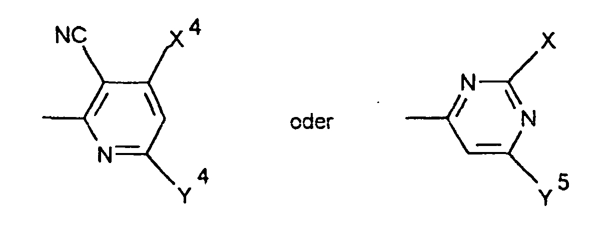 einer der Reste X und Y Wasserstoff, Halogen, (1-3)Alkyl oder (1-3)Alkoxy, wobei jeder der beiden letztgenannten Reste unsubstituiert oder durch einen oder mehrere Reste aus der Gruppe Halogen, (1-3)Alkoxy und (1-3)Alkylthio substituiert ist, und
der andere der Reste X und Y Wasserstoff, Halogen, (1-3)Alkyl, (1-3)Alkoxy oder (1-3)Alkylthio, wobei jeder der drei letztgenannten alkylhaltigen Reste unsubstituiert oder durch einen oder mehreren Reste aus der Gruppe Halogen, (1-3)Alkoxy und (1-3)Alkylthio substituiert ist, oder einen Rest der Formel NR^{a}R^{b}, (3-6)Cycloalkyl, (2-4)Alkenyl, (2-4)Alkinyl, (3-4)-Alkenyloxy oder (3-4)Alkinyloxy,
- Z: CH oder N,
- R^{a} und R^{b}: unabhängig voneinander H, (1-4)Alkyl oder (2-4)Alkenyl,
- X¹: CH₃, OCH₃, OC₂H₅ oder OCHF₂,
- Y¹: -O- oder -CH₂-,
- X²: CH₃, C₂H₅ oder CH₂CF₃,
- **Y²**: OCH₃, OC₂H₅, SCH₃, SCH₂CH₃, CH₃ oder C₂H₅,
- X³: CH₃ oder OCH₃,
- Y³: H oder CH₃,
- X⁴: CH₃, OCH₃, OC₂H₅, CH₂OCH₃ oder Cl,
- Y⁴: CH₃, OCH₃, OC₂H₅ oder Cl und
- Y⁵: CH₃, C₂H₅, OCH₃ oder Cl
bedeuten.

Definitionen für allgemeine Reste mit Kohlenstoffatomen in Formel (I) enthalten oft Bereiche oder Einzelangaben für die Anzahl der möglichen Kohlenstoffatome. Die Bereichsangabe bzw. Zahl für die C-Atome wird der Bezeichnung für die allgemeine chemische Gruppe in Klammern vorangestellt; so bedeutet z.B. (1-4)Alkyl einen Alkylrest mit 1 bis 4 C-Atomen; oder (1-4)Haloalkyl bedeutet Haloalkyl mit 1 bis 4 C-Atomen im Alkylteil oder Alkylgerüst; (1)Alkyl ist gleichbedeutend mit Methyl; unter die allgemeine Definition unsubstituiertes (3)Alkyl fallen demnach n-Propyl und i-Propyl.

Die Verbindungen der Formel (I) können Salze bilden, bei denen der Wasserstoff der -SO₂-NH-Gruppe durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze oder Salze mit organischen Aminen. Ebenso kann Salzbildung durch Anlagerung einer Säure an basischen Gruppen, wie z.B. Amino und Alkylamino, erfolgen. Geeignete Säuren hierfür sind starke anorganische und organische Säuren, beispielsweise HCl, HBr, H₂SO₄ oder HNO₃.

In Formel (I) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Cycloalkyl bedeutet ein carbocyclisches, gesättigtes Ringsystem, z.B. mit 3-8 C-Atomen, z.B. Cyclopropyl, Cyclopentyl oder Cyclohexyl.

Alkenyl in der Form "(3-4)Alkenyl" oder "(3-6)Alkenyl" bedeutet vorzugsweise einen Alkenylrest mit 3 bis 4 bzw. 3 bis 6 C-Atomen, bei dem die Doppelbindung nicht an dem C-Atom liegt, das mit dem übrigen Molekülteil der Verbindung (I) verbunden ist ("yl"-Position). Entsprechendes gilt für (3-4)Alkinyl usw.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl; Aryl bedeutet dabei ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl;
vorzugsweise bedeutet ein Kohlenwasserstoffrest Alkyl, Alkenyl oder Alkinyl mit bis zu 12 C-Atomen oder Cycloalkyl mit 3, 4, 5, 6 oder 7 Ringatomen oder Phenyl; entsprechendes gilt für einen Kohlenwasserstoffrest in einem Kohlenwasserstoffoxyrest.

Ein heterocyclischer Rest oder Ring (Heterocyclyl) kann gesättigt, ungesättigt oder heteroaromatisch sein; er enthält vorzugsweise ein oder mehrere Heteroatome im Ring, vorzugsweise aus der Gruppe N, O und S; vorzugsweise ist er ein aliphatischer Heterocyclylrest mit 3 bis 7 Ringatomen oder ein heteroaromatischer Rest mit 5 oder 6 Ringatomen und enthält 1, 2 oder 3 Heteroatome. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, oder ist ein partiell oder vollständig hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heterocyclyl oder Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (1-4)Alkyl, vorzugsweise Methyl oder Ethyl, (1-4)Haloalkyl, vorzugsweise Trifluormethyl, (1-4)Alkoxy, vorzugsweise Methoxy oder Ethoxy, (1-4)Haloalkoxy, Nitro und Cyano. Besonders bevorzugt sind dabei die Substituenten Methyl, Methoxy und Chlor.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (1-4)Alkanoyl. Entsprechenes gilt für substituiertes Hydroxylamino oder Hydrazino.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (1-4)Alkyl, (1-4)Alkoxy, (1-4)Halogenalkyl, (1-4)Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, mund p-Methoxyphenyl.

Ein Acylrest bedeutet den Rest einer organischen Säure, z.B. den Rest einer Carbonsäure und Reste davon abgeleiteter Säuren wie der Thiocarbonsäure, gegebenenfalls N-substituierten lminocarbonsäuren oder den Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie [(1-4)-Alkyl]-carbonyl, Phenylcarbonyl, wobei der Phenylring substituiert sein kann, z.B. wie oben für Phenyl gezeigt, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die vorstehenden Beispiele für Reste oder Restebereiche, die unter die allgemeinen Begriffe wie "Alkyl", "Acyl", "substituierte Reste" etc. fallen, bedeuten keine vollständige Aufzählung. Die allgemeinen Begriffe umfassen insbesondere auch die weiter unten angeführten Definitionen für Restebereiche in Gruppen bevorzugter Verbindungen, insbesondere Restebereiche, welche spezifische Reste aus den Tabellenbeispielen umfassen.

Vor allem aus Gründen der höheren herbiziden Wirkung, besseren Selektivität und/oder besseren Herstellbarkeit sind erfindungsgemäße Verbindungen der Formel (1) oder deren Salze von besonderem Interesse, worin
- R¹: S(O)ₙ-R¹⁰ oder CO-OR¹¹,
- n: die Zahl 0, 1 oder2, außer im Fall R¹⁰ = NR¹⁷R¹⁸, für den n = 2 ist,
- R⁶: H oder (1-4)Alkyl, das unsubstituiert oder durch einen oder mehrere Halogenatome oder durch einen oder mehrere Reste aus der Gruppe (1-4)Alkoxy und (1-4)Alkylthio substituiert ist,
- R⁷: Formyl, [(1-6)Alkyl]-carbonyl, [(2-4)Alkenyl]-carbonyl, [(2-4)Alkinyl]-carbonyl, [(3-6)Cycloalkyl]-carbonyl oder (1-6)Alkylsutfonyl, wobei jeder der 5 letztgenannten Reste unsubstituiert oder durch einen oder mehrere Reste aus der Gruppe Halogen, (1-4)Alkoxy, (1-4)Alkylthio, (1-4)Alkylsulfonyl, [(1-4)Alkyl]-carbonyl, [(1-4)Alkoxy]-carbonyl, [(1-4)Alkyl]-carbonyloxy und CN und im Fall cyclischer Reste auch (1-4)Alkyl und (1-4)Haloalkyl substituiert ist, oder
Phenylcarbonyl oder Phenylsulfonyl, wobei jeder der beiden letztgenannten Reste unsubstituiert oder durch einen oder mehrere Reste aus der Gruppe Halogen, CN, SO₂, (1-4)Alkyl, (1-4)Haloalkyl, (1-4)Alkoxy und (1-4)Haloalkoxy substituiert ist, oder
Mono- oder Di-[(1-4)-alkyl]-aminosulfonyl, oder eine Gruppe der Formel -CO-CO-R', worin R' (1-4)Alkoxy bedeutet, oder
eine Gruppe der Formel -CW^{o}-R¹², -CW^{o}-NR¹³R¹⁴ oder -CW^{o}-N(R¹⁵)₂ oder
- R⁶, R⁷: gemeinsam eine Kette der Formel (-CH₂)ₘ₁ B¹- oder -B¹-(CH₂)ₘ₂B², wobei m1 = 3, 4 oder 5 und m2 = 2, 3 oder 4 bedeuten, und
- W, W^{o}: jeweils unabhängig ein Sauerstoff- oder Schwefelatom,
- T^{o}: ein Sauerstoff- oder Schwefelatom,
- B¹: SO₂ oder CO,
- B²: SO₂ oder CO,
- Q: O, S oder NR¹⁶,
- R⁸: ein Wasserstoffatom,
- R⁹: H oder CH₃,
- R¹⁰: NR¹⁷R¹⁸, (1-6)Alkyl oder (3-6)Cycloalkyl,
- R¹¹: H, (1-6)Alkyl, (3-6)Cycloalkyl oder einen Rest der Formeln A-1 bis A-6, 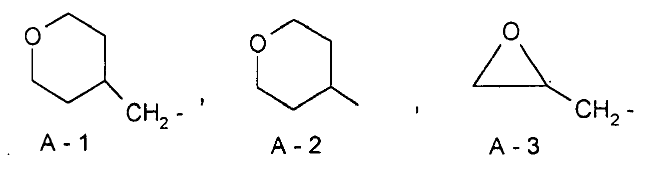 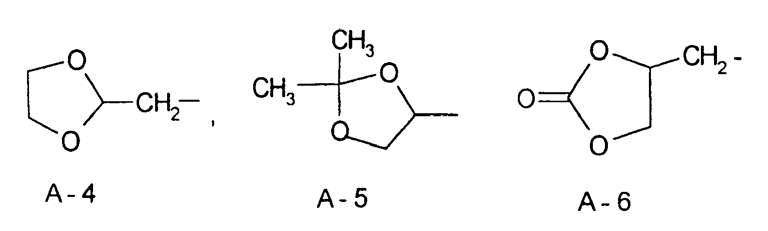
- R¹²: (1-4)Alkyl oder (1-4)Haloalkyl,
- R¹³, R¹⁴: unabhängig voneinander H oder (1-4)Alkyl,
- die Reste R¹⁵: gemeinsam eine divalente Kette der Formel -(CH₂)ₘ₃-, worin m3 = 3, 4 oder 5 ist, oder der Formel -CH₂CH₂-O-CH₂CH₂-,
- R¹⁶: H oder (1-4)Alkyl,
- R¹⁷: H oder (1-4)Alkyl und
- R¹⁸: H oder (1-4)Alkyl
bedeuten.

Bevorzugt sind erfindungsgemäße Verbindungen der Formel (I) und deren Salze, worin
- R⁶: H oder (1-4)Alkyl,
- R⁷: CHO, [(1-6)Alkyl]-carbonyl, [(1-4)Haloalkyl]-carbonyl, [(1-4)Alkoxy-(1-4)alkyl]-carbonyl, [(3-6)Cycloalkyl]-carbonyl, Phenylcarbonyl, das unsubstituiert oder durch einen oder mehrere Reste aus der Gruppe Halogen, CN, NO₂, (1-4)Alkyl, (1-4)Haloalkyl, (1-4)Alkoxy und (1-4)Haloalkoxy substituiert ist, oder Phenylsulfonyl, das unsubstituiert oder durch einen oder mehrere Reste aus der Gruppe (1-4)Alkyl und (1-4)Alkoxy substituiert ist, oder Mono- oder Di-[(1-4)alkyl]-aminosulfonyl, (1-6)Alkylsulfonyl, (1-4)Haloalkylsulfonyl oder eine Gruppe der Formel -CW^{o}-R¹² oder -CW^{o}-NR¹³R¹⁴
- W, W^{o}: jeweils unabhängig voneinander O oder S,
- T°: O oder S,
- Q: 0, S oder NR¹⁶,
- R¹⁰: NR¹⁷R¹⁸, (1-4)Alkyl oder (3-6)Cycloalkyl,
- R¹¹: H oder (1-4)Alkyl,
- R¹²: (1-4)Alkyl,
- R¹³, R¹⁴: unabhängig voneinander H oder (1-4)Alkyl,
- R¹⁶: H oder (1-3)Alkyl,
- R¹⁷: (1-4)Alkyl und
- R¹⁸: H oder (1-4)Alkyl
bedeuten.

Bevorzugt sind auch erfindungsgemäße Verbindungen der Formel (I) und deren Salze, welche eine Kombination von Resten aus den obengenannten Verbindungen von besonderem Interesse bzw. den bevorzugten Verbindungen enthalten, sowie solche, welche einzelne oder mehrere Reste aus den in den Tabellen 1 oder 2 (s.u.) aufgeführten Verbindungen enthalten.

Gegenstand der vorliegenden Erfindung sind auch Verfahren zur Herstellung der Verbindungen der allgemeinen Formel (I) oder deren Salze, dadurch gekennzeichnet, daß man
a) eine Verbindung der Formel (II) 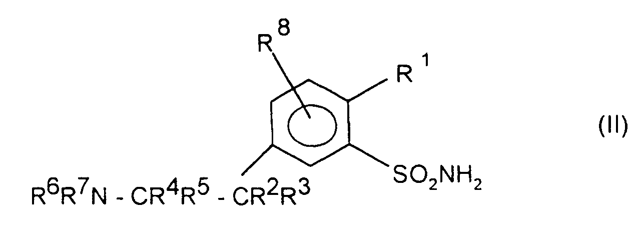 mit einem heterocyclischen Carbamat der Formel (III),

   R* - O - CO - NR⁹ - A (III)

   worin R* gegebenenfalls substituiertes Phenyl oder (C₁-C₄)Alkyl bedeutet, umsetzt oder
b) ein Arylsulfonylcarbamat der Formel (IV) 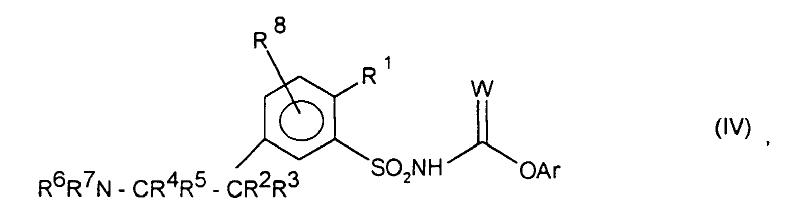 worin Ar einen Arylrest, vorzugsweise gegebenenfalls substituiertes Phenyl, bedeutet, mit einem Aminoheterocyclus der Formel (V)

   H-NR⁹-A (V)

   umsetzt oder
c) ein Sulfonylisocyanat der Formel (VI) 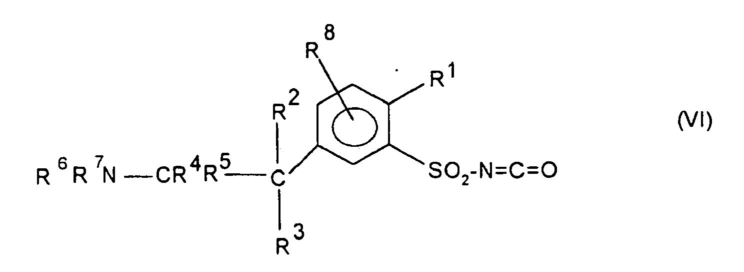 mit einem Aminoheterocyclus der Formel H-NR⁹-A (V) umsetzt oder
d) in einer Eintopfreaktion zunächst einen Aminoheterocyclus der Formel H-NR⁹-A (V) in Gegenwart einer Base mit Phosgen umsetzt und das gebildete Intermediat mit einem Phenylsulfonamid der Formel (II) umsetzt oder
e) ein Sulfonylchlorid der Formel (VII) 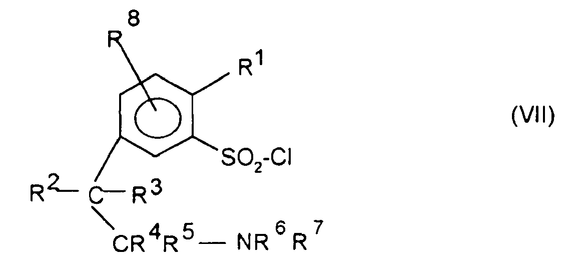 mit einem Cyanat M-OCN, worin M = ein Kation, z.B. NH₄, Na oder K bedeutet, und mit einem Aminoheterocyclus der Formel H-NR⁹-A (V) in Gegenwart einer Base umsetzt, oder
f) ein Sulfonamid der genannten Formel (II) mit einem (Thio-)Isocyanat der Formel (V')

   W = C = N - A (V')

   in Gegenwart einer Base umsetzt,
wobei in den Formeln (II)-(VII) und (V') die Reste bzw. Symbole R¹-R⁹, A, W und n wie in Formel (I) definiert sind und wobei in den Varianten a) und c)-e) zunächst Verbindungen der Formel (I) mit W = 0 erhalten werden.

Die Umsetzung der Verbindungen der Formeln (11) und (III) erfolgt vorzugsweise basenkatalysiert in einem inerten organischen Lösungsmittel, wie z.B. Dichlormethan, Acetonitril, Dioxan oder THF bei Temperaturen zwischen 0°C und dem Siedepunkt des Lösungsmittels. Als Basen werden dabei beispielsweise organische Aminbasen, wie 1,8-Diazabicyclo[5.4.0]undec-7-en (DBU), insbesondere bei R* = (subst.) Phenyl (vgl. EP-A-44807), oder Trimethylaluminium oder Triethylaluminium, letztere insbesondere bei R* = Alkyl (vgl. EP-A-166516) verwendet.

Die Sulfonamide (II) sind neue Verbindungen. Sie und ihre Herstellung sind ebenfalls Gegenstand dieser Erfindung.

Am Beispiel der Verbindungen (11) mit R² bis R⁵ = H und R¹ = COOR¹¹ mit Q = O werden im folgenden Herstellungsmöglichkeiten näher erläutert, die mit geringfügigen Modifikationen auch für Verbindungen mit R²-R⁵ ungleich Wasserstoff bzw. mit R¹ wie oben beschrieben eingesetzt werden können.

Ausgehend von gegebenenfalls substituierten Benzylhalogeniden (VIII) (siehe Schema 1; vgl. WO 95/10507) können Benzylcyanide (IX) durch nucleophilen Austausch des Halogenids mit Cyanid gewonnen werden. Nach Reduktion oder Hydrogenolyse von (IX) und eventuell nachfolgender Funktionalisierung der Aminogruppe, z.B. durch Alkylierung, werden Phenethylamine (X) bzw. Phenethylamine (X1) erhalten, worin R¹ = COR¹¹, R² = R³ = R⁴ = R⁵ = R⁷ = H bedeuten, welche gegebenenfalls nach weiterer Funktionalisierung, z.B. durch Acylierung, sowie Abspaltung der tert. Butylgruppe analog bekannter Verfahren (z.B. mit CF₃COOH) zu den Sulfonamiden (II') umgesetzt werden (siehe Schema 1).

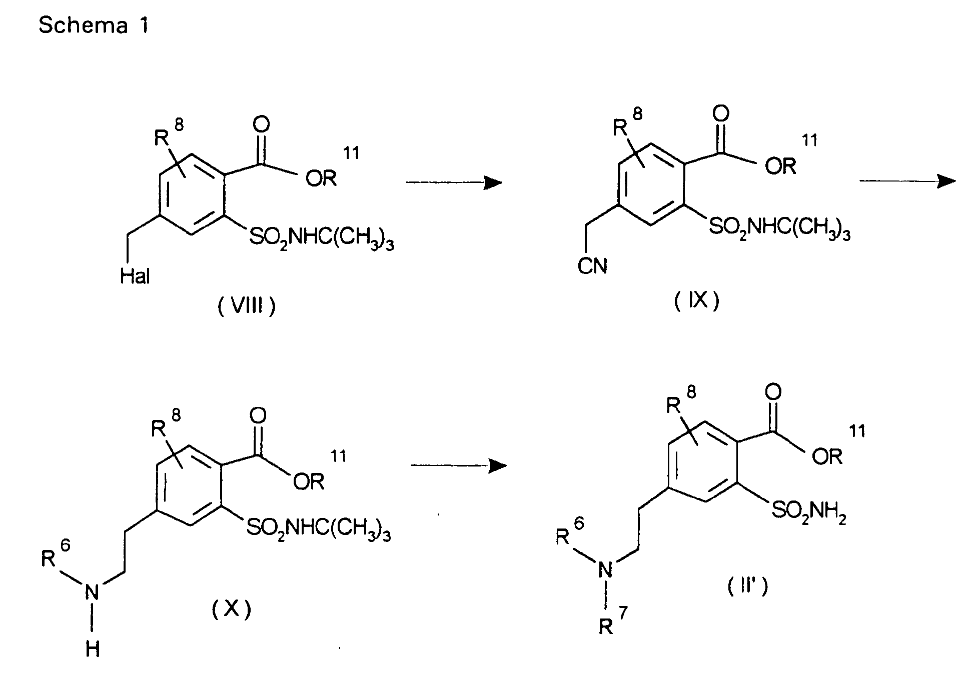

Die Verfahrensweise läßt sich analog auch zur Herstellung anderer Verbindungen der Formel (II) einsetzen, wobei im letzten Schritt die Verbindung der Formel (XI) 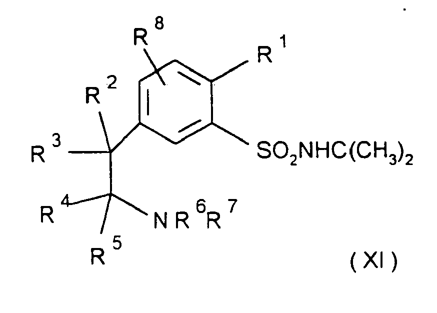 eingesetzt wird.

Die Carbamate der Formel (III) können nach Methoden hergestellt werden, die in den südafrikanischen Patentanmeldungen 82/5671 und 82/5045 bzw.
EP-A-70804 (US-A-4 480 101) oder RD 275056 beschrieben sind.

Die Umsetzung der Verbindungen (IV) mit den Aminoheterocyclen (V) führt man vorzugsweise in inerten, aprotischen Lösungsmitteln wie z.B. Dioxan, Acetonitril oder Tetrahydrofuran bei Temperaturen zwischen 0°C und der Siedetemperatur des Lösungsmittels durch. Die benötigten Ausgangsmaterialien (V) sind literaturbekannt oder können nach literaturbekannten Verfahren hergestellt werden. Die Arylsulfonylcarbamate der Formel (IV) erhält man analog US-PS 4 684 393 oder US-PS-4 743 290.

Die Aryl- bzw. Phenylsulfonylisocyanate der Formel (Vl) lassen sich analog US-PS-4 481 029 herstellen und mit den Aminoheterocyclen (V) umsetzen.

Die Phosgenierung von Verbindungen der Formel (V) nach Variante d) kann vorzugsweise in Gegenwart von Basen, wie sterisch gehinderten organischen Aminbasen, beispielsweise Triethylamin, erfolgen. Die anschließende Umsetzung mit Verbindungen der Formel (II) nach Variante d) kann analog bekannten Verfahren durchgeführt werden (vgl. EP-A-232 067).

Die Sulfochloride (Vll) können aus entsprechenden Sulfonsäuren erhalten werden, z.B. nach Standardmethoden wie der Umsetzung des Kaliumsalzes mit Phoshoroxychlorid oder Thionylchlorid in inerten Solventien wie Acetonitril und/oder Sulfolan oder in Substanz durch Erwärmen zum Rückfluß (vgl. Houben-Weyl-Klamann, "Methoden der organischen Chemie", 4. Aufl. Bd. 3 XI/2, S. 1067-1073, Thieme Verlag Stuttgart, 1985).

Die entsprechenden Sulfonsäuren sind aus entsprechenden Nitroverbindungen analog der Umsetzung von Verbindungen (Xl) erhältlich.

Alternativ sind in Einzelfällen Sulfochloride (VII) durch Sulfonierung (+Chlorierung) oder Sulfochlorierung geeigneter substituierter Benzoesäureester herstellbar; Sulfochlorierung analog Houben-Weyl-Klamann, "Methoden der organischen Chemie", 4. Aufl. Bd. E XI/2, S. 1067 ff., Thieme Verlag Stuttgart, 1985; Houben-Weyl-Müller, "Methoden der organischen Chemie", 4. Aufl. Bd. IX, s. 563ff., Thieme Verlag Stuttgart, 1955; Sulfonierung analog Houben-Weyl-Klamann, "Methoden der organischen Chemie, 4. Aufl. Bd. E XI/2, S.1055ff., Thieme Verlag Stuttgart, 1985; Houben-Weyl-Müller, "Methoden der organischen Chemie", 4. Aufl. Bd. IX, S. 435 ff., Thieme Verlag Stuttgart, 1955.

Die (Thio)-Isocyanate der Formel (V') sind nach literaturbekannten Verfahren erhältlich (EP-A-232067, EP-A-166516). Die Umsetzung der (Thio)-Isocyanate (V') mit Verbindungen (II) erfolgt beispielsweise bei -10°C bis 100°C, vorzugsweise 20 bis 100°C, in einem inerten aprotischen Lösungsmittel, wie z.B. Aceton oder Acetonitril, in Gegenwart einer geeigneten Base, z.B. N(C₂H₅)₃ oder K₂CO₃.

Die Salze der Verbindungen der Formel (I) werden vorzugsweise in inerten polaren Lösungsmitteln wie z.B. Wasser, Methanol oder Aceton bei Temperaturen von 0-100°C hergestellt. Geeignete Basen zur Herstellung der erfindungsgemäßen Salze sind beispielsweise Alkalicarbonate, wie Kaliumcarbonat, Alkali- und Erdalalkalihydroxide, z.B. NaOH oder KOH, oder Ammoniak oder Ethanolamin.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.
Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten..

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. lnc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'- disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden:
Alkylarylsulfonsaure Calzium-Salze wie
Ca-dodecylbenzolsulfonat oder nichtionische Emulgatoren wie
Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B.
Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrig organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.
Zur Herstellung von Teller-, Fließbett-, Extruder- und Sprühgranulate siehe z.B.

Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie aus z.B. aus Weed Research 26, 441-445 (1986), oder "The Pesticide Manual", 10th edition, The British Crop Protection Council and the Royal Soc. of Chemistry, 1994 und dort zitierter Literatur beschrieben sind. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1 H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4, 5-dihydro-5-oxo-1 H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazamethabenz-methyl; imazapyr; imazaquin und Salze wie das Ammoniumsalz; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1 H-1,2,4-triazol-1-carboxamid; thenylchlor (NSK-850); thiazafluron; thiazopyr (Mon-13200); thidiazimin (SN-24085); thifensulfuron-methyl; thiobencarb; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1 H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (l). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### A. Chemische Beispiele

- Abkürzungen:: Die Mengenangaben wie Prozent-Angaben und Mengenverhältnisse beziehen sich auf das Gewicht, wenn nicht näher spezifiziert, i.Vak. bedeutet unter reduzierten Druck,
h = Stunde(n)

### Beispiel A 1

### 2-(tert.-Butylaminosulfonyl)-4-cyanomethylbenzoesäuremethylester

Ein Zweiphasengemisch von 100 g 61,5 %igem 2-(tert.-Butylaminosulfonyl)-4-brommethylbenzoesäuremethylester (61,5 g = 0,169 mol), 12.09 g (0,186 mol) Kaliumcyanid und 10,89 g (33,8 mmol) Tetrabutylammoniumbromid in 750ml Dichlormethan und 150 ml Wasser wurde 6 h bei Raumtemperatur gerührt.
Zur Aufarbeitung wurde der Ansatz mit Wasser verdünnt, die Phasen getrennt und die wäßrige Phase mit Dichlormethan zweimal nachextrahiert. Die vereinigten organischen Extrakte wurden über Na₂SO₄ getrocknet. Das nach Einengen erhaltene Rohprodukt wurde chromatographisch mit einem Essigester/Petrolethergemisch (1:2, v/v) an Kieselgel aufgetrennt. Einengen der Fraktionen mit R_{F}= 0.18 lieferte 48,1 g (91,8 %) 2-(tert.-Butylaminosulfonyl)-4-cyanomethylbenzoesäuremethyl-ester vom Schmp.: 86-88°C.

### Beispiel A2

### 4-(2-Aminoethyl)-2-tert.-butylaminosulfonylbenzoesäuremethylester

Eine Lösung von 2,5 g (8,05 mmol) 2-(tert.-Butylaminosulfonyl)-4-cyanomethylbenzoesäuremethylester in 200 ml Methanol und 4 ml konz. HCI wurde nach Zugabe von 0,5 g Platinoxidhydrat 10 h bei Raumtemperatur und 20 bar Wasserstoffdruck hydriert. Zur Aufarbeitung wurde vom Katalysator abfiltriert und das Filtrat eingeengt. Nach Aufnahme des Rückstandes in Essigester wurde die Lösung viermal mit 2 N HCl gewaschen. Die vereinigten sauren wäßrigen Phasen werden mit konz. Ammoniak auf pH 10-11 eingestellt und mit Essigester dreimal extrahiert. Die organischen Extrakte wurden über Na₂SO₄ getrocknet und i. Vak. eingeengt. Man erhielt 1,9 g (75 %) 4-(2-Aminoethyl)-2-tert.-butylaminosulfonyl-benzoesäuremethylester als Öl.
- ¹H-NMR (300 MHz, CDCl₃): δ:: 1,26 (s, 9H, C(CH₂)₃); 1,70 (bs, 2H, NH₂); 2,88 und 3,02 (2m, je 2H, Ar-CH₂CH₂₋N); 3,96 (s, 3H, OCH₃); 6,04 (bs, 1 H, SO₂NH); 7,42 (dd, 1 H, Ar-H, J = 2Hz, 8 Hz); 7,77 (d, 1 H, Ar-H, J = 8 Hz); 8,00 (d, 1 H, Ar-H,J = 2Hz).

### Beispiel A3

### 2 -tert.-Butylaminosulfonyl-4- [2-(methoxycarbonylamino)ethyl]benzoesäuremethylester

Zu einer eisgekühlten Lösung von 1,31 g (13,8 mmol) Chlorameisensäuremethylester in 20 ml Dichlormethan tropfte man eine Lösung von 1,45 g (4,6 mmol) 4-(2-Aminoethyl)-2-tert.-butylaminosulfonylbenzoesäuremethylester und 0,71 ml (5,1 mmol) Triethylamin in 10 ml Dichlormethan. Nach 2 h bei Raumtemperatur wurde mit Wasser versetzt, die Phasen getrennt und die wäßrige Phase mit Dichlormethan zweimal nachextrahiert. Die vereinigten organischen Extrakte wurden mit Wasser gewaschen, über Na₂SO₄ getrocknet und i.Vak. eingeengt. Man erhielt 1,4 g (81 %) 2-tert.-Butylaminosulfonyl-4-[2-(methoxycarbonylamino)ethyl]benzoesäuremethylester als zähes Öl.
- ¹H-NMR (300 MHz, CDCl₃): δ =: 1,24 (s, 9H, C(CH₃)₃), 2,95 (m, 2H, Ar-CH₂-CH₂N); 3,45 (m, 2H, Ar-CH₂CH₂N); 3,65 (s, 3H, OCH₃); 3,95 (s,3H, OCH₃); 4,70 (bs, 1 H, CONH); 6,20 (s, 1 H, SO₂NH); 7,40 (bd, 1 H, Ar-H, J = 8Hz); 7,80 (d, 1 H, Ar-H, J = 8Hz); 8,00 (bs, 1 H, Ar-H).

### Beispiel A4

### 2-Aminosulfonyl-4-[2-(methoxycarbonyfamino)ethyl]benzoesäuremethylester

Eine Lösung von 1,4 g (3,8 mmol) 2-tert.-Butylaminosulfonyl-4-[2-(methoxycarbonylamino]ethyl]benzoesäuremethylester in 20 ml Trifluoressigsäure wurde 8 h bei Raumtemperatur gerührt. Der Ansatz wurde vollständig eingeengt, mit Toluol nachgedampft und der erhaltene Rückstand mit Essigester/Diisopropylether kristallisiert. Man erhielt 0,54 g (45 %) 2-Aminosulfonyl-4-[2-(methoxycarbonylamino)-ethyl]benzoesäuremethylester vom Schmp.: 146-149°C.

### Beispiel A5

### 2-[(4,6-Dimethoxypyrimidin-2-yl)aminocarbonylaminosulfonyl]-4-[2-(methoxycarbonylamino)ethyl]benzoesäuremethylester

Zu einer Suspension von 0,54 g (1,7 mmol) 2-Aminosulfonyl-4-[2-(methoxycarbonylamino)ethyl]benzoesäuremethylester und 0,47 g (1,7 mmol) N-(4,6-Dimethoxypyrimidin-2-yl)phenylcarbamat in 15 ml Acetonitril gab man tropfenweise 0,26 ml (1,7 mmol) DBU. Nach 2 h wurde mit Wasser und Diethylether verdünnt, mit HCl auf pH 1-2 angesäuert und das ausgefallene Produkt abfiltriert, mit Wasser und Diethylether nachgewaschen und getrocknet. Ausbeute: 0,47 g (55,6 %). 2-[(4,6-Dimethoxypyrimidin-2-yl)aminocarbonylaminosulfonyl]-4-[2-(methoxycarbonylamino)ethyl]benzoesäuremethylester vom Schmp.: 165-169°C/Zers.

Die Verbindungen aus den folgenden Tabellen 1 und 2 werden in analoger Weise zu den Beispielen A1 bis A5 und den obengenannten chemischen Verfahren oder allgemein bekannten Verfahren erhalten.

Abkürzungen und Erläuterungen zu den Tabellen 1 und 2:
- Nr. =: Beispielnummer, Beispielverbindung Nr.
- Me =: CH₃ = Methyl
- Et =: C₂H₅ = Ethyl
- Pr =: n-Pr = nPr = n-C₃H₇ = n-Propyl,
- i-Pr =: iPr = i-C₃H₇ = Isopropyl
- n-, i-, s-, tert-Bu =: n-, i-, s-, tert-Butyl
- c-Alkyl =: Cycloalkyl

Teilstrukturen der Formel T* (zur 4. Spalte bzw. 5. Spalte in Tabellen 1 und 2) sind Reste der Formel 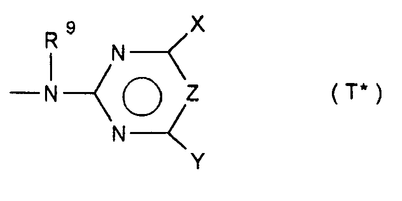 mit den in der nachfolgenden Tabelle angegebenen Bedeutungen:

| T* | R⁹ | X | Y | Z |
|---|---|---|---|---|
| T1 | H | OMe | OMe | CH |
| T2 | H | OMe | Me | CH |
| T3 | H | Me | Me | CH |
| T4 | H | Me | OEt | CH |
| T5 | H | Et | OMe | CH |
| T6 | H | OEt | OEt | CH |
| T7 | H | OCHF₂ | OCHF₂ | CH |
| T8 | H | Me | OCHF₂ | CH |
| T9 | H | Cl | OMe | CH |
| T10 | H | OMe | OMe | N |
| T11 | H | OMe | Me | N |
| T12 | H | Me | Me | N |
| T13 | H | NMe₂ | OCH₂CF₃ | N |
| T14 | H | OMe | CF₃ | N |
| T15 | H | OEt | NHMe | N |
| T16 | Me | OMe | OMe | CH |
| T17 | Me | OMe | Me | N |

### Speziell zu Tabelle 1:

Die kombinierte Tabelle 1 führt Beispiele zu Verbindungen der Formeln (la), (lb), (lc) und (ld) auf. Eine Zeile in der Tabelle 1 kennzeichnet eine Definition der Kombination der Reste R⁶, R⁷ und T* für jede der Formeln (la), (lb), (lc) und (ld), in denen R¹ jeweils fest definiert ist. Dabei erhält die Verbindung der Formel (la) in einer Zeile die Beispielnummer aus der Zeilenziffer mit angehängtem Buchstaben "a". Entsprechendes gilt für Verbindungen der Formeln (lb), (lc) und (ld), zu denen in einer Zeile jeweils das Beispiel mit angehängtem "b", "c" bzw. "d" gehört.
Etwaige Angaben zu physikalischen Daten, in der Regel zum Schmelzpunkt der jeweiligen Beispiele, sind in dem Kästchen eingetragen, das in der Zeile mit der Beispielnummer und in der Spalte unter der Formelnummer (la, lb, lc oder ld) steht und somit der Verbindung eindeutig zugeordnet ist.

Ein Kästchen in der Spalte unter (la) definiert somit exakt eine Verbindung der Formel (la), deren Beispielnummer die in der Spalte unter "Nr." angegebene Nummer mit angehängten Buchstaben "a" ist; entsprechendes gilt für die Beispiele zu den Verbindungen der Formeln (1b) bis (1d).

Wenn nicht anders angegeben bedeuten die Zahlen in den Kästchen in der Spalte unter (la), (lb), (lc) bzw. (ld) Schmelzpunkte in Grad Celsius (°C).

**Tabelle 1: Verbindungen der Formeln (la), (lb), (lc) und (ld)**

| Bsp.Nr. | R⁶ | R⁷ | T* | (la) | (lb) | (lc) | (ld) |
|---|---|---|---|---|---|---|---|
| 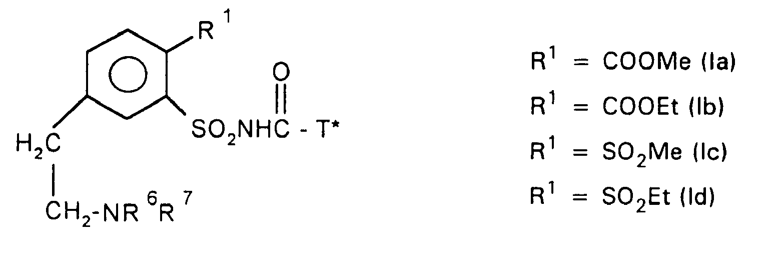 | | | | | | | |
| 1 a-d | H | CHO | T1 | 110-115 | | | |
| 2 a-d | H | " | T2 | | | | |
| 3 a-d | H | " | T3 | | | | |
| 4 a-d | H | " | T4 | | | | |
| 5 a-d | H | " | T5 | | | | |
| 6 a-d | H | " | T6 | | | | |
| 7 a-d | H | " | T7 | | | | |
| 8 a-d | H | " | T8 | | | | |
| 9 a-d | H | " | T9 | | | | |
| 10 a-d | H | " | T10 | | | | |
| 11 a-d | H | " | T 11 | | | | |
| 12 a-d | H | " | T12 | | | | |
| 13 a-d | H | " | T13 | | | | |
| 14 a-d | H | " | T14 | | | | |
| 15 a-d | H | " | T15 | | | | |
| 16 a-d | H | " | T16 | | | | |
| 17 a-d | H | " | T17 | | | | |
| 18 a-d | H | COMe | T1 | 199-200 | | | |
| 19 a-d | H | COMe | T2 | | | | |
| 20 a-d | H | COMe | T3 | | | | |
| 21 a-d | H | COMe | T4 | | | | |
| 22 a-d | H | COMe | T5 | | | | |
| 23 a-d | H | COMe | T6 | | | | |
| 24 a-d | H | COMe | T7 | | | | |
| 25 a-d | H | COMe | T8 | | | | |
| 26 a-d | H | COMe | T9 | | | | |
| 27 a-d | H | COMe | T10 | | | | |
| 28 a-d | H | COMe | T11 | | | | |
| 29 a-d | H | COMe | T12 | | | | |
| 30 a-d | H | COMe | T13 | | | | |
| 31 a-d | H | COMe | T14 | | | | |
| 32 a-d | H | COMe | T15 | | | | |
| 33 a-d | H | COMe | T16 | | | | |
| 34 a-d | H | COMe | T17 | | | | |
| 35 a-d | H | COEt | T1 | | | | |
| 36 a-d | H | COEt | T2 | | | | |
| 37 a-d | H | COEt | T3 | | | | |
| 38 a-d | H | COEt | T4 | | | | |
| 39 a-d | H | COEt | T5 | | | | |
| 40 a-d | H | COEt | T6 | | | | |
| 41 a-d | H | COEt | T7 | | | | |
| 42 a-d | H | COEt | T8 | | | | |
| 43 a-d | H | COEt | T9 | | | | |
| 44 a-d | H | COEt | T10 | | | | |
| 45 a-d | H | COEt | T11 | | | | |
| 46 a-d | H | COEt | T12 | | | | |
| 47 a-d | H | COEt | T13 | | | | |
| 48 a-d | H | COEt | T14 | | | | |
| 49 a-d | H | COEt | T15 | | | | |
| 50 a-d | H | COEt | T16 | | | | |
| 51 a-d | H | COEt | T17 | | | | |
| 52 a-d | H | COₙC₃H₇ | T1 | | | | |
| 53 a-d | H | COₙC₃H₇ | T2 | | | | |
| 54 a-d | H | COₙC₃H₇ | T3 | | | | |
| 55 a-d | H | COₙC₃H₇ | T4 | | | | |
| 56 a-d | H | COₙC₃H₇ | T5 | | | | |
| 57 a-d | H | COₙC₃H₇ | T6 | | | | |
| 58 a-d | H | COₙC₃H₇ | T7 | | | | |
| 59 a-d | H | COₙC₃H₇ | T8 | | | | |
| 60 a-d | H | COₙC₃H₇ | T9 | | | | |
| 61 a-d | H | COₙC₃H₇ | T10 | | | | |
| 62 a-d | H | COₙC₃H₇ | T11 | | | | |
| 63 a-d | H | COₙC₃H₇ | T12 | | | | |
| 64 a-d | H | COₙC₃H₇ | T13 | | | | |
| 65 a-d | H | COₙC₃H₇ | T14 | | | | |
| 66 a-d | H | COₙC₃H₇ | T15 | | | | |
| 67 a-d | H | COₙC₃H₇ | T16 | | | | |
| 68 a-d | H | CO_{nC3}H₇ | T17 | | | | |
| 69 a-d | H | CO-i-Pr | T1 1 | 103-108 | | | |
| 70 a-d | H | CO-i-Pr | T2 | | | | |
| 71 a-d | H | CO-i-Pr | T3 | | | | |
| 72 a-d | H | CO-i-Pr | T4 | | | | |
| 73 a-d | H | CO-i-Pr | T5 | | | | |
| 74 a-d | H | CO-i-Pr | T6 | | | | |
| 75 a-d | H | CO-i-Pr | T7 | | | | |
| 76 a-d | H | CO-i-Pr | T8 | | | | |
| 77 a-d | H | CO-i-Pr | T9 | | | | |
| 78 a-d | H | CO-i-Pr | T10 | | | | |
| 79 a-d | H | CO-i-Pr | T11 | | | | |
| 80 a-d | H | CO-i-Pr | T12 | | | | |
| 81 a-d | H | CO-i-Pr | T13 | | | | |
| 82 a-d | H | CO-i-Pr | T14 | | | | |
| 83 a-d | H | CO-i-Pr | T15 | | | | |
| 84 a-d | H | CO-i-Pr | T16 | | | | |
| 85 a-d | H | CO-i-Pr | T17 | | | | |
| 86 a-d | H | COCF₃ | T1 | 200-203 | | | |
| 87 a-d | H | COCF₃ | T2 | | | | |
| 88 a-d | H | COCF₃ | T3 | | | | |
| 89 a-d | H | COCF₃ | T4 | | | | |
| 90 a-d | H | COCF₃ | T5 | | | | |
| 91 a-d | H | COCF₃ | T6 | | | | |
| 92 a-d | H | COCF₃ | T7 | | | | |
| 93 a-d | H | COCF₃ | T8 | | | | |
| 94 a-d | H | COCF₃ | T9 | | | | |
| 95 a-d | H | COCF₃ | T10 | | | | |
| 96 a-d | H | COCF₃ | T11 | | | | |
| 97 a-d | H | COCF₃ | T12 | | | | |
| 98 a-d | H | COCF₃ | T13 | | | | |
| 99 a-d | H | COCF₃ | T14 | | | | |
| 100 a-d | H | COCF₃ | T15 | | | | |
| 101 a-d | H | COCF₃ | T16 | | | | |
| 102 a-d | H | COCF₃ | T17 | | | | |
| 103 a-d | H | COOCH₃ | T1 | 165-169 | | | |
| 104 a-d | H | COOCH₃ | T2 | | | | |
| 105 a-d | H | COOCH₃ | T3 | | | | |
| 106 a-d | H | COOCH₃ | T4 | | | | |
| 107 a-d | H | COOCH₃ | T5 | | | | |
| 108 a-d | H | COOCH₃ | T6 | | | | |
| 109 a-d | H | COOCH₃ | T7 | | | | |
| 110 a-d | H | COOCH₃ | T8 | | | | |
| 111 a-d | H | COOCH₃ | T9 | | | | |
| 112 a-d | H | COOCH₃ | T10 | | | | |
| 113 a-d | H | COOCH₃ | T11 | | | | |
| 114 a-d | H | COOCH₃ | T12 | | | | |
| 115 a-d | H | COOCH₃ | T13 | | | | |
| 116 a-d | H | COOCH₃ | T14 | | | | |
| 117 a-d | H | COOCH₃ | T15 | | | | |
| 118 a-d | H | COOCH₃ | T16 | | | | |
| 119 a-d | H | COOCH₃ | T17 | | | | |
| 120 a-d | H | COOEt | T1 | | | | |
| 121 a-d | H | " | T2 | | | | |
| 122 a-d | H | " | T3 | | | | |
| 123 a-d | H | " | T4 | | | | |
| 124 a-d | H | " | T5 | | | | |
| 125 a-d | H | " | T6 | | | | |
| 126 a-d | H | " | T7 | | | | |
| 127 a-d | H | " | T8 | | | | |
| 128 a-d | H | " | T9 | | | | |
| 129 a-d | H | " | T10 | | | | |
| 130 a-d | H | " | T11 | | | | |
| 131 a-d | H | " | T12 | | | | |
| 132 a-d | H | " | T13 | | | | |
| 133 a-d | H | " | T14 | | | | |
| 134 a-d | H | " | T15 | | | | |
| 135 a-d | H | " | T16 | | | | |
| 136 a-d | H | " | T17 | | | | |
| 137 a-d | H | SO₂CH₃ | T1 | 112-114 | | | |
| 138 a-d | H | SO₂CH₃ | T2 | | | | |
| 139 a-d | H | SO₂CH₃ | T3 | | | | |
| 140 a-d | H | SO₂CH₃ | T4 | | | | |
| 141 a-d | H | SO₂CH₃ | T5 | | | | |
| 142 a-d | H | SO₂CH₃ | T6 | | | | |
| 143 a-d | H | SO₂CH₃ | T7 | | | | |
| 144 a-d | H | SO₂CH₃ | T8 | | | | |
| 145 a-d | H | SO₂CH₃ | T9 | | | | |
| 146 a-d | H | SO₂CH₃ | T10 | | | | |
| 147 a-d | H | SO₂CH₃ | T11 | | | | |
| 148 a-d | H | SO₂CH₃ | T12 | | | | |
| 149 a-d | H | SO₂CH₃ | T13 | | | | |
| 150 a-d | H | SO₂CH₃ | T14 | | | | |
| 151 a-d | H | SO₂CH₃ | T15 | | | | |
| 152 a-d | H | SO₂CH₃ | T16 | | | | |
| 153 a-d | H | SO₂CH₃ | T17 | | | | |
| 154 a-d | H | SO₂Et | T1 | 103-105 | | | |
| 155 a-d | H | SO₂Et | T2 | | | | |
| 156 a-d | H | SO₂Et | T3 | | | | |
| 157 a-d | H | SO₂Et | T4 | | | | |
| 158 a-d | H | SO₂Et | T5 | | | | |
| 159 a-d | H | SO₂Et | T6 | | | | |
| 160 a-d | H | SO₂Et | T7 | | | | |
| 161 a-d | H | SO₂Et | T8 | | | | |
| 162 a-d | H | SO₂Et | T9 | | | | |
| 163 a-d | H | SO₂Et | T10 | | | | |
| 164 a-d | H | SO₂Et | T11 | | | | |
| 165 a-d | H | SO₂Et | T12 | | | | |
| 166 a-d | H | SO₂Et | T13 | | | | |
| 167 a-d | H | SO₂Et | T14 | | | | |
| 168 a-d | H | SO₂Et | T15 | | | | |
| 169 a-d | H | SO₂Et | T16 | | | | |
| 170 a-d | H | SO₂Et | T17 | | | | |
| 171 a-d | H | SO₂nPr | T1 | | | | |
| 172 a-d | H | SO₂nPr | T2 | | | | |
| 173 a-d | H | SO₂nPr | T3 | | | | |
| 174 a-d | H | SO₂nPr | T4 | | | | |
| 175 a-d | H | SO₂nPr | T5 | | | | |
| 176 a-d | H | SO₂nPr | T6 | | | | |
| 177 a-d | H | SO₂nPr | T7 | | | | |
| 178 a-d | H | SO₂nPr | T8 | | | | |
| 179 a-d | H | SO₂nPr | T9 | | | | |
| 180 a-d | H | SO₂nPr | T10 | | | | |
| 181 a-d | H | SO₂nPr | T11 | | | | |
| 182 a-d | H | SO₂nPr | T12 | | | | |
| 183 a-d | H | SO₂nPr | T13 | | | | |
| 184 a-d | H | SO₂nPr | T14 | | | | |
| 185 a-d | H | SO₂nPr | T15 | | | | |
| 186 a-d | H | SO₂nPr | T16 | | | | |
| 187 a-d | H | SO₂nPr | T17 | | | | |
| 188 a-d | H | SO₂iPr | T1 | | | | |
| 189 a-d | H | SO₂iPr | T2 | | | | |
| 190 a-d | H | SO₂iPr | T3 | | | | |
| 191 a-d | H | SO₂iPr | T4 | | | | |
| 192 a-d | H | SO₂iPr | T5 | | | | |
| 193 a-d | H | SO₂iPr | T6 | | | | |
| 194 a-d | H | SO₂iPr | T7 | | | | |
| 195 a-d | H | SO₂iPr | T8 | | | | |
| 196 a-d | H | SO₂iPr | T9 | | | | |
| 197 a-d | H | SO₂iPr | T10 | | | | |
| 198 a-d | H | SO₂iPr | T11 | | | | |
| 199 a-d | H | SO₂iPr | T12 | | | | |
| 200 a-d | H | SO₂iPr | T13 | | | | |
| 201 a-d | H | SO₂iPr | T14 | | | | |
| 202 a-d | H | SO₂iPr | T15 | | | | |
| 203 a-d | H | SO₂iPr | T16 | | | | |
| 204 a-d | H | SO₂iPr | T17 | | | | |
| 205 a-d | H | ClCH₂CO- | T1 | | | | |
| 206 a-d | H | ClCH₂CO- | T2 | | | | |
| 207 a-d | H | ClCH₂CO- | T3 | | | | |
| 208 a-d | H | ClCH₂CO- | T9 | | | | |
| 209 a-d | H | ClCH₂CO- | T10 | | | | |
| 210 a-d | H | ClCH₂CO- | T11 | | | | |
| 211 a-d | H | ClCH₂CO- | T14 | | | | |
| 212 a-d | H | ClCH₂CO- | T16 | | | | |
| 213 a-d | H | ClCH₂CO- | T17 | | | | |
| 214 a-d | H | Cl₂CHCO- | T1 | | | | |
| 215 a-d | H | Cl₂CHCO- | T2 | | | | |
| 216 a-d | H | Cl₂CHCO- | T3 | | | | |
| 217 a-d | H | Cl₂CHCO- | T9 | | | | |
| 218 a-d | H | Cl₂CHCO- | T10 | | | | |
| 219 a-d | H | Cl₂CHCO- | T11 | | | | |
| 220 a-d | H | Cl₂CHCO- | T14 | | | | |
| 221 a-d | H | Cl₂CHCO- | T16 | | | | |
| 222 a-d | H | Cl₂CHCO- | T17 | | | | |
| 223 a-d | H | Cl₃CCO- | T1 | | | | |
| 224 a-d | H | Cl₃CCO- | T2 | | | | |
| 225 a-d | H | Cl₃CCO- | T3 | | | | |
| 226 a-d | H | Cl₃CCO- | T9 | | | | |
| 227 a-d | H | Cl₃CCO- | T10 | | | | |
| 228 a-d | H | Cl₃CCO- | T11 | | | | |
| 229 a-d | H | Cl₃CCO- | T14 | | | | |
| 230 a-d | H | Cl₃CCO- | T16 | | | | |
| 231 a-d | H | Cl₃CCO- | T17 | | | | |
| 232 a-d | H | CH₃OCH₂CO | T1 | | | | |
| 233 a-d | H | CH₃OCH₂CO | T2 | | | | |
| 234 a-d | H | CH₃OCH₂CO | T3 | | | | |
| 235 a-d | H | CH₃OCH₂CO | T9 | | | | |
| 236 a-d | H | CH₃OCH₂CO | T10 | | | | |
| 237 a-d | H | CH₃OCH₂CO | T11 | | | | |
| 238 a-d | H | CH₃OCH₂CO | T14 | | | | |
| 239 a-d | H | CH₃OCH₃CO | T16 | | | | |
| 240 a-d | H | CH₃OCH₃CO | T17 | | | | |
| 241 a-d | H | CH₂=CHCO | T1 | | | | |
| 242 a-d | H | CH₂ = CHCO | T2 | | | | |
| 243 a-d | H | CH₂ = CHCO | T3 | | | | |
| 244 a-d | H | CH₂ = CHCO | T9 | | | | |
| 245 a-d | H | CH₂ = CHCO | T10 | | | | |
| 246 a-d | H | CH₂ = CHCO | T1 | | | | |
| 247 a-d | H | CH₂ = CHCO | T14 | | | | |
| 248 a-d | H | CH₂=CHCO | T16 | | | | |
| 249 a-d | H | CH₂ = CHCO | T17 | | | | |
| 250 a-d | H | CH≡CCO | T1 | | | | |
| 251 a-d | H | CH≡CCO | T2 | | | | |
| 252 a-d | H | CH≡CCO | T3 | | | | |
| 253 a-d | H | CH≡CCO | T9 | | | | |
| 254 a-d | H | CH≡CCO | T10 | | | | |
| 255 a-d | H | CH≡CCO | T11 | | | | |
| 256 a-d | H | CH≡CCO | T14 | | | | |
| 257 a-d | H | CH≡CCO | T16 | | | | |
| 258 a-d | H | CH≡CCO | T17 | | | | |
| 259 a-d | H | 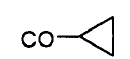 | T1 | 180-181 | | | |
| 260 a-d | H | " | T2 | | | | |
| 261 a-d | H | " | T3 | | | | |
| 262 a-d | H | " | T9 | | | | |
| 263 a-d | H | " | T10 | | | | |
| 264 a-d | H | " | T11 | | | | |
| 265 a-d | H | " | T14 | | | | |
| 266 a-d | H | " | T16 | | | | |
| 267 a-d | H | " | T17 | | | | |
| 268 a-d | H | 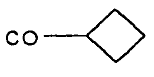 | T1 | | | | |
| 269 a-d | H | " | T2 | | | | |
| 270 a-d | H | " | T3 | | | | |
| 271 a-d | H | " | T9 | | | | |
| 272 a-d | H | " | T10 | | | | |
| 273 a-d | H | " | T11 | | | | |
| 274 a-d | H | " | T14 | | | | |
| 275 a-d | H | " | T16 | | | | |
| 276 a-d | H | " | T17 | | | | |
| 277 a-d | H | 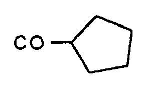 | T1 | | | | |
| 278 a-d | H | " | T2 | | | | |
| 279 a-d | H | " | T3 | | | | |
| 280 a-d | H | " | T9 | | | | |
| 281 a-d | H | " | T10 | | | | |
| 282 a-d | H | " | T11 | | | | |
| 283 a-d | H | " | T14 | | | | |
| 284 a-d | H | " | T16 | | | | |
| 285 a-d | H | " | T17 | | | | |
| 286 a-d | H | 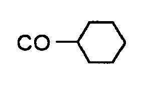 | T1 | | | | |
| 287 a-d | H | " | T2 | | | | |
| 288 a-d | H | " | T3 | | | | |
| 289 a-d | H | " | T9 | | | | |
| 290 a-d | H | " | T10 | | | | |
| 291 a-d | H | " | T11 | | | | |
| 292 a-d | H | " | T14 | | | | |
| 293 a-d | H | " | T16 | | | | |
| 294 a-d | H | " | T17 | | | | |
| 295 a-d | H | SO₂CF₃ | T1 | | | | |
| 296 a-d | H | SO₂CF₃ | T2 | | | | |
| 297 a-d | H | SO₂CF₃ | T3 | | | | |
| 298 a-d | H | SO₂CF₃ | T9 | | | | |
| 299 a-d | H | SO₂CF₃ | T10 | | | | |
| 300 a-d | H | SO₂CF₃ | T11 | | | | |
| 301 a-d | H | SO₂CF₃ | T14 | | | | |
| 302 a-d | H | SO₂CF₃ | T16 | | | | |
| 303 a-d | H | SO₂CF₃ | T17 | | | | |
| 304 a-d | H | SO₂CH₂F | T1 | | | | |
| 305 a-d | H | SO₂CH₂F | T2 | | | | |
| 306 a-d | H | SO₂CH₂F | T3 | | | | |
| 307 a-d | H | SO₂CH₂F | T9 | | | | |
| 308 a-d | H | SO₂CH₂F | T10 | | | | |
| 309 a-d | H | SO₂CH₂F | T11 | | | | |
| 310 a-d | H | SO₂CH₂F | T14 | | | | |
| 311 a-d | H | SO₂CH₂F | T16 | | | | |
| 312 a-d | H | SO₂CH₂F | T17 | | | | |
| 313 a-d | H | SO₂CH₂Cl | T1 | | | | |
| 314 a-d | H | SO₂CH₂Cl | T2 | | | | |
| 315 a-d | H | SO₂CH₂Cl | T3 | | | | |
| 316 a-d | H | SO₂CH₂Cl | T9 | | | | |
| 317 a-d | H | SO₂CH₂Cl | T10 | | | | |
| 318 a-d | H | SO₂CH₂Cl | T11 | | | | |
| 319 a-d | H | SO₂CH₂Cl | T14 | | | | |
| 320 a-d | H | SO₂CH₂Cl | T16 | | | | |
| 321 a-d | H | SO₂CH₂Cl | T17 | | | | |
| 322 a-d | H | SO₂CHCl₂ | T1 | | | | |
| 323 a-d | H | SO₂CHCl₂ | T2 | | | | |
| 324 a-d | H | SO₂CHCl₂ | T3 | | | | |
| 325 a-d | H | SO₂CHCl₂ | T9 | | | | |
| 326 a-d | H | SO₂CHCl₂ | T10 | | | | |
| 327 a-d | H | SO₂CHCl₂ | T11 | | | | |
| 328 a-d | H | SO₂CHCl₂ | T14 | | | | |
| 329 a-d | H | SO₂CHCl₂ | T16 | | | | |
| 330 a-d | H | SO₂CHCl₂ | T17 | | | | |
| 331 a-d | H | SO₂CCl₃ | T1 | | | | |
| 332 a-d | H | SO₂CCl₃ | T2 | | | | |
| 333 a-d | H | SO₂CCl₃ | T3 | | | | |
| 334 a-d | H | SO₂CCl₃ | T9 | | | | |
| 335 a-d | H | SO₂CCl₃ | T10 | | | | |
| 336 a-d | H | SO₂CCl₃ | T11 | | | | |
| 337 a-d | H | SO₂CCl₃ | T14 | | | | |
| 338 a-d | H | SO₂CCl₃ | T16 | | | | |
| 339 a-d | H | SO₂CCl₃ | T17 | | | | |
| 340 a-d | H | SO₂n-Bu | T1 | | | | |
| 341 a-d | H | SO₂n-Bu | T2 | | | | |
| 342 a-d | H | SO₂n-Bu | T3 | | | | |
| 343 a-d | H | SO₂n-Bu | T9 | | | | |
| 344 a-d | H | SO₂n-Bu | T10 | | | | |
| 345 a-d | H | SO₂n-Bu | T11 | | | | |
| 346 a-d | H | SO₂n-Bu | T14 | | | | |
| 347 a-d | H | SO₂n-Bu | T16 | | | | |
| 348 a-d | H | SO₂n-Bu | T17 | | | | |
| 349 a-d | H | SO₂CH₂CF₃ | T1 | | | | |
| 350 a-d | H | SO₂CH₂CF₃ | T2 | | | | |
| 351 a-d | H | SO₂CH₂CF₃ | T3 | | | | |
| 352 a-d | H | SO₂CH₂CF₃ | T9 | | | | |
| 353 a-d | H | SO₂CH₂CF₃ | T10 | | | | |
| 354 a-d | H | SO₂CH₂CF₃ | T11 | | | | |
| 355 a-d | H | SO₂CH₂CF₃ | T14 | | | | |
| 356 a-d | H | SO₂CH₂CF₃ | T16 | | | | |
| 357 a-d | H | SO₂CH₂CF₃ | T17 | | | | |
| 358 a-d | H | SO₂NHCH₃ | T1 | | | | |
| 359 a-d | H | SO₂NHCH₃ | T2 | | | | |
| 360 a-d | H | SO₂NHCH₃ | T3 | | | | |
| 361 a-d | H | SO₂NHCH₃ | T9 | | | | |
| 362 a-d | H | SO₂NHCH₃ | T10 | | | | |
| 363 a-d | H | SO₂NHCH₃ | T11 | | | | |
| 364 a-d | H | SO₂NHCH₃ | T14 | | | | |
| 365 a-d | H | SO₂NHCH₃ | T16 | | | | |
| 366 a-d | H | SO₂NHCH₃ | T17 | | | | |
| 367 a-d | H | SO₂N(CH₃)₂ | T1 | | | | |
| 368 a-d | H | SO₂N(CH₃)₂ | T2 | | | | |
| 369 a-d | H | SO₂N(CH₃)₂ | T3 | | | | |
| 370 a-d | H | SO₂N(CH₃)₂ | T9 | | | | |
| 371 a-d | H | SO₂N(CH₃)₂ | T10 | | | | |
| 372 a-d | H | SO₂N(CH₃)₂ | T11 | | | | |
| 373 a-d | H | SO₂N(CH₃)₂ | T14 | | | | |
| 374 a-d | H | SO₂N(CH₃)₂ | T16 | | | | |
| 375 a-d | H | SO₂N(CH₃)₂ | T17 | | | | |
| 376 a-d | H | (CH₃)₃COCO | T1 | | | | |
| 377 a-d | H | (CH₃)₃COCO | T2 | | | | |
| 378 a-d | H | (CH₃)₃COCO | T3 | | | | |
| 379 a-d | H | (CH₃)₃COCO | T9 | | | | |
| 380 a-d | H | (CH₃)₃COCO | T10 | | | | |
| 381 a-d | H | (CH₃)₃COCO | T11 | | | | |
| 382 a-d | H | (CH₃)₃COCO | T14 | | | | |
| 383 a-d | H | (CH₃)₃COCO | T16 | | | | |
| 384 a-d | H | (CH₃)₃COCO | T17 | | | | |
| 385 a-d | H | PhCO | T1 | | | | |
| 386 a-d | H | PhCO | T2 | | | | |
| 387 a-d | H | PhCO | T3 | | | | |
| 388 a-d | H | PhCO | T9 | | | | |
| 389 a-d | H | PhCO | T10 | | | | |
| 390 a-d | H | PhCO | T11 | | | | |
| 391 a-d | H | PhCO | T14 | | | | |
| 392 a-d | H | PhCO | T16 | | | | |
| 393 a-d | H | PhCO | T17 | | | | |
| 394 a-d | H | PhSO₂ | T1 | | | | |
| 395 a-d | H | PhSO₂ | T2 | | | | |
| 396 a-d | H | PhSO₂ | T3 | | | | |
| 387 a-d | H | PhSO₂ | T9 | | | | |
| 398 a-d | H | PhSO₂ | T10 | | | | |
| 399 a-d | H | PhSO₂ | T11 | | | | |
| 400 a-d | H | PhSO₂ | T14 | | | | |
| 401 a-d | H | PhSO₂ | T16 | | | | |
| 402 a-d | H | PhSO₂ | T17 | | | | |
| 403 a-d | H | MeNHCO | T1 | | | | |
| 404 a-d | H | MeNHCO | T2 | | | | |
| 405 a-d | H | MeNHCO | T3 | | | | |
| 406 a-d | H | MeNHCO | T9 | | | | |
| 407 a-d | H | MeNHCO | T10 | | | | |
| 408 a-d | H | MeNHCO | T11 | | | | |
| 409 a-d | H | MeNHCO | T14 | | | | |
| 410 a-d | H | MeNHCO | T16 | | | | |
| 411 a-d | H | MeNHCO | T17 | | | | |
| 412 a-d | H | EtNHCO | T1 | | | | |
| 413 a-d | H | EtNHCO | T2 | | | | |
| 414 a-d | H | EtNHCO | T3 | | | | |
| 415 a-d | H | EtNHCO | T9 | | | | |
| 416 a-d | H | EtNHCO | T10 | | | | |
| 417 a-d | H | EtNHCO | T11 | | | | |
| 418 a-d | H | EtNHCO | T14 | | | | |
| 419 a-d | H | EtNHCO | T16 | | | | |
| 420 a-d | H | EtNHCO | T17 | | | | |
| 421 a-d | H | MeNHCS | T1 | | | | |
| 422 a-d | H | MeNHCS | T2 | | | | |
| 423 a-d | H | MeNHCS | T3 | | | | |
| 424 a-d | H | MeNHCS | T9 | | | | |
| 425 a-d | H | MeNHCS | T10 | | | | |
| 426 a-d | H | MeNHCS | T11 | | | | |
| 427 a-d | H | MeNHCS | T14 | | | | |
| 428 a-d | H | MeNHCS | T16 | | | | |
| 429 a-d | H | MeNHCS | T17 | | | | |
| 430 a-d | H | EtNHCS | T1 | | | | |
| 431 a-d | H | EtNHCS | T2 | | | | |
| 432 a-d | H | EtNHCS | T3 | | | | |
| 433 a-d | H | EtNHCS | T9 | | | | |
| 434 a-d | H | EtNHCS | T10 | | | | |
| 435 a-d | H | EtNHCS | T11 | | | | |
| 436 a-d | H | EtNHCS | T14 | | | | |
| 437 a-d | H | EtNHCS | T16 | | | | |
| 438 a-d | H | EtNHCS | T17 | | | | |
| 439 a-d | 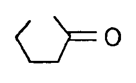 | | T1 | | | | |
| 440 a-d | " | | T2 | | | | |
| 441 a-d | " | | T3 | | | | |
| 442 a-d | " | | T9 | | | | |
| 443 a-d | " | | T10 | | | | |
| 444 a-d | " | | T11 | | | | |
| 445 a-d | " | | T14 | | | | |
| 446 a-d | " | | T16 | | | | |
| 447 a-d | " | | T17 | | | | |
| 448 a-d | 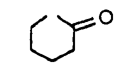 | | T1 | | | | |
| 449 a-d | " | | T2 | | | | |
| 450 a-d | " | | T3 | | | | |
| 451 a-d | " | | T9 | | | | |
| 452 a-d | " | | T10 | | | | |
| 453 a-d | " | | T11 | | | | |
| 454 a-d | " | | T14 | | | | |
| 455 a-d | " | | T16 | | | | |
| 456 a-d | " | | T17 | | | | |
| 457 a-d | 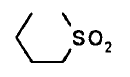 | | T1 | | | | |
| 458 a-d | " | | T2 | | | | |
| 459 a-d | " | | T3 | | | | |
| 460 a-d | " | | T9 | | | | |
| 461 a-d | " | | T10 | | | | |
| 462 a-d | " | | T11 | | | | |
| 463 a-d | " | | T14 | | | | |
| 464 a-d | " | | T16 | | | | |
| 465 a-d | " | | T17 | | | | |
| 466 a-d | 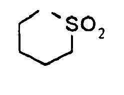 | | T1 | | | | |
| 467 a-d | " | | T2 | | | | |
| 468 a-d | " | | T3 | | | | |
| 469 a-d | " | | T9 | | | | |
| 470 a-d | " | | T10 | | | | |
| 471 a-d | " | | T11 | | | | |
| 472 a-d | " | | T14 | | | | |
| 473 a-d | " | | T16 | | | | |
| 474 a-d | " | | T17 | | | | |
| 475 a-d | 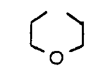 | | T1 | | | | |
| 476 a-d | " | | T2 | | | | |
| 477 a-d | " | | T3 | | | | |
| 478 a-d | " | | T9 | | | | |
| 479 a-d | " | | T10 | | | | |
| 480 a-d | " | | T11 | | | | |
| 481 a-d | " | | T14 | | | | |
| 482 a-d | " | | T16 | | | | |
| 483 a-d | " | | T17 | | | | |
| 484 a-d | H | COSMe | T1 | | | | |
| 485 a-d | H | COSMe | T2 | | | | |
| 486 a-d | H | COSMe | T3 | | | | |
| 487 a-d | H | COSMe | T9 | | | | |
| 488 a-d | H | COSMe | T10 | | | | |
| 489 a-d | H | COSMe | T11 | | | | |
| 490 a-d | H | COSMe | T14 | | | | |
| 491 a-d | H | COSMe | T16 | | | | |
| 492 a-d | H | COSMe | T17 | | | | |
| 493 a-d | H | CSOMe | T1 | | | | |
| 494 a-d | H | CSOMe | T2 | | | | |
| 495 a-d | H | CSOMe | T3 | | | | |
| 496 a-d | H | CSOMe | T9 | | | | |
| 497 a-d | H | CSOMe | T10 | | | | |
| 498 a-d | H | CSOMe | T11 | | | | |
| 499 a-d | H | CSOMe | T14 | | | | |
| 500 a-d | H | CSOMe | T16 | | | | |
| 501 a-d | H | CSOMe | T17 | | | | |
| 502 a-d | H | CSSMe | T1 | | | | |
| 503 a-d | H | CSSMe | T2 | | | | |
| 504 a-d | H | CSSMe | T3 | | | | |
| 505 a-d | H | CSSMe | T9 | | | | |
| 506 a-d | H | CSSMe | T10 | | | | |
| 507 a-d | H | CSSMe | T11 | | | | |
| 508 a-d | H | CSSMe | T14 | | | | |
| 509 a-d | H | CSSMe | T16 | | | | |
| 510 a-d | H | CSSMe | T17 | | | | |
| 511 a-d | H | COCOOMe | T1 | | | | |
| 512 a-d | H | COCOOMe | T2 | | | | |
| 513 a-d | H | COCOOMe | T3 | | | | |
| 514 a-d | H | COCOOMe | T9 | | | | |
| 515 a-d | H | COCOOMe | T10 | | | | |
| 516 a-d | H | COCOOMe | T11 | | | | |
| 517 a-d | H | COCOOMe | T14 | | | | |
| 518 a-d | H | COCOOMe | T16 | | | | |
| 519 a-d | H | COCOOMe | T17 | | | | |
| 520 a-d | H | i-C₃H₇OCO | T1 | | | | |
| 521 a-d | H | i-C₃H₇OCO | T2 | | | | |
| 522 a-d | H | i-C₃H₇OCO | T3 | | | | |
| 523 a-d | H | i-C₃H₇OCO | T9 | | | | |
| 524 a-d | H | i-C₃H₇OCO | T10 | | | | |
| 525 a-d | H | i-C₃H₇OCO | T11 | | | | |
| 526 a-d | H | i-C₃H₇OCO | T14 | | | | |
| 527 a-d | H | i-C₃H₇OCO | T16 | | | | |
| 528 a-d | H | i-C₃H₇ OCO | T17 | | | | |
| 529 a-d | H | CHO | T1 | Na-Salz 75-77 | | | |
| 530 a-d | H | Me₂CHCO | T1 | Na-Salz 179-183 | | | |
| 531 a-d | H | SO₂Et | T1 | Na-Salz 154-160 | | | |
| 532 a-d | Me | CHO | T1 | 179-181 | | | |
| 533 a-d | Me | CHO | T2 | | | | |
| 534 a-d | Me | CHO | T3 | | | | |
| 535 a-d | Me | CHO | T4 | | | | |
| 536 a-d | Me | CHO | T5 | | | | |
| 537 a-d | Me | CHO | T6 | | | | |
| 538 a-d | Me | CHO | T7 | | | | |
| 539 a-d | Me | CHO | T8 | | | | |
| 540 a-d | Me | CHO | T9 | | | | |
| 541 a-d | Me | CHO | T10 | | | | |
| 542 a-d | Me | CHO | T11 | | | | |
| 543 a-d | Me | CHO | T12 | | | | |
| 544 a-d | Me | CHO | T13 | | | | |
| 545 a-d | Me | CHO | T14 | | | | |
| 546 a-d | Me | CHO | T15 | | | | |
| 547 a-d | Me | CHO | T16 | | | | |
| 548 a-d | Me | CHO | T17 | | | | |
| 549 a-d | Me | COCH₃ | T1 | 217-218 | | | |
| 550 a-d | Me | COCH₃ | T2 | | | | |
| 551 a-d | Me | COCH₃ | T3 | | | | |
| 552 a-d | Me | COCH₃ | T4 | | | | |
| 553 a-d | Me | COCH₃ | T5 | | | | |
| 554 a-d | Me | COCH₃ | T6 | | | | |
| 555 a-d | Me | COCH₃ | T7 | | | | |
| 556 a-d | Me | COCH₃ | T8 | | | | |
| 557 a-d | Me | COCH₃ | T9 | | | | |
| 558 a-d | Me | COCH₃ | T10 | | | | |
| 559 a-d | Me | COCH₃ | T11 | | | | |
| 560 a-d | Me | COCH₃ | T12 | | | | |
| 561 a-d | Me | COCH₃ | T13 | | | | |
| 562 a-d | Me | COCH₃ | T14 | | | | |
| 563 a-d | Me | COCH₃ | T15 | | | | |
| 564 a-d | Me | COCH₃ | T16 | | | | |
| 565 a-d | Me | COCH₃ | T17 | | | | |
| 566 a-d | Me | COEt | T1 | 163-165 | | | |
| 567 a-d | Me | COEt | T2 | | | | |
| 568 a-d | Me | COEt | T3 | | | | |
| 569 a-d | Me | COEt | T4 | | | | |
| 570 a-d | Me | COEt | T5 | | | | |
| 571 a-d | Me | COEt | T6 | | | | |
| 572 a-d | Me | COEt | T7 | | | | |
| 573 a-d | Me | COEt | T8 | | | | |
| 574 a-d | Me | COEt | T9 | | | | |
| 575 a-d | Me | COEt | T10 | | | | |
| 576 a-d | Me | COEt | T11 | | | | |
| 577 a-d | Me | COEt | T12 | | | | |
| 578 a-d | Me | COEt | T13 | | | | |
| 579 a-d | Me | COEt | T14 | | | | |
| 580 a-d | Me | COEt | T15 | | | | |
| 581 a-d | Me | COEt | T16 | | | | |
| 582 a-d | Me | COEt | T17 | | | | |
| 583 a-d | Me | COₙC₃H₇ | T1 | | | | |
| 584 a-d | Me | COₙC₃H₇ | T2 | | | | |
| 585 a-d | Me | COₙC₃H₇ | T3 | | | | |
| 586 a-d | Me | COₙC₃H₇ | T4 | | | | |
| 587 a-d | Me | COₙC₃H₇ | T5 | | | | |
| 588 a-d | Me | COₙC₃H₇ | T6 | | | | |
| 589 a-d | Me | COₙC₃H₇ | T7 | | | | |
| 590 a-d | Me | COₙC₃H₇ | T8 | | | | |
| 591 a-d | Me | COₙC₃H₇ | T9 | | | | |
| 592 a-d | Me | COₙC₃H₇ | T10 | | | | |
| 593 a-d | Me | COₙC₃H₇ | T11 | | | | |
| 594 a-d | Me | COₙC₃H₇ | T12 | | | | |
| 595 a-d | Me | COₙC₃H₇ | T13 | | | | |
| 596 a-d | Me | COₙC₃H₇ | T14 | | | | |
| 597 a-d | Me | COₙC₃H₇ | T15 | | | | |
| 598 a-d | Me | COₙC₃H₇ | T16 | | | | |
| 599 a-d | Me | COₙC₃H₇ | T17 | | | | |
| 600 a-d | Me | CO-i-C₃H₇ | T1 | | | | |
| 601 a-d | Me | CO-i-C₃H₇ | T2 | | | | |
| 602 a-d | Me | CO-i-C₃H₇ | T3 | | | | |
| 603 a-d | Me | CO-i-C₃H₇ | T4 | | | | |
| 604 a-d | Me | CO-i-C₃H₇ | T5 | | | | |
| 605 a-d | Me | CO-i-C₃H₇ | T6 | | | | |
| 606 a-d | Me | CO-i-C₃H₇ | T7 | | | | |
| 607 a-d | Me | CO-i-C₃H₇ | T8 | | | | |
| 608 a-d | Me | CO-i-C₃H₇ | T9 | | | | |
| 609 a-d | Me | CO-i-C₃H₇ | T10 | | | | |
| 610 a-d | Me | CO-i-C₃H₇ | T11 | | | | |
| 611 a-d | Me | CO-i-C₃H₇ | T12 | | | | |
| 612 a-d | Me | CO-i-C₃H₇ | T13 | | | | |
| 613 a-d | Me | CO-i-C₃H₇ | T14 | | | | |
| 614 a-d | Me | CO-i-C₃H₇ | T15 | | | | |
| 615 a-d | Me | CO-i-C₃H₇ | T16 | | | | |
| 616 a-d | Me | CO-i-C₃H₇ | T17 | | | | |
| 617 a-d | Me | COCF₃ | T1 | | | | |
| 618 a-d | Me | COCF₃ | T2 | | | | |
| 619 a-d | Me | COCF₃ | T3 | | | | |
| 620 a-d | Me | COCF₃ | T4 | | | | |
| 621 a-d | Me | COCF₃ | T5 | | | | |
| 622 a-d | Me | COCF₃ | T6 | | | | |
| 623 a-d | Me | COCF₃ | T7 | | | | |
| 624 a-d | Me | COCF₃ | T8 | | | | |
| 625 a-d | Me | COCF₃ | T9 | | | | |
| 626 a-d | Me | COCF₃ | T10 | | | | |
| 627 a-d | Me | COCF₃ | T11 | | | | |
| 628 a-d | Me | COCF₃ | T12 | | | | |
| 629 a-d | Me | COCF₃ | T13 | | | | |
| 630 a-d | Me | COCF₃ | T14 | | | | |
| 631 a-d | Me | COCF₃ | T15 | | | | |
| 632 a-d | Me | COCF₃ | T16 | | | | |
| 633 a-d | Me | COCF₃ | T17 | | | | |
| 634 a-d | Me | COOMe | T1 | 181-183 | | | |
| 635 a-d | Me | COOMe | T2 | | | | |
| 636 a-d | Me | COOMe | T3 | | | | |
| 637 a-d | Me | COOMe | T4 | | | | |
| 638 a-d | Me | COOMe | T5 | | | | |
| 639 a-d | Me | COOMe | T6 | | | | |
| 640 a-d | Me | COOMe | T7 | | | | |
| 641 a-d | Me | COOMe | T8 | | | | |
| 642 a-d | Me | COOMe | T9 | | | | |
| 643 a-d | Me | COOMe | T10 | | | | |
| 644 a-d | Me | COOMe | T11 | | | | |
| 645 a-d | Me | COOMe | T12 | | | | |
| 646 a-d | Me | COOMe | T13 | | | | |
| 647a-d | Me | COOMe | T14 | | | | |
| 648 a-d | Me | COOMe | T15 | | | | |
| 649 a-d | Me | COOMe | T16 | | | | |
| 650 a-d | Me | COOMe | T17 | | | | |
| 651 a-d | Me | COOEt | T1 | | | | |
| 652 a-d | Me | COOEt | T2 | | | | |
| 653 a-d | Me | COOEt | T3 | | | | |
| 654 a-d | Me | COOEt | T4 | | | | |
| 655 a-d | Me | COOEt | T5 | | | | |
| 656 a-d | Me | COOEt | T6 | | | | |
| 657 a-d | Me | COOEt | T7 | | | | |
| 658 a-d | Me | COOEt | T8 | | | | |
| 659 a-d | Me | COOEt | T9 | | | | |
| 660 a-d | Me | COOEt | T10 | | | | |
| 661 a-d | Me | COOEt | T11 | | | | |
| 662 a-d | Me | COOEt | T12 | | | | |
| 663 a-d | Me | COOEt | T13 | | | | |
| 664 a-d | Me | COOEt | T14 | | | | |
| 665 a-d | Me | COOEt | T15 | | | | |
| 666 a-d | Me | COOEt | T16 | | | | |
| 667 a-d | Me | COOEt | T17 | | | | |
| 668 a-d | Me | SO₂CH₃ | T1 | 201-203 | | | |
| 669 a-d | Me | SO₂CH₃ | T2 | | | | |
| 670 a-d | Me | SO₂CH₃ | T3 | | | | |
| 671 a-d | Me | SO₂CH₃ | T4 | | | | |
| 672 a-d | Me | SO₂CH₃ | T5 | | | | |
| 673 a-d | Me | SO₂CH₃ | T6 | | | | |
| 674 a-d | Me | SO₂CH₃ | T7 | | | | |
| 675 a-d | Me | SO₂CH₃ | T8 | | | | |
| 676 a-d | Me | SO₂CH₃ | T9 | | | | |
| 677 a-d | Me | SO₂CH₃ | T10 | | | | |
| 678 a-d | Me | SO₂CH₃ | T11 | | | | |
| 679 a-d | Me | SO₂CH₃ | T12 | | | | |
| 680 a-d | Me | SO₂CH₃ | T13 | | | | |
| 681 a-d | Me | SO₂CH₃ | T14 | | | | |
| 682 a-d | Me | SO₂CH₃ | T15 | | | | |
| 683 a-d | Me | SO₂CH₃ | T16 | | | | |
| 684 a-d | Me | SO₂CH₃ | T17 | | | | |
| 685 a-d | Me | SO₂Et | T1 | 149-151 | | | |
| 686 a-d | Me | SO₂Et | T2 | | | | |
| 687 a-d | Me | SO₂Et | T3 | | | | |
| 688 a-d | Me | SO₂Et | T4 | | | | |
| 689 a-d | Me | SO₂Et | T5 | | | | |
| 690 a-d | Me | SO₂Et | T6 | | | | |
| 691 a-d | Me | SO₂Et | T7 | | | | |
| 692 a-d | Me | SO₂Et | T8 | | | | |
| 693 a-d | Me | SO₂Et | T9 | | | | |
| 694 a-d | Me | SO₂Et | T10 | | | | |
| 695 a-d | Me | SO₂Et | T11 | | | | |
| 696 a-d | Me | SO₂Et | T12 | | | | |
| 697 a-d | Me | SO₂Et | T13 | | | | |
| 698 a-d | Me | SO₂Et | T14 | | | | |
| 699 a-d | Me | SO₂Et | T15 | | | | |
| 700 a-d | Me | SO₂Et | T16 | | | | |
| 701 a-d | Me | SO₂Et | T17 | | | | |
| 702 a-d | Me | SO₂nPr | T1 | | | | |
| 703 a-d | Me | SO₂nPr | T2 | | | | |
| 704 a-d | Me | SO₂nPr | T3 | | | | |
| 705 a-d | Me | SO₂nPr | T4 | | | | |
| 706 a-d | Me | SO₂nPr | T5 | | | | |
| 707 a-d | Me | SO₂nPr | T6 | | | | |
| 708 a-d | Me | SO₂nPr | T7 | | | | |
| 709 a-d | Me | SO₂nPr | T8 | | | | |
| 710 a-d | Me | SO₂nPr | T9 | | | | |
| 711 a-d | Me | SO₂nPr | T10 | | | | |
| 712 a-d | Me | SO₂nPr | T11 | | | | |
| 713 a-d | Me | SO₂nPr | T12 | | | | |
| 714 a-d | Me | SO₂nPr | T13 | | | | |
| 715 a-d | Me | SO₂nPr | T14 | | | | |
| 716 a-d | Me | SO₂nPr | T15 | | | | |
| 717 a-d | Me | SO₂nPr | T16 | | | | |
| 718 a-d | Me | SO₂nPr | T17 | | | | |
| 719 a-d | Me | SO₂iPr | T1 | | | | |
| 720 a-d | Me | SO₂iPr | T2 | | | | |
| 721 a-d | Me | SO₂iPr | T3 | | | | |
| 722 a-d | Me | SO₂iPr | T4 | | | | |
| 723 a-d | Me | SO₂iPr | T5 | | | | |
| 724 a-d | Me | SO₂iPr | T6 | | | | |
| 725 a-d | Me | SO₂iPr | T7 | | | | |
| 726 a-d | Me | SO₂iPr | T8 | | | | |
| 727 a-d | Me | SO₂iPr | T9 | | | | |
| 728 a-d | Me | SO₂iPr | T10 | | | | |
| 729 a-d | Me | SO₂iPr | T11 | | | | |
| 730 a-d | Me | SO₂iPr | T12 | | | | |
| 731 a-d | Me | SO₂iPr | T13 | | | | |
| 732 a-d | Me | SO₂iPr | T14 | | | | |
| 733 a-d | Me | SO₂iPr | T15 | | | | |
| 734 a-d | Me | SO₂iPr | T16 | | | | |
| 735 a-d | Me | SO₂iPr | T17 | | | | |
| 736 a-d | Me | ClCH₂CO | T1 | | | | |
| 737 a-d | Me | ClCH₂CO | T2 | | | | |
| 738 a-d | Me | ClCH₂CO | T3 | | | | |
| 739 a-d | Me | ClCH₂CO | T9 | | | | |
| 740 a-d | Me | ClCH₂CO | T10 | | | | |
| 741 a-d | Me | ClCH₂CO | T11 | | | | |
| 742 a-d | Me | ClCH₂CO | T14 | | | | |
| 743 a-d | Me | ClCH₂CO | T16 | | | | |
| 744 a-d | Me | ClCH₂CO | T17 | | | | |
| 745 a-d | Me | Cl₂CHCO | T1 | | | | |
| 746 a-d | Me | Cl₂CHCO | T2 | | | | |
| 747 a-d | Me | Cl₂CHCO | T3 | | | | |
| 748 a-d | Me | Cl₂CHCO | T9 | | | | |
| 749 a-d | Me | Cl₂CHCO | T10 | | | | |
| 750 a-d | Me | Cl₂CHCO | T11 | | | | |
| 751 a-d | Me | Cl₂CHCO | T14 | | | | |
| 752 a-d | Me | Cl₂CHCO | T16 | | | | |
| 753 a-d | Me | Cl₂CHCO | T17 | | | | |
| 754 a-d | Me | Cl₃CCO | T1 | | | | |
| 755 a-d | Me | Cl₃CCO | T2 | | | | |
| 756 a-d | Me | Cl₃CCO | T3 | | | | |
| 757 a-d | Me | Cl₃CCO | T9 | | | | |
| 758 a-d | Me | Cl₃CCO | T10 | | | | |
| 759 a-d | Me | Cl₃CCO | T11 | | | | |
| 760 a-d | Me | Cl₃CCO | T14 | | | | |
| 761 a-d | Me | Cl₃CCO | T16 | | | | |
| 762 a-d | Me | Cl₃CCO | T17 | | | | |
| 763 a-d | Me | CH₃OCH₂CO | T1 | | | | |
| 764 a-d | Me | CH₃OCH₂CO | T2 | | | | |
| 765 a-d | Me | CH₃OCH₂CO | T3 | | | | |
| 766 a-d | Me | CH₃OCH₂CO | T9 | | | | |
| 767 a-d | Me | CH₃OCH₂CO | T10 | | | | |
| 768 a-d | Me | CH₃OCH₂CO | T11 | | | | |
| 769 a-d | Me | CH₃OCH₂CO | T14 | | | | |
| 770 a-d | Me | CH₃OCH₂CO | T16 | | | | |
| 771 a-d | Me | CH₃OCH₂CO | T17 | | | | |
| 772 a-d | Me | CH₂ = CHCO | T1 | | | | |
| 773 a-d | Me | CH₂=CHCO | T2 | | | | |
| 774 a-d | Me | CH₂ = CHCO | T3 | | | | |
| 775 a-d | Me | CH₂ = CHCO | T9 | | | | |
| 776 a-d | Me | CH₂ = CHCO | T10 | | | | |
| 777 a-d | Me | CH₂=CHCO | T11 | | | | |
| 778 a-d | Me | CH₂=CHCO | T14 | | | | |
| 779 a-d | Me | CH₂=CHCO | T16 | | | | |
| 780 a-d | Me | CH₂=CHCO | T17 | | | | |
| 781 a-d | Me | CH≡CCO | T1 | | | | |
| 782 a-d | Me | CH≡CCO | T2 | | | | |
| 783 a-d | Me | CH≡CCO | T3 | | | | |
| 784 a-d | Me | CH≡CCO | T9 | | | | |
| 785 a-d | Me | CH≡CCO | T10 | | | | |
| 786 a-d | Me | CH≡CCO | T11 | | | | |
| 787 a-d | Me | CH=CCO | T14 | | | | |
| 788 a-d | Me | CH=CCO | T16 | | | | |
| 789 a-d | Me | CH≡CCO | T17 | | | | |
| 790 a-d | Me | 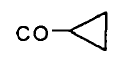 | T1 | | | | |
| 791 a-d | Me | " | T2 | | | | |
| 792 a-d | Me | " | T3 | | | | |
| 793 a-d | Me | " | T9 | | | | |
| 794 a-d | Me | " | T10 | | | | |
| 795 a-d | Me | " | T11 | | | | |
| 796 a-d | Me | " | T14 | | | | |
| 797 a-d | Me | " | T16 | | | | |
| 798 a-d | Me | " | T17 | | | | |
| 799 a-d | Me | 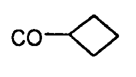 | T1 | | | | |
| 800 a-d | Me | " | T2 | | | | |
| 801 a-d | Me | " | T3 | | | | |
| 802 a-d | Me | " | T9 | | | | |
| 803 a-d | Me | " | T10 | | | | |
| 804 a-d | Me | " | T11 | | | | |
| 805 a-d | Me | " | T14 | | | | |
| 806 a-d | Me | " | T16 | | | | |
| 807 a-d | Me | " | T17 | | | | |
| 808 a-d | Me | 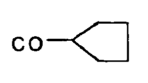 | T1 | | | | |
| 809 a-d | Me | " | T2 | | | | |
| 810 a-d | Me | " | T3 | | | | |
| 811 a-d | Me | " | T9 | | | | |
| 812 a-d | Me | " | T10 | | | | |
| 813 a-d | Me | " | T11 | | | | |
| 814 a-d | Me | " | T14 | | | | |
| 815 a-d | Me | " | T16 | | | | |
| 816 a-d | Me | " | T17 | | | | |
| 817 a-d | Me | 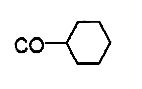 | T1 | | | | |
| 818 a-d | Me | " | T2 | | | | |
| 819 a-d | Me | " | T3 | | | | |
| 820 a-d | Me | " | T9 | | | | |
| 821 a-d | Me | " | T10 | | | | |
| 822 a-d | Me | " | T11 | | | | |
| 823 a-d | Me | " | T14 | | | | |
| 824 a-d | Me | " | T16 | | | | |
| 825 a-d | Me | " | T17 | | | | |
| 826 a-d | Me | SO₂CF₃ | T1 | | | | |
| 827 a-d | Me | SO₂CF₃ | T2 | | | | |
| 828 a-d | Me | SO₂CF₃ | T3 | | | | |
| 829 a-d | Me | SO₂CF₃ | T9 | | | | |
| 830 a-d | Me | SO₂CF₃ | T10 | | | | |
| 831 a-d | Me | SO₂CF₃ | T11 | | | | |
| 832 a-d | Me | SO₂CF₃ | T14 | | | | |
| 833 a-d | Me | SO₂CF₃ | T16 | | | | |
| 834 a-d | Me | SO₂CF₃ | T17 | | | | |
| 835 a-d | Me | SO₂CH₂F | T1 | 164-166 | | | |
| 836 a-d | Me | SO₂CH₂F | T2 | | | | |
| 837 a-d | Me | SO₂CH₂F | T3 | | | | |
| 838 a-d | Me | SO₂CH₂F | T9 | | | | |
| 839 a-d | Me | SO₂CH₂F | T10 | | | | |
| 840 a-d | Me | SO₂CH₂F | T11 | | | | |
| 841 a-d | Me | SO₂CH₂F | T14 | | | | |
| 842 a-d | Me | SO₂CH₂F | T16 | | | | |
| 843 a-d | Me | SO₂CH₂F | T17 | | | | |
| 844 a-d | Me | SO₂CH₂Cl | T1 | | | | |
| 845 a-d | Me | SO₂CH₂Cl | T2 | | | | |
| 846 a-d | Me | SO₂CH₂Cl | T3 | | | | |
| 847 a-d | Me | SO₂CH₂Cl | T9 | | | | |
| 848 a-d | Me | SO₂CH₂Cl | T10 | | | | |
| 849 a-d | Me | SO₂CH₂Cl | T11 | | | | |
| 850 a-d | Me | SO₂CH₂Cl | T14 | | | | |
| 851 a-d | Me | SO₂CH₂Cl | T16 | | | | |
| 852 a-d | Me | SO₂CH₂Cl | T17 | | | | |
| 853 a-d | Me | SO₂CHCl₂ | T1 | | | | |
| 854 a-d | Me | SO₂CHCl₂ | T2 | | | | |
| 855 a-d | Me | SO₂CHCl₂ | T3 | | | | |
| 856 a-d | Me | SO₂CHCl₂ | T9 | | | | |
| 857 a-d | Me | SO₂CHCl₂ | T10 | | | | |
| 858 a-d | Me | SO₂CHCl₂ | T11 | | | | |
| 859 a-d | Me | SO₂CHCl₂ | T14 | | | | |
| 860 a-d | Me | SO₂CHCl₂ | T16 | | | | |
| 861 a-d | Me | SO₂CHCl₂ | T17 | | | | |
| 862 a-d | Me | SO₂CCl₃ | T1 | | | | |
| 863 a-d | Me | SO₂CCl₃ | T2 | | | | |
| 864 a-d | Me | SO₂CCl₃ | T3 | | | | |
| 865 a-d | Me | SO₂CCl₃ | T9 | | | | |
| 866 a-d | Me | SO₂CCl₃ | T10 | | | | |
| 867 a-d | Me | SO₂CCl₃ | T11 | | | | |
| 868 a-d | Me | SO₂CCl₃ | T14 | | | | |
| 869 a-d | Me | SO₂CCl₃ | T16 | | | | |
| 870 a-d | Me | SO₂CCl₃ | T17 | | | | |
| 871 a-d | Me | SO₂nBu | T1 | | | | |
| 872 a-d | Me | SO₂nBu | T2 | | | | |
| 873 a-d | Me | SO₂nBu | T3 | | | | |
| 874 a-d | Me | SO₂nBu | T9 | | | | |
| 875 a-d | Me | SO₂nBu | T10 | | | | |
| 87 a-d6 | Me | SO₂nBu | T11 | | | | |
| 877 a-d | Me | SO₂nBu | T14 | | | | |
| 878 a-d | Me | SO₂nBu | T16 | | | | |
| 879 a-d | Me | SO₂nBu | T17 | | | | |
| 880 a-d | Me | SO₂CH₂CF₃ | T1 | | | | |
| 881 a-d | Me | SO₂CH₂CF₃ | T2 | | | | |
| 882 a-d | Me | SO₂CH₂CF₃ | T3 | | | | |
| 883 a-d | Me | SO₂CH₂CF₃ | T9 | | | | |
| 884 a-d | Me | SO₂CH₂CF₃ | T10 | | | | |
| 885 a-d | Me | SO₂CH₂CF₃ | T11 | | | | |
| 886 a-d | Me | SO₂CH₂CF₃ | T14 | | | | |
| 887 a-d | Me | SO₂CH₂CF₃ | T16 | | | | |
| 888 a-d | Me | SO₂CH₂CF₃ | T17 | | | | |
| 889 a-d | Me | SO₂NHCH₃ | T1 | | | | |
| 890 a-d | Me | SO₂NHCH₃ | T2 | | | | |
| 891 a-d | Me | SO₂NHCH₃ | T3 | | | | |
| 892 a-d | Me | SO₂NHCH₃ | T9 | | | | |
| 893 a-d | Me | SO₂NHCH₃ | T10 | | | | |
| 894 a-d | Me | SO₂NHCH₃ | T11 | | | | |
| 895 a-d | Me | SO₂NHCH₃ | T14 | | | | |
| 896 a-d | Me | SO₂NHCH₃ | T16 | | | | |
| 897 a-d | Me | SO₂NHCH₃ | T17 | | | | |
| 898 a-d | Me | SO₂N(CH₃)₂ | T1 | | | | |
| 899 a-d | Me | SO₂N(CH₃)₂ | T2 | | | | |
| 900 a-d | Me | SO₂N(CH₃)₂ | T3 | | | | |
| 901 a-d | Me | SO₂N(CH₃)₂ | T9 | | | | |
| 902 a-d | Me | SO₂N(CH₃)₂ | T10 | | | | |
| 903 a-d | Me | SO₂N(CH₃)₂ | T11 | | | | |
| 904 a-d | Me | SO₂N(CH₃)₂ | T14 | | | | |
| 905 a-d | Me | SO₂N(CH₃)₂ | T16 | | | | |
| 906 a-d | Me | SO₂N(CH₃)₂ | T17 | | | | |
| 907 a-d | Me | (CH₃)₃COCO | T1 | | | | |
| 908 a-d | Me | (CH₃)₃COCO | T2 | | | | |
| 909 a-d | Me | (CH₃)₃COCO | T3 | | | | |
| 910 a-d | Me | (CH₃)₃COCO | T9 | | | | |
| 911 a-d | Me | (CH₃)₃COCO | T10 | | | | |
| 912 a-d | Me | (CH₃)₃COCO | T11 | | | | |
| 913 a-d | Me | (CH₃)₃COCO | T14 | | | | |
| 914 a-d | Me | (CH₃)₃COCO | T16 | | | | |
| 915 a-d | Me | (CH₃)₃COCO | T17 | | | | |
| 916 a-d | Me | PhCO | T1 | | | | |
| 917 a-d | Me | PhCO | T2 | | | | |
| 918 a-d | Me | PhCO | T3 | | | | |
| 919 a-d | Me | PhCO | T9 | | | | |
| 920 a-d | Me | PhCO | T10 | | | | |
| 921 a-d | Me | PhCO | T11 | | | | |
| 922 a-d | Me | PhCO | T14 | | | | |
| 923 a-d | Me | PhCO | T16 | | | | |
| 924 a-d | Me | PhCO | T17 | | | | |
| 925 a-d | Me | PhSO₂ | T1 | | | | |
| 926 a-d | Me | PhSO2 | T2 | | | | |
| 927 a-d | Me | PhSO2 | T3 | | | | |
| 928 a-d | Me | PhSO2 | T9 | | | | |
| 929 a-d | Me | PhSO2 | T10 | | | | |
| 930 a-d | Me | PhSO2 | T11 | | | | |
| 931 a-d | Me | PhSO2 | T14 | | | | |
| 932 a-d | Me | PhSO2 | T16 | | | | |
| 933 a-d | Me | PhSO2 | T17 | | | | |
| 934 a-d | Me | MeNHCO | T1 | | | | |
| 935 a-d | Me | MeNHCO | T2 | | | | |
| 936 a-d | Me | MeNHCO | T3 | | | | |
| 937 a-d | Me | MeNHCO | T9 | | | | |
| 938 a-d | Me | MeNHCO | T10 | | | | |
| 939 a-d | Me | MeNHCO | T11 | | | | |
| 940 a-d | Me | MeNHCO | T14 | | | | |
| 941 a-d | Me | MeNHCO | T16 | | | | |
| 942 a-d | Me | MeNHCO | T17 | | | | |
| 943 a-d | Me | EtNHCO | T1 | | | | |
| 944 a-d | Me | EtNHCO | T2 | | | | |
| 945 a-d | Me | EtNHCO | T3 | | | | |
| 946 a-d | Me | EtNHCO | T9 | | | | |
| 947 a-d | Me | EtNHCO | T10 | | | | |
| 948 a-d | Me | EtNHCO | T11 | | | | |
| 949 a-d | Me | EtNHCO | T14 | | | | |
| 950 a-d | Me | EtNHCO | T16 | | | | |
| 951 a-d | Me | EtNHCO | T17 | | | | |
| 952 a-d | Me | MeNHCS | T1 | | | | |
| 953 a-d | Me | MeNHCS | T2 | | | | |
| 954 a-d | Me | MeNHCS | T3 | | | | |
| 955 a-d | Me | MeNHCS | T9 | | | | |
| 956 a-d | Me | MeNHCS | T10 | | | | |
| 957 a-d | Me | MeNHCS | T11 | | | | |
| 958 a-d | Me | MeNHCS | T14 | | | | |
| 959 a-d | Me | MeNHCS | T16 | | | | |
| 960 a-d | Me | MeNHCS | T17 | | | | |
| 961 a-d | Me | EtNHCS | T1 | | | | |
| 962 a-d | Me | EtNHCS | T2 | | | | |
| 963 a-d | Me | EtNHCS | T3 | | | | |
| 964 a-d | Me | EtNHCS | T9 | | | | |
| 965 a-d | Me | EtNHCS | T10 | | | | |
| 966 a-d | Me | EtNHCS | T11 | | | | |
| 967 a-d | Me | EtNHCS | T14 | | | | |
| 968 a-d | Me | EtNHCS | T16 | | | | |
| 969 a-d | Me | EtNHCS | T17 | | | | |
| 970 a-d | Me | COSMe | T1 | | | | |
| 971 a-d | Me | COSMe | T2 | | | | |
| 972 a-d | Me | COSMe | T3 | | | | |
| 973 a-d | Me | COSMe | T9 | | | | |
| 974 a-d | Me | COSMe | T10 | | | | |
| 975 a-d | Me | COSMe | T11 | | | | |
| 976 a-d | Me | COSMe | T14 | | | | |
| 977 a-d | Me | COSMe | T16 | | | | |
| 978 a-d | Me | COSMe | T17 | | | | |
| 979 a-d | Me | CSOMe | T1 | | | | |
| 980 a-d | Me | CSOMe | T2 | | | | |
| 981 a-d | Me | CSOMe | T3 | | | | |
| 982 a-d | Me | CSOMe | T9 | | | | |
| 983 a-d | Me | CSOMe | T10 | | | | |
| 984 a-d | Me | CSOMe | T11 | | | | |
| 985 a-d | Me | CSOMe | T14 | | | | |
| 986 a-d | Me | CSOMe | T16 | | | | |
| 987 a-d | Me | CSOMe | T17 | | | | |
| 988 a-d | Me | CSSMe | T1 | | | | |
| 989 a-d | Me | CSSMe | T2 | | | | |
| 990 a-d | Me | CSSMe | T3 | | | | |
| 991 a-d | Me | CSSMe | T9 | | | | |
| 992 a-d | Me | CSSMe | T10 | | | | |
| 993 a-d | Me | CSSMe | T11 | | | | |
| 994 a-d | Me | CSSMe | T14 | | | | |
| 995 a-d | Me | CSSMe | T16 | | | | |
| 996 a-d | Me | CSSMe | T17 | | | | |
| 997 a-d | Me | COCOOMe | T1 | | | | |
| 998 a-d | Me | COCOOMe | T2 | | | | |
| 999 a-d | Me | COCOOMe | T3 | | | | |
| 1000 a-d | Me | COCOOMe | T9 | | | | |
| 1001 a-d | Me | COCOOMe | T10 | | | | |
| 1002 a-d | Me | COCOOMe | T11 | | | | |
| 1003 a-d | Me | COCOOMe | T14 | | | | |
| 1004 a-d | Me | COCOOMe | T16 | | | | |
| 1005 a-d | Me | COCOOMe | T17 | | | | |
| 1006 a-d | Me | i-PrOCO | T1 | | | | |
| 1007 a-d | Me | i-PrOCO | T2 | | | | |
| 1008 a-d | Me | i-PrOCO | T3 | | | | |
| 1009 a-d | Me | i-PrOCO | T9 | | | | |
| 1010 a-d | Me | i-PrOCO | T10 | | | | |
| 1011 a-d | Me | i-PrOCO | T11 | | | | |
| 1012 a-d | Me | i-PrOCO | T14 | | | | |
| 1013 a-d | Me | i-PrOCO | T16 | | | | |
| 1014 a-d | Me | i-PrOCO | T17 | | | | |
| 1015 a-d | Et | CHO | T1 | | | | |
| 1016 a-d | Et | CHO | T2 | | | | |
| 1017 a-d | Et | CHO | T3 | | | | |
| 1018 a-d | Et | CHO | T9 | | | | |
| 1019 a-d | Et | CHO | T10 | | | | |
| 1020 a-d | Et | CHO | T11 | | | | |
| 1021 a-d | Et | CHO | T14 | | | | |
| 1022 a-d | Et | CHO | T16 | | | | |
| 1023 a-d | Et | CHO | T17 | | | | |
| 1024 a-d | Et | COMe | T1 | | | | |
| 1025 a-d | Et | COMe | T2 | | | | |
| 1026 a-d | Et | COMe | T3 | | | | |
| 1027 a-d | Et | COMe | T9 | | | | |
| 1028 a-d | Et | COMe | T10 | | | | |
| 1029 a-d | Et | COMe | T11 | | | | |
| 1030 a-d | Et | COMe | T14 | | | | |
| 1031 a-d | Et | COMe | T16 | | | | |
| 1032 a-d | Et | COMe | T17 | | | | |
| 1033 a-d | Et | COOMe | T1 | | | | |
| 1034 a-d | Et | COOMe | T2 | | | | |
| 1035 a-d | Et | COOMe | T3 | | | | |
| 1036 a-d | Et | COOMe | T9 | | | | |
| 1037 a-d | Et | COOMe | T10 | | | | |
| 1038 a-d | Et | COOMe | T11 | | | | |
| 1039 a-d | Et | COOMe | T14 | | | | |
| 1040 a-d | Et | COOMe | T16 | | | | |
| 1041 a-d | Et | COOMe | T17 | | | | |
| 1042 a-d | Et | COOEt | T1 | | | | |
| 1043 a-d | Et | COOEt | T2 | | | | |
| 1044 a-d | Et | COOEt | T3 | | | | |
| 1045 a-d | Et | COOEt | T9 | | | | |
| 1046 a-d | Et | COOEt | T10 | | | | |
| 1047 a-d | Et | COOEt | T11 | | | | |
| 1048 a-d | Et | COOEt | T14 | | | | |
| 1049 a-d | Et | COOEt | T16 | | | | |
| 1050 a-d | Et | COOEt | T17 | | | | |
| 1051 a-d | Et | SO₂Me | T1 | | | | |
| 1052 a-d | Et | SO₂Me | T2 | | | | |
| 1053 a-d | Et | SO₂Me | T3 | | | | |
| 1054 a-d | Et | SO₂Me | T9 | | | | |
| 1055 a-d | Et | SO₂Me | T10 | | | | |
| 1056 a-d | Et | SO₂Me | T11 | | | | |
| 1057 a-d | Et | SO₂Me | T14 | | | | |
| 1058 a-d | Et | SO₂Me | T16 | | | | |
| 1059 a-d | Et | SO₂Me | T17 | | | | |
| 1060 a-d | Et | SO₂Et | T1 | | | | |
| 1061 a-d | Et | SO₂Et | T2 | | | | |
| 1062 a-d | Et | SO₂Et | T3 | | | | |
| 1063 a-d | Et | SO₂Et | T9 | | | | |
| 1064 a-d | Et | SO₂Et | T10 | | | | |
| 1065 a-d | Et | SO₂Et | T11 | | | | |
| 1066 a-d | Et | SO₂Et | T14 | | | | |
| 1067 a-d | Et | SO₂Et | T16 | | | | |
| 1068 a-d | Et | SO₂Et | T17 | | | | |
| 1069a-d | H | 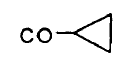 | T1 | Na-Salz 187-190 | | | |
| 1070a-d | H | CO-Me | T1 | Na-Salz 179-181 | | | |
| 1071 a-d | Me | SO₂Et | T1 | Na-Salz 125-135 | | | |
| 1072a-d | Me | SO₂CH₂F | T1 | Na-Salz 170-175 | | | |

**Tabelle 2**

| Verbindungen der Formel (le) | | | | | |
|---|---|---|---|---|---|
| 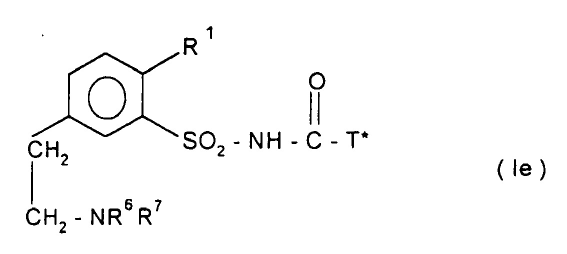 | | | | | |
| Nr. | R¹ | R⁶ | R⁷ | T* | Physik. Daten |
| 1 e | 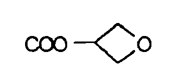 | H | CHO | T1 | |
| 2e | " | H | CHO | T11 | |
| 3e | " | Me | CHO | T1 | |
| 4e | " | Me | CHO | T11 | |
| 5e | " | H | COCH₃ | T1 | |
| 6e | " | H | COCH₃ | T11 | |
| 7e | " | Me | COCH₃ | T1 | |
| 8e | " | Me | COCH₃ | T11 | |
| 9e | " | H | COCF₃ | T1 | |
| 10e | " | H | COCF₃ | T11 | |
| 11e | " | Me | COCF₃ | T1 | |
| 12e | " | Me | COCF₃ | T11 | |
| 13e | " | H | COOMe | T1 | |
| 14e | " | H | COOMe | T11 | |
| 15e | " | Me | COOMe | T1 | |
| 16e | " | Me | COOMe | T11 | |
| 17e | " | H | COOEt | T1 | |
| 18e | " | H | COOEt | T11 | |
| 19e | " | Me | COOEt | T1 | |
| 20e | " | Me | COOEt | T11 | |
| 21e | " | H | SO₂Me | T1 | |
| 22e | " | H | " | T11 | |
| 23e | " | Me | " | T1 | |
| 24e | " | Me | " | T11 | |
| 25e | " | H | SO₂Et | T1 | |
| 26e | " | H | " | T11 | |
| 27e | " | Me | " | T1 | |
| 28e | " | Me | " | T11 | |
| 29e | 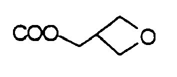 | H | CHO | T1 | |
| 30e | " | H | CHO | T11 | |
| 31e | " | Me | " | T1 | |
| 32e | " | Me | " | T11 | |
| 33e | " | H | COCH₃ | T1 | |
| 34e | " | H | COCH₃ | T11 | |
| 35e | " | Me | " | T1 | |
| 36e | " | Me | " | T11 | |
| 37e | " | H | COCF₃ | T1 | |
| 38e | " | H | COCF₃ | T11 | |
| 39e | " | Me | " | T1 | |
| 40e | " | Me | " | T11 | |
| 41e | " | H | COOMe | T1 | |
| 42e | " | H | COOMe | T11 | |
| 43e | " | Me | " | T1 | |
| 44e | " | Me | " | T11 | |
| 45e | " | H | COOEt | T1 | |
| 46e | " | H | COOEt | T11 | |
| 47e | " | Me | " | T1 | |
| 48e | 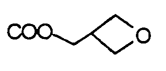 | Me | COOEt | T11 | |
| 49e | " | H | SO₂Me | T1 | |
| 50e | " | H | SO₂Me | T11 | |
| 51e | " | Me | " | T1 | |
| 52e | " | Me | " | T11 | |
| 53e | " | H | SO₂Et | T1 | |
| 54e | " | H | SO₂Et | T11 | |
| 55e | " | Me | " | T1 | |
| 56e | " | Me | " | T11 | |
| 57e | 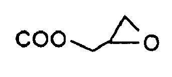 | H | CHO | T1 | |
| 58e | " | H | CHO | T11 | |
| 59e | " | Me | CHO | T1 | |
| 60e | " | Me | " | T11 | |
| 61e | " | H | COMe | T1 | |
| 62e | " | H | COMe | T11 | |
| 63e | " | Me | COMe | T1 | |
| 64e | " | Me | COMe | T11 | |
| 65e | " | H | COCF₃ | T1 | |
| 66e | " | H | COCF₃ | T11 | |
| 67e | " | Me | " | T1 | |
| 68e | " | Me | " | T11 | |
| 69e | " | H | COOMe | T1 | |
| 70e | " | H | COOMe | T11 | |
| 71e | " | Me | " | T1 | |
| 72e | " | Me | " | T11 | |
| 73e | " | H | COOEt | T1 | |
| 74e | " | H | COOEt | T11 | |
| 75e | " | Me | " | T1 | |
| 76e | " | Me | " | T11 | |
| 77e | " | H | SO₂Me | T1 | |
| 78e | " | H | " | T11 | |
| 79e | " | Me | " | T1 | |
| 80e | " | Me | " | T11 | |
| 81e | " | H | SO₂Et | T1 | |
| 82e | " | H | " | T11 | |
| 83e | " | Me | " | T1 | |
| 84e | " | Me | " | T11 | |
| 85e | " | H | SO₂CF₃ | T1 | |
| 86e | " | H | " | T11 | |
| 87e | " | Me | " | T1 | |
| 88e | " | Me | " | T11 | |
| 89e | SO₂n-Pr | H | CHO | T1 | |
| 90e | " | H | CHO | T11 | |
| 91e | " | H | COMe | T1 | |
| 92e | " | H | COMe | T11 | |
| 93e | " | H | COEt | T1 | |
| 94e | " | H | COEt | T11 | |
| 95e | " | H | COCF₃ | T1 | |
| 96e | " | H | COCF₃ | T11 | |
| 97e | " | H | COOMe | T1 | |
| 98e | " | H | COOMe | T11 | |
| 99e | " | H | COOEt | T1 | |
| 100e | " | H | COOEt | T11 | |
| 101e | " | H | SO₂Me | T1 | |
| 102e | " | H | SO₂Me | T11 | |
| 103e | " | H | SO₂Et | T1 | |
| 104e | " | H | SO₂Et | T11 | |
| 105e | " | Me | CHO | T1 | |
| 106e | " | Me | CHO | T11 | |
| 107e | " | Me | COMe | T1 | |
| 108e | " | Me | COMe | T11 | |
| 109e | " | Me | COEt | T1 | |
| 110e | " | Me | COEt | T11 | |
| 111e | " | Me | COCF₃ | T1 | |
| 112e | " | Me | COCF₃ | T11 | |
| 113e | " | Me | COOMe | T1 | |
| 114e | " | Me | COOMe | T11 | |
| 115e | " | Me | COOEt | T1 | |
| 116e | " | Me | COOEt | T11 | |
| 117e | " | Me | SO₂Me | T1 | |
| 118e | " | Me | SO₂Me | T11 | |
| 119e | " | Me | SO₂Et | T1 | |
| 120e | " | Me | SO₂Et | T11 | |
| 121e | " | Et | CHO | T1 | |
| 122e | " | Et | CHO | T11 | |
| 123e | " | Et | COMe | T1 | |
| 124e | " | Et | COMe | T11 | |
| 125e | " | Et | COEt | T1 | |
| 126e | " | Et | COEt | T11 | |
| 127e | " | Et | COCF₃ | T1 | |
| 128e | " | Et | COCF₃ | T11 | |
| 129e | " | Et | COOMe | T1 | |
| 130e | " | Et | COOMe | T11 | |
| 131 e | " | Et | COOEt | T1 | |
| 132e | " | Et | COOEt | T11 | |
| 133e | " | Et | SO₂Me | T1 | |
| 134e | " | Et | SO₂Me | T11 | |
| 135e | " | Et | SO₂Et | T1 | |
| 136e | " | Et | SO₂Et | T11 | |
| 137e | SO₂NMe₂ | H | CHO | T1 | |
| 138e | " | H | CHO | T11 | |
| 139e | " | H | COMe | T1 | |
| 140e | " | H | COMe | T11 | |
| 141 e | " | H | COEt | T1 | |
| 142e | " | H | COCF₃ | T1 | |
| 143e | " | H | COCF₃ | T11 | |
| 144e | " | H | COOMe | T1 | |
| 145e | " | H | COOMe | T11 | |
| 146e | " | H | COOEt | T1 | |
| 147e | " | H | COOEt | T11 | |
| 148e | " | H | SO₂Me | T1 | |
| 149e | " | H | SO₂Me | T11 | |
| 150e | " | H | SO₂Et | T1 | |
| 151e | " | H | SO₂Et | T11 | |
| 152e | " | Me | CHO | T1 | |
| 153e | " | Me | CHO | T11 | |
| 154e | " | Me | COMe | T1 | |
| 155e | " | Me | COMe | T11 | |
| 156e | " | Me | COEt | T1 | |
| 157e | " | Me | COEt | T11 | |
| 158e | " | Me | COCF₃ | T1 | |
| 159e | " | Me | COCF₃ | T11 | |
| 160e | " | Me | COOMe | T1 | |
| 161e | " | Me | COOMe | T11 | |
| 162e | " | Me | COOEt | T1 | |
| 163e | " | Me | COOEt | T11 | |
| 164e | " | Me | SO₂Me | T1 | |
| 165e | " | Me | SO₂Me | T11 | |
| 166e | " | Me | SO₂Et | T1 | |
| 167e | " | Me | SO₂Et | T11 | |
| 168e | " | Et | CHO | T1 | |
| 169e | " | Et | CHO | T11 | |
| 170e | " | Et | COMe | T1 | |
| 171e | " | Et | COMe | T11 | |
| 172e | " | Et | COEt | T1 | |
| 173e | " | Et | COEt | T11 | |
| 174e | " | Et | COCF₃ | T1 | |
| 175e | " | Et | COCF₃ | T11 | |
| 176e | " | Et | COOMe | T1 | |
| 177e | " | Et | COOMe | T11 | |
| 178e | " | Et | COOEt | T1 | |
| 179e | " | Et | COOEt | T11 | |
| 180e | " | Et | SO₂Me | T1 | |
| 181e | " | Et | SO₂Me | T11 | |
| 182e | " | Et | SO₂Et | T1 | |
| 183e | " | Et | SO₂Et | T11 | |
| 184e | SCH₃ | H | CHO | T1 | |
| 185e | " | H | CHO | T11 | |
| 186e | " | H | COOMe | T1 | |
| 187e | " | H | COOMe | T11 | |
| 188e | " | H | SO₂Me | T1 | |
| 189e | " | H | " | T11 | |
| 190e | " | Me | CHO | T1 | |
| 191 e | " | Me | CHO | T11 | |
| 192e | " | Me | COOMe | T1 | |
| 193e | " | Me | COOMe | T11 | |
| 194e | " | Me | SO₂Me | T1 | |
| 195e | " | Me | SO₂Me | T11 | |
| 196e | SC₂H₅ | H | CHO | T1 | |
| 197e | " | H | CHO | T11 | |
| 198e | " | H | COOMe | T1 | |
| 199e | " | H | COOMe | T11 | |
| 200e | " | H | SO₂Me | T1 | |
| 201e | " | H | " | T11 | |
| 202e | " | Me | CHO | T1 | |
| 203e | " | Me | CHO | T11 | |
| 204e | " | Me | COOMe | T1 | |
| 205e | " | Me | COOMe | T11 | |
| 206e | " | Me | SO₂Me | T1 | |
| 207e | " | Me | SO₂Me | T11 | |
| 208e | SO-CH₃ | H | CHO | T1 | |
| 209e | " | H | CHO | T11 | |
| 210e | " | H | COOMe | T1 | |
| 211e | " | H | COOMe | T1 | |
| 212e | " | H | SO₂Me | T1 | |
| 213e | " | H | " | T11 | |
| 214e | " | Me | CHO | T1 | |
| 215e | " | Me | CHO | T11 | |
| 216e | " | Me | COOMe | T1 | |
| 217e | " | Me | COOMe | T11 | |
| 218e | " | Me | SO₂Me | T1 | |
| 219e | " | Me | SO₂Me | T11 | |
| 220e | SO-C₂H₅ | H | CHO | T1 | |
| 221e | " | H | CHO | T11 | |
| 222e | " | H | COOMe | T1 | |
| 223e | " | H | COOMe | T11 | |
| 224e | " | H | SO₂Me | T1 | |
| 225e | " | H | " | T11 | |
| 226e | " | Me | CHO | T1 | |
| 227e | " | Me | CHO | T11 | |
| 228e | " | Me | COOMe | T1 | |
| 229e | " | Me | COOMe | T11 | |
| 230e | " | Me | SO₂Me | T1 | |
| 231e | " | Me | SO₂Me | T11 | |
| 232e | " | Et | CHO | T1 | |
| 233e | " | Et | CHO | T11 | |
| 234e | " | Et | COOCH₃ | T1 | |
| 235e | " | Et | COOCH₃ | T11 | |
| 236e | " | Et | SO₂Me | T1 | |
| 237e | " | Et | SO₂Me | T11 | |
| 238e | CO-NMe₂ | H | CHO | T11 | |
| 239e | " | H | COMe | T1 | |
| 240e | " | H | COMe | T11 | |
| 241e | " | H | COEt | T1 | |
| 242e | " | H | COEt | T11 | |
| 243e | " | H | COCF₃ | T1 | |
| 244e | " | H | COCF₃ | T11 | |
| 245e | " | H | COOMe | T1 | |
| 246e | " | H | COOMe | T11 | |
| 247e | " | H | COOEt | T1 | |
| 248e | " | H | COOEt | T11 | |
| 249e | " | H | SO₂Me | T1 | |
| 250e | " | H | SO₂Me | T11 | |
| 251e | " | H | SO₂Et | T1 | |
| 252e | " | H | SO₂Et | T11 | |
| 253e | " | Me | CHO | T1 | |
| 254e | " | Me | CHO | T11 | |
| 255e | " | Me | COMe | T1 | |
| 256e | " | Me | COMe | T11 | |
| 257e | " | Me | COEt | T1 | |
| 258e | " | Me | COEt | T11 | |
| 260e | " | Me | COCF₃ | T1 | |
| 261 e | " | Me | COCF₃ | T11 | |
| 262e | " | Me | COOMe | T1 | |
| 263e | " | Me | COOMe | T11 | |
| 264e | " | Me | COOEt | T1 | |
| 265e | " | Me | COOEt | T11 | |
| 266e | " | Me | SO₂Me | T1 | |
| 267e | " | Me | SO₂Me | T11 | |
| 268e | " | Me | SO₂Et | T1 | |
| 269e | " | Me | SO₂Et | T11 | |
| 270e | " | Et | CHO | T1 | |
| 271e | " | Et | CHO | T11 | |
| 272e | " | Et | COMe | T1 | |
| 273e | " | Et | COMe | T11 | |
| 274e | " | Et | COEt | T1 | |
| 275e | " | Et | COEt | T11 | |
| 276e | " | Et | COCF₃ | T1 | |
| 278e | " | Et | COCF₃ | T11 | |
| 279e | " | Et | COOMe | T1 | |
| 280e | " | Et | COOMe | T11 | |
| 281 e | " | Et | COOEt | T1 | |
| 282e | " | Et | COOEt | T11 | |
| 283e | " | Et | SO₂Me | T1 | |
| 284e | " | Et | SO₂Me | T11 | |
| 285e | " | Et | SO₂Et | T1 | |
| 286e | " | Et | SO₂Et | T11 | |
| 287e | " | H | CHO | T1 | |

### B. Formulierungsbeispiele

a) Ein Stäubemittel wird erhalten, indem man 10 Gew.-Teile einer Verbindung der Formel (I) und 90 Gew.-Teile Talkum als Inerstoff mischt und in einer Schlagmühle zerkleinert.
b) Ein in Wasser leicht dispergierbarer, benetzbares Pulver wird erhalten, indem man 25 Gewichtsteile einer Verbindung der Formel (I), 64 Gewichtsteile kaolinhaltigen Quarz als Inerstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (1) mit 6 Gew.-Teilen Alkylphenolpolyglykolether (^{®}Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen ethoxyliertes Nonylphenol als Emulgator.
e) Ein in Wasser dispergierbares Granulat wird erhalten indem man 75 Gewichtsteile einer Verbindung der Formel (I),

| | | |
|---|---|---|
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftsmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gewichtsteil(e) einer Verbindung der Formel (I),

| | | |
|---|---|---|
| 5 | " | 2, 2'-dinaphthylmethan-6, 6'-disulfonsaures Natrium |
| 2 | " | oleoylmethyltaurinsaures Natrium, |
| 1 | " | Polyvinylalkohol, |
| 17 | " | Calciumcarbonat und |
| 50 | " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen der Formel (I) oder deren Salze wurden dann als wäßrige Suspension bzw. Emulsion mit einer Wasseraufwandmenge von umgerechnet 600 bis 800 I/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Verbindungen der Beispiele Nr. 1a, 18a, 69a, 86a, 103a,137a,154a, 259a, 529a, 530a, 531 a, 532a, 549a, 566a, 634a, 668a, 685a, 835a, 1069a, 1070a, 1071 a und 1072a, (siehe Abschnitt A, Tabelle 1) sehr gute herbizide Wirkung gegen Schadpflanzen wie Sinapis alba, Stellaria media, Chrysanthemum segetum und Lolium multiflorum im Vorauflaufverfahren bei einer Aufwendmenge von 0,3 kg bis 0,005 kg Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen der Formel (I) oder deren Salze wurden in verschiedenen Dosierungen mit einer Wasseraufwandmenge von umgerechnet 600 bis 800 I/ha auf die grünen Pflanzenteile gesprüht. Nach ca. 3 bis 4 Wochen Standzeit der Versuchspflanzen im Gewächshaus unter optimalen Wachstumsbedingungen wurde die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Beispiele Nr. 1 a, 18a, 69a, 86a, 103a,137a,154a, 259a, 529a, 530a, 531 a, 532a, 549a, 566a, 634a, 668a, 685a, 835a, 1069a, 1070a, 1071 a und 1072a, (siehe Abschnitt A, Tabelle 1) sehr gute herbizide Wirkung gegen Schadpflanzen wie Sinapis alba, Stellaria media, Chrysanthemum segetum und Lolium multiflorum im Nachauflaufverfahren bei einer Aufwandmenge von 0,3 kg bis 0,005 kg Aktivsubstanz pro Hektar.

### 3. Kulturpflanzenverträglichkeit

In weiteren Versuchen im Gewächshaus wurden Samen einer größeren Anzahl von Kulturpflanzen und Unkräutern in sandigen Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen der Formel (I) oder deren Salzen in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrigen Kulturen, wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel (I) oder deren Salze weisen somit eine hohe Selektivität bei Anwendung zur Bekämpfung von unerwünschtem Pflanzenwuchs in landwirtschaftlichen Kulturen auf.

## Patentansprüche

1. Verbindungen der Formel (l) oder deren Salze, 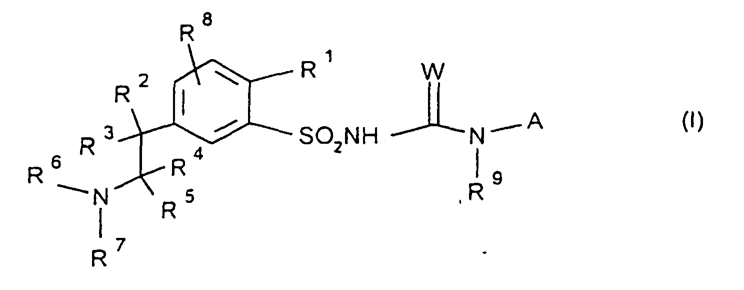 worin
R¹ S(O)ₙ-R¹⁰ oder COOR¹¹,
R², R³, R⁴, R⁵ unabhängig voneinander H oder (1-4)Alkyl,
R⁶ H, OH, Formyl, (1-6)Alkyl, (2-6)Alkenyl, (2-4)Alkinyl, (1-6)Alkoxy, (2-6)Alkenyloxy, (2-6)Alkinyloxy, [(1-6)Alkyl]-carbonyl, [(2-6)Alkenyl]-carbonyl, [(2-6)Alkinyl]-carbonyl), (1-4)Alkyl-sulfonyl, (2-6)Alkenylsulfonyl, (2-6)Alkinylsulfonyl, (3-6)Cycloalkyl, (5-6)Cycloalkenyl, [(3-6)Cycloalkyl]-carbonyl, [(5-6)Cycloalkenyl]-carbonyl, [(3-6)Cycloalkyl]-sulfonyl, [(5-6)Cycloalkenyl]-sulfonyl, wobei jeder der 18 letztgenannten Reste unsubstituiert oder durch einen oder mehrere Reste aus der Gruppe Halogen, (1-4)Alkoxy, (1-4)Alkylthio, (1-4)Alkylsulfinyl, (1-4)Alkylsulfonyl, [(1-4)Alkoxy]carbonyl, [(1-4)Alkyl]carbonyl, [(1-4)Alkyl]carbonyloxy und CN und im Falle cyclischer Reste auch (1-4)Alkyl und (1-4)Haloalkyl substituiert ist,
oder
Phenylcarbonyl oder Phenylsulfonyl, wobei jeder der beiden letztgenannten Reste im Phenylring unsubstituiert oder durch einen oder mehrere Reste aus der Gruppe Halogen, CN, NO₂, (1-4)Alkyl, (1-4)Haloalkyl, (1-4)Alkoxy und (1-4)Haloalkoxy substituiert ist,
R⁷ CHO, [(1-6)Alkyl]-carbonyl, [(2-6)Alkenyl]-carbonyl, [(2-6)Alkinyl]-carbonyl, (1-6)Alkylsulfonyl, (2-6)Alkenylsulfonyl, (2-6)Alkinylsulfonyl, [(3-6)Cycloalkyl]-carbonyl, [(5-6)Cycloalkenyl]-carbonyl, [(3-6)Cycloalkyl]sulfonyl, (5-6)Cycloalkenylsulfonyl, wobei jeder der 10 letztgenannten Reste unsubstituiert oder durch einen oder mehrere Reste aus der Gruppe Halogen, (1-4)Alkoxy, (1-4)Alkylthio, (1-4)Alkylsulfonyl, (1-4)Alkylsulfinyl, (1-4)Alkylcarbonyl, [(1-4)Alkoxy]-carbonyl, [(1-4)Alkyl]-carbonyloxy und CN und im Falle cyclischer Reste auch (1-4)Alkyl und (1-4)Haloalkyl substituiert ist,
oder
Phenylcarbonyl oder Phenylsulfonyl, wobei jeder der beiden letztgenannten Reste im Phenylring unsubstituiert oder durch einen oder mehrere Reste aus der Gruppe Halogen, CN, NO₂, (1-4)Alkyl, (1-4)Haloalkyl, (1-4)Alkoxy und (1-4)Haloalkoxy substituiert ist,
oder
Mono- oder Di-[(1-4)alkyl]aminosulfonyl, das im Alkylteil unsubstituiert oder durch einen oder mehrere Reste aus der Gruppe Halogen, (1-4)Alkoxy, (1-4)Alkylthio, (1-4)Alkylsulfinyl, (1-4)Alkyfsulfonyl, [(1-4)Alkyl]-carbonyl, [(1-4)Alkyl]-carbonyloxy, [(1-4)Alkoxy]-carbonyl und CN substituiert ist, oder eine Gruppe der Formel COCOR', worin R' = H, OH, (1-4)Alkoxy oder (1-4)Alkyl ist, oder eine Gruppe der Formel 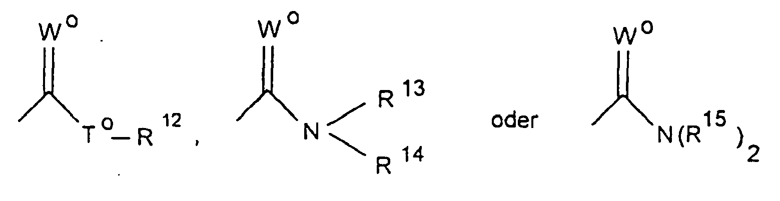 oder
R⁶ und R ⁷ gemeinsam eine Kette der Formel (-CH₂)ₘ₁ B¹- oder -B¹-(CH₂)ₘ₂B²-, wobei die Kette unsubstituiert oder durch einen oder mehrere (1-3)Alkylreste oder Halogen substituiert ist und m1 = 3, 4 oder 5 bzw. m2 = 2, 3 oder 4 sind, sowie
W,W° ein Sauerstoffatom oder ein Schwefelatom,
B¹,B² unabhängig voneinander SO₂ oder CO,
Q O, S oder NR¹⁶,
T^{o} O oder S,
R⁸ H, (1-4)Alkyl, (1-4)Alkoxy, (1-4)Alkylthio, [(1-4)Alkyl]carbonyl oder [(1-4)-Alkoxy]carbonyl, wobei jeder der letztgenannten 5 Reste unsubstituiert oder im Alkylteil durch ein oder mehrere Halogenatome substituiert ist, oder Halogen, NO₂, CN oder Mono- oder Di(1-4)alkylamino,
R⁹ H oder CH₃,
R¹⁰ NR¹⁷R¹⁸, (1-6)Alkyl, (2-6)Alkenyl, (2-6)Alkinyl, (3-6)Cycloalkyl, (5-6)Cycloalkenyl oder Phenyl, wobei jeder der letztgenannten 6 Reste unsubstituiert oder durch einen oder mehrere Reste aus der Gruppe Halogen, CN, (1-4)Alkoxy, (1-4)Alkylthio, (1-4)Alkylsulfinyl, (1-4)Alkylsulfonyl, [(1-4)Alkyl]-carbonyl, [(1-4)Alkoxy]-carbonyl und [(1-4)Alkyl]-carbonyloxy substituiert ist,
n die Zahl 0, 1 oder 2, außer für R¹⁰ = NR¹⁷R¹⁸, wo n = 2 ist, sowie
R¹¹ H, (1-6)Alkyl, (2-6)Alkenyl, (2-6)Alkinyl, (3-6)Cycloalkyl, (5-6)Cycloalkenyl oder Phenyl, wobei jeder der letztgenannten 6 Reste unsubstituiert oder durch einen oder mehrere Reste aus der Gruppe Halogen, CN, (1-4)Alkoxy, (1-4)Alkylthio, (1-4)Alkylsulfinyl, (1-4)Alkylsulfonyl, [(1-4)Alkyl]carbonyl, [(1-4)Alkoxylcarbonyl und [(1-4)Alkyl]carbonyloxy und im Falle cyclischer Reste auch (1-4)Alkyl und (1-4)Haloalkyl substituiert ist, oder einen Rest des Typs Heterocyclyl oder Heterocyclyl(1-4)alkyl mit 3-7 Ringatomen und 1 bis 3 Heteroringatomen aus der Gruppe N, O und S,
R¹² (1-4)Alkyl, (3-4)Alkenyl oder (3-4)Alkinyl, wobei jeder der drei letztgenannten Reste unsubstituiert oder durch einen oder mehrere Reste aus der Gruppe Halogen, (1-4)Alkoxy, (1-4)Alkylthio, [(1-4)Alkyl]-carbonyl und [(1-4)Alkoxy]-carbonyl substituiert ist,
R¹³, R¹⁴ unabhängig voneinander H, (1-4)Alkyl, (3-4)Alkenyl oder (3-4)Alkinyl, wobei jeder drei letztgenannten Reste unsubstituiert oder durch einen oder mehrere der Reste aus der Gruppe Halogen, (1-4)Alkoxy, (1-4)Alkylthio, [(1-4)Alkyl]-carbonyl und [(1-4)Alkoxy]-carbonyl substituiert ist,
die Reste R¹⁵ gemeinsam mit dem N-Atom einen heterocyclischen Ring mit 5 oder 6 Ringgliedern, der weitere Heteroatome aus der Gruppe N, O und S in den möglichen Oxidationsstufen enthalten kann und unsubstituiert oder durch (1-4)-Alkyl oder die Oxogruppe substituiert ist oder benzokondensiert ist,
R¹⁶ H, (1-4)Alkyl, (3-4)Alkenyl oder (3-4)Alkinyl, wobei jeder der drei letztgenannten Reste unsubstituiert oder durch einen oder mehrere Reste aus der Gruppe Halogen, (1-4)Alkoxy und (1-4)Alkylthio substituiert ist,
R¹⁷ H, (1-4)Alkyl oder (1-4)Alkoxy,
R¹⁸ H oder (1-4)Alkyl,
A einen Rest der Formel 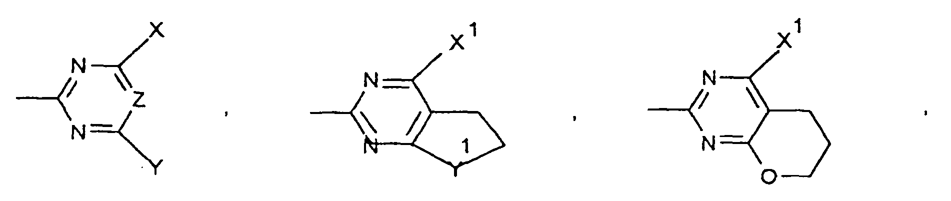 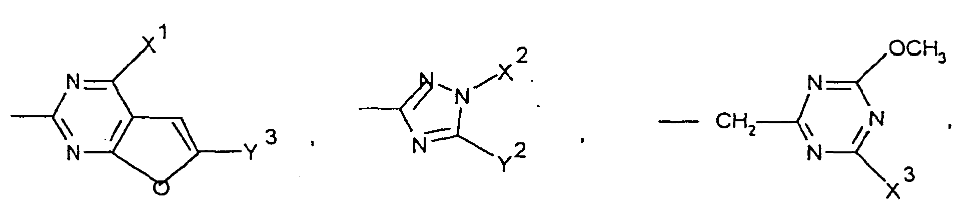 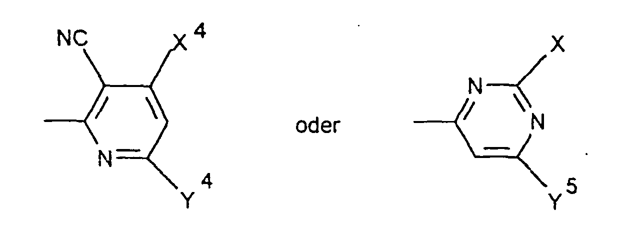
einer der Reste X und Y Wasserstoff, Halogen, (1-3)Alkyl oder (1-3)Alkoxy, wobei jeder der beiden letztgenannten Reste unsubstituiert oder durch einen oder mehrere Reste aus der Gruppe Halogen, (1-3)Alkoxy und (1-3)Alkylthio substituiert ist, und
der andere der Reste X und Y Wasserstoff, Halogen, (1-3)Alkyl, (1-3)Alkoxy oder (1-3)Alkylthio, wobei jeder der drei letztgenannten alkylhaltigen Reste unsubstituiert oder durch einen oder mehreren Reste aus der Gruppe Halogen, (1-3)Alkoxy und (1-3)Alkylthio substituiert ist, oder einen Rest der Formel NR^{a}R^{b}, (3-6)Cycloalkyl, (2-4)Alkenyl, (2-4)Alkinyl, (3-4)-Alkenyloxy oder (3-4)Alkinyloxy,
Z CH oder N,
R^{a} und R^{b} unabhängig voneinander H, (1-4)Alkyl oder (2-4)Alkenyl,
X¹ CH₃, OCH₃, OC₂H₅ oder OCHF₂,
Y¹ -O- oder -CH₂-,
X² CH₃, C₂H₅ oder CH₂CF₃,
Y² OCH₃, OC₂H₅, SCH₃, SCH₂CH₃, CH₃ oder C₂H₅,
x³ CH₃ oder OCH₃,
Y³ H oder CH₃,
X⁴ CH₃, OCH₃, OC₂H₅, CH₂OCH₃ oder Cl,
Y⁴ CH₃, OCH₃, OC₂H₅ oder Cl und
Y⁵ CH₃, C₂H₅, OCH₃ oder Cl
bedeuten.

2. Verbindungen oder deren Salze nach Anspruch 1, **dadurch gekennzeichnet, daß**
R¹ S(O)ₙ-R¹⁰ oder CO-OR¹¹,
n die Zahl 0, 1 oder 2, außer im Fall R¹⁰ = NR¹⁷R¹⁸, für den n = 2 ist,
R⁶ H oder (1-4)Alkyl, das unsubstituiert oder durch einen oder mehrere Halogenatome oder durch einen oder mehrere Resteausder Gruppe (1-4)Alkoxy und (1-4)Alkylthio substituiert ist,
R⁷ Formyl [(1-6)Alkyl]-carbonyl, [(2-4)Alkenyl]-carbonyl, [(2-4)Alkinyl]-carbonyl, [(3-6)Cycloalkyl]-carbonyl oder (1-6)Alkylsulfonyl, wobei jeder der 5 letztgenannten Reste unsubstituiert oder durch einen oder mehrere Reste aus der Gruppe Halogen, (1-4)Alkoxy, (1-4)Alkylthio, (1-4)Alkylsulfonyl, [(1-4)Alkyl]-carbonyl, [(1-4)Alkoxy]-carbonyl, [(1-4)Alkyl]-carbonyloxy und CN und im Fall cyclischer Reste auch (1-4)Alkyl und (1-4) Haloalkyl substituiert ist, oder
Phenylcarbonyl oder Phenylsulfonyl, wobei jeder der beiden letztgenannten Reste unsubstituiert oder durch einen oder mehrere Reste aus der Gruppe Halogen, CN, SO₂, (1-4)Alkyl, (1-4)Haloalkyl, (1-4)Alkoxy und (1-4)Haloalkoxy substituiert ist, oder
Mono- oder Di-[(1-4)-alkyl]-aminosufonyl, oder eine Gruppe der Formel -CO-CO-R', worin R' (1-4)Alkoxy bedeutet, oder
eine Gruppe der Formel -CW^{o}-R¹², -CW^{o}-NR¹³R¹⁴ oder -CW^{o}-N(R¹⁵)₂ oder
R⁶, R⁷ gemeinsam eine Kette der Formel (-CH₂)ₘ₁B¹- oder -B¹-(CH₂)ₘ₂B²-, wobei m1 = 3, 4 oder 5 und m2 = 2, 3 oder 4 bedeuten, und
W, W^{o} jweils unabhängig ein Sauerstoff- oder Schwefelatom,
T^{o} ein Sauerstoff- oder Schwefelatom,
B¹ SO₂ oder CO,
B² SO₂ oder GO,
Q O, S oder NR¹⁶,
R⁸ ein Wasserstoffatom,
R⁹ H oder CH₃,
R¹⁰ NR¹⁷R¹⁸, (1-6)Alkyl oder (3-6)Cycloalkyl,
R¹¹ H, (1-6)Alkyl, (3-6)Cycloalkyl oder einen Rest der Formeln A-1 bis A-6, 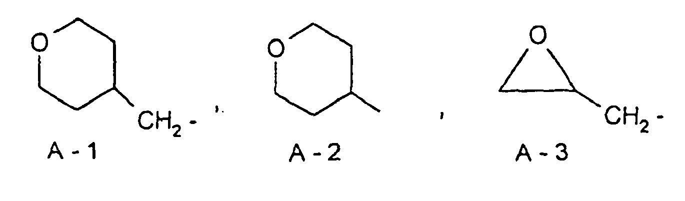 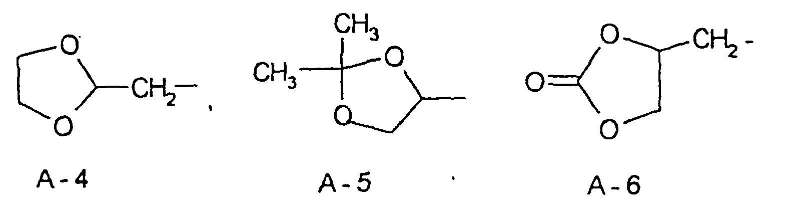
R¹² (1-4)Alkyl oder (1-4)Haloalkyl,
R¹³, R¹⁴ unabhängig voneinander H oder (1-4)Alkyl,
die Reste R¹⁵ gemeinsam eine divalente Kette der Formel -(CH₂)ₘ₃-, worin m3 = 3, 4 oder 5 ist, oder der Formel -CH₂CH₂-O-CH₂CH₂-,
R¹⁶ H oder (1-4)Alkyl,
R¹⁷ H oder (1-4)Alkyl und
R¹⁸ H oder (1-4)Alkyl
bedeuten.

3. Verbindungen oder deren Salze nach einem der Ansprüche 1 oder 2, **dadurch gekennzeichnet, daß**
R⁶ H oder (1-4)Alkyl,
R⁷ CHO, [(1-6)Alkyl]-carbonyl, [(1-4)Haloalkyl]-carbonyl, [(1-4)Alkoxy-(1-4)alkyl]-carbonyl, [(3-6)Cycloalkyl]-carbonyl, Phenylcarbonyl, das unsubstituiert oder durch einen oder mehrere Reste aus der Gruppe Halogen, CN, NO₂, (1-4)Alkyl, (1-4)Haloalkyl, (1-4)Alkoxy und (1-4)Haloalkoxy substituiert ist, oder Phenylsulfonyl, das unsubstituiert oder durch einen oder mehrere Reste aus der Gruppe (1-4)Alkyl und (1-4)Alkoxy substituiert ist, oder Mono- oder Di-[(1-4)alkyl]-aminosulfonyl, (1-6)Alkylsulfonyl, (1-4)Haloalkylsulfonyl oder eine Gruppe der Formel -CW^{o}-T-R¹² oder -CW^{o}-NR¹³R¹⁴
W, W^{o} jeweils unabhängig voneinander O oder S,
T^{o} O oder S,
Q O, S oder NR¹⁶,
R¹⁰ NR¹⁷R¹⁸, (1-4)Alkyl oder (3-6)Cycloalkyl,
R¹¹ H oder (1-4)Alkyl,
R¹² (1-4)Alkyl,
R¹³, R¹⁴ unabhängig voneinander H oder (1-4)Alkyl,
R¹⁶ H oder (1-3)Alkyl,
R¹⁷ (1-4)Alkyl und
R¹⁸ H oder (1-4)Alkyl
bedeuten.

4. Verfahren zur Herstellung von Verbindungen der Formel (I) oder deren Salze, wie sie nach einem der Ansprüche 1 bis 3 definiert sind, **dadurch gekennzeichnet, daß** man
a) eine Verbindung der Formel (11) 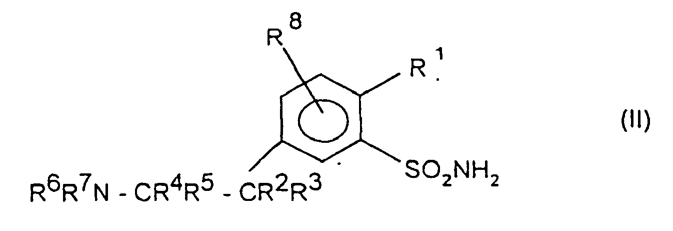 mit einem heterocyclischen Carbamat der Formel (III),
R* - O - CO - NR⁹ - A (III)
worin R* gegebenenfalls substituiertes Phenyl oder (C₁-C₄)Alkyl bedeutet, umsetzt oder
b) ein Arylsulfonylcarbamat der Formel (IV) 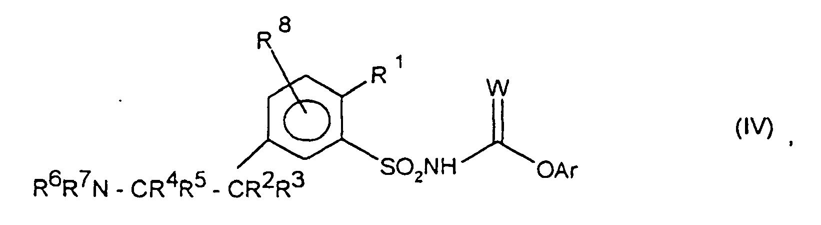 worin Ar einen Arylrest, bedeutet mit einem Aminoheterocyclus der Formel (V)
H - NR⁹ - A (V)
umsetzt oder
c) ein Sulfonylisocyanat der Formel (VI) 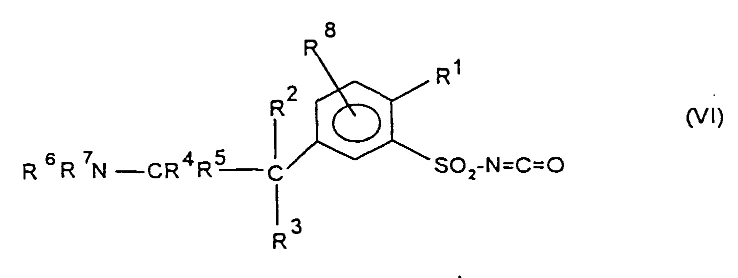 mit einem Aminoheterocyclus der Formel H-NR⁹-A (V) umsetzt oder
d) in einer Eintopfreaktion zunächst einen Aminoheterocyclus der Formel H-NR⁹-A (V) in Gegenwart einer Base mit Phosgen umsetzt und das gebildete Intermediat mit einem Phenylsulfonamid der Formel (II) umsetzt oder
e) ein Sulfonylchlorid der Formel (VII) 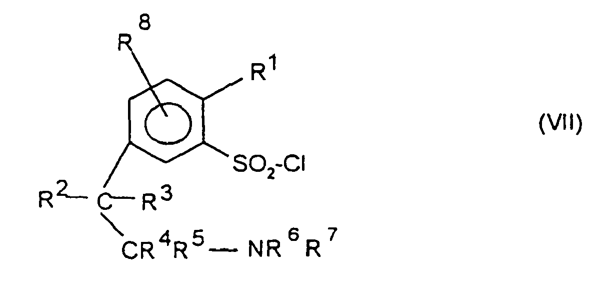 mit einem Cyanat M-OCN, worin M = ein Kation bedeutet, und mit einem Aminoheterocyclus der Formel H-NR⁹-A (V) in Gegenwart einer Base umsetzt, oder
f) ein Sulfonamid der genannten Formel (II) mit einem (Thio-)lsocyanat der Formel (V')
W = C = N - A (V')
in Gegenwart einer Base umsetzt,
wobei in den Formeln (II)-(VII) und (V') die Reste bzw. Symbole R¹-R⁹, A, W und n wie in Formel (I) gemäß Anspruch 1 definiert sind und wobei in den Varianten a) und c)-e) zunächst Verbindungen der Formel (I) mit W = O erhalten werden.

5. Herbizides oder pflanzenwachstumsregulierendes Mittel, **dadurch gekennzeichnet, daß** es ein oder mehrere Verbindungen der Formel (I) oder deren Salze nach einem der Ansprüche 1 bis 3 und im Pflanzenschutz übliche Formulierungshilfsmittel enthält.

6. Verfahren zur Bekämpfung von Schadpflanzen oder zur Wachstumsregulierung von Pflanzen, **dadurch gekennzeichnet, daß** man eine wirksame Menge von einer oder mehreren Verbindungen der Formel (I) oder deren Salzen nach einem der Ansprüche 1 bis 3 auf die Pflanzen, Pflanzensamen oder die Anbaufläche appliziert.

7. Verwendung von Verbindungen der Formel (I) oder deren Salzen nach einem der Ansprüche 1 bis 3 als Herbizide und Pflanzenwachstumsregulatoren.

8. Verbindungen der Formeln (II) und (IV), (VI) und (VII), wie sie in Anspruch 4 definiert sind.

## Claims

1. A compound of the formula (I) or a salt thereof 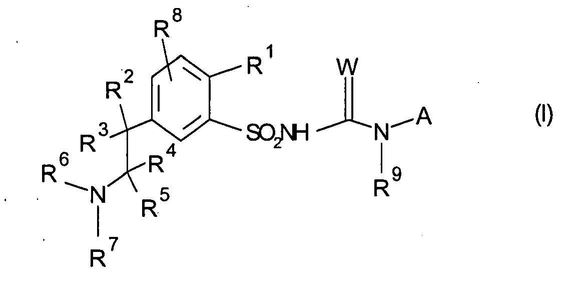 in which
R¹ is S(O)ₙ-R¹⁰ or COQR¹¹,
R², R³, R⁴, R⁵ independently of one another are H or (1-4)alkyl,
R⁶ is H, OH, formyl, (1-6)alkyl, (2-6)alkenyl, (2-4)alkynyl, (1-6)alkoxy, (2-6)alkenyloxy, (2-6)alkynyloxy, [(1-6)alkyl]carbonyl, [(2-6)alkenyl]-carbonyl, [(2-6)alkynyl]carbonyl, (1-4)alkylsulfonyl, (2-6)alkenylsulfonyl, (2-6)alkynylsulfonyl, (3-6)cycloalkyl, (5-6)cycloalkenyl, [(3-6)cycloalkyl]-carbonyl, [(5-6)cycloalkenyl]carbonyl, [(3-6)cycloalkyl]sulfonyl, [(5-6)cycloalkenyl]sulfonyl, each of the 18 last-mentioned radicals being unsubstituted or substituted by one or more radicals selected from the group consisting of halogen, (1-4)alkoxy, (1-4)alkylthio, (1-4)alkylsulfinyl, (1-4)alkylsulfonyl, [(1-4)alkoxy]carbonyl, [(1-4)alkyl]carbonyl, [(1-4)alkyl]carbonyloxy and CN and, in the case of cyclic radicals, also by (1-4)alkyl and (1-4)haloalkyl,
or
phenylcarbonyl or phenylsulfonyl, each of the two last-mentioned radicals being unsubstituted or substituted in the phenyl ring by one or more radicals selected from the group consisting of halogen, CN, NO₂, (1-4)alkyl, (1-4)haloalkyl, (1-4)alkoxy and (1-4)haloalkoxy,
R⁷ is CHO, [(1-6)alkyl]carbonyl, [(2-6)alkenyl]carbonyl, [(2-6)alkynyl]-carbonyl, (1-6)alkylsulfonyl, (2-6)alkenylsulfonyl, (2-6)alkynylsulfonyl, [(3-6)cycloalkyl] carbonyl, [(5-6)cycloalkenyl] carbonyl, [(3-6)cycloalkyl]sulfonyl, (5-6)cycloalkenylsulfonyl, each of the 10 last-mentioned radicals being unsubstituted or substituted by one or more radicals selected from the group consisting of halogen, (1-4)alkoxy, (1-4)alkylthio, (1-4)alkylsulfonyl, (1-4)alkylsulfinyl, (1-4)alkylcarbonyl, [(1-4)alkoxy]-carbonyl, [(1-4)alkyl]carbonyloxy and CN and, in the case of cyclic radicals, also by (1-4)alkyl and (1-4)haloalkyl,
or
phenylcarbonyl or phenylsulfonyl, each of the two last-mentioned radicals being unsubstituted or substituted in the phenyl ring by one or more radicals selected from the group consisting of halogen, CN, NO₂, (1-4)alkyl, (1-4)haloalkyl, (1-4)alkoxy and (1-4)haloalkoxy,
or
mono- or di[(1-4)alkyl]aminosulfonyl which is unsubstituted or substituted in the alkyl moiety by one or more radicals selected from the group consisting of halogen, (1-4)alkoxy, (1-4)alkylthio, (1-4)alkylsulfinyl, (1-4)alkylsulfonyl, [(1-4)alkyl]carbonyl, [(1-4)alkyl]carbonyloxy, [(1-4)alkoxy]carbonyl and CN, or a group of the formula COCOR' in which R' = H, OH, (1-4)alkoxy or (1-4)alkyl, or a group of the formula 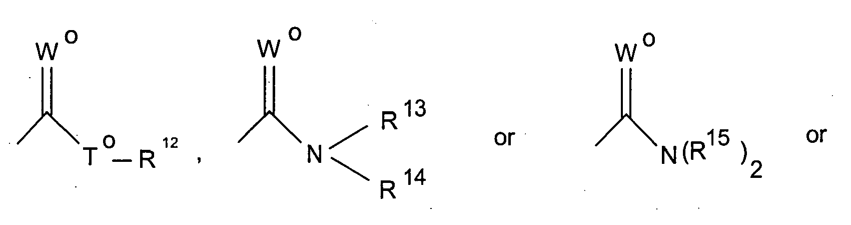
R⁶ and R⁷ together are a chain of the formula (-CH₂)ₘ₁ B¹- or -B¹-(CH₂)ₘ₂B²-,
the chain being unsubstituted or substituted by one or more (1-3)alkyl radicals or halogen and m1 is 3, 4 or 5 and m2 is 2, 3 or 4, and
W, W^{o} are an oxygen atom or a sulfur atom,
B¹,B² independently of one another are SO₂ or CO,
Q is O, S or NR¹⁶,
T^{o} is O or S,
R⁸ is H, (1-4)alkyl, (1-4)alkoxy, (1-4)alkylthio, [(1-4)alkyl]carbonyl or [(1-4)alkoxy]carbonyl, each of the last-mentioned 5 radicals being unsubstituted or substituted in the alkyl moiety by one or more halogen atoms, or is halogen, NO₂, CN or mono- or di(1-4)alkylamino,
R⁹ is H or CH₃,
R¹⁰ is NR¹⁷R¹⁸, (1-6)alkyl, (2-6)alkenyl, (2-6)alkynyl, (3-6)cycloalkyl, (5-6)cycloalkenyl or phenyl, each of the last-mentioned 6 radicals being unsubstituted or substituted by one or more radicals selected from the group consisting of halogen, CN, (1-4)alkoxy, (1-4)alkylthio, (1-4)alkylsulfinyl, (1-4)alkylsulfonyl, [(1-4)alkyl]carbonyl, [(1-4)alkoxy]carbonyl and [(1-4)alkyl]carbonyloxy,
n is the number 0, 1 or 2, unless R¹⁰ = NR¹⁷R¹⁸, in which case n = 2, and
R¹¹ is H, (1-6)alkyl, (2-6)alkenyl, (2-6)alkynyl, (3-6)cycloalkyl, (5-6)cycloalkenyl or phenyl, each of the last-mentioned 6 radicals being unsubstituted or substituted by one or more radicals selected from the group consisting of halogen, CN, (1-4)alkoxy, (1-4)alkylthio, (1-4)alkylsulfinyl, (1-4)alkylsulfonyl, [(1-4)alkyl]carbonyl, [(1-4)alkoxy]carbonyl and [(1-4)alkyl]carbonyloxy, and, in the case of cyclic radicals, also by (1-4)alkyl and (1-4)haloalkyl, or is a radical of the heterocyclyl or heterocyclyl(1-4)alkyl type which has 3-7 ring atoms and 1 to 3 hetero ring atoms selected from the group consisting of N, O and S,
R¹² is (1-4)alkyl, (3-4)alkenyl or (3-4)alkynyl, each of the three last-mentioned radicals being unsubstituted or substituted by one or more radicals selected from the group consisting of halogen, (1-4)alkoxy, (1-4)alkylthio, [(1-4)alkyl]carbonyl and [(1-4)alkoxy]carbonyl,
R¹³, R¹⁴ independently of one another are H, (1-4)alkyl, (3-4)alkenyl oder (3-4)alkynyl, each of the three last-mentioned radicals being unsubstituted or substituted by one or more of the radicals selected from the group consisting of halogen, (1-4)alkoxy, (1-4)alkylthio, [(1-4)alkyl]carbonyl and [(1-4)alkoxy]carbonyl,
the radicals R¹⁵ together with the nitrogen atom are a heterocyclic ring which has 5 or 6 ring members, may contain further hetero atoms selected from the group consisting of N, O and S at the oxidation levels which are possible and which is unsubstituted or substituted by (1-4)alkyl or the oxo group, or which is benzo-fused,
R¹⁶ is H, (1-4)alkyl, (3-4)alkenyl or (3-4)alkynyl, each of the three last-mentioned radicals being unsubstituted or substituted by one or more radicals selected from the group consisting of halogen, (1-4)alkoxy and (1-4)alkylthio,
R¹⁷ is H, (1-4)alkyl or (1-4)alkoxy, and
R¹⁸ is H or (1-4)alkyl,
A is a radical of the formula 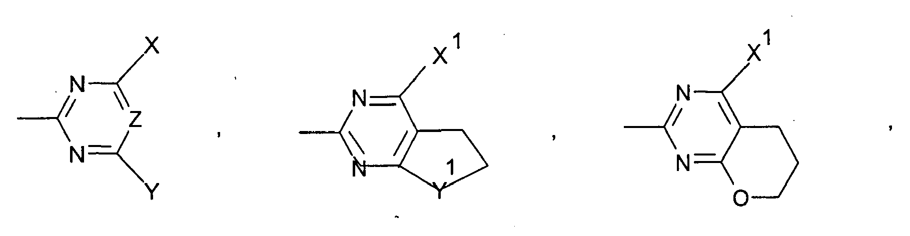 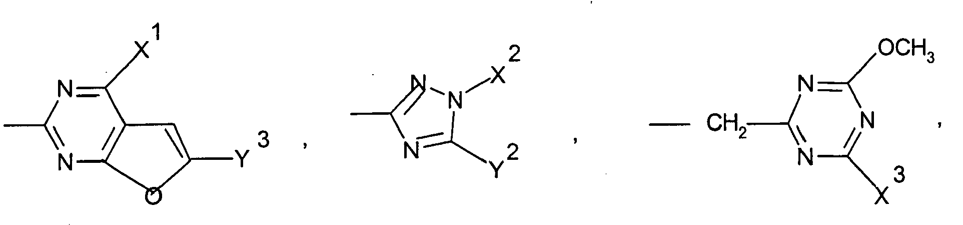 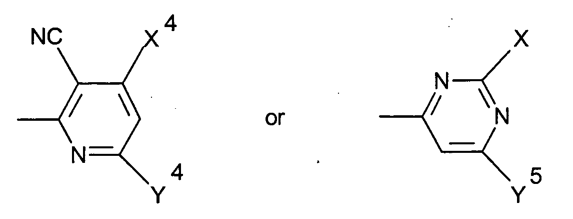
one of the radicals × and Y is hydrogen, halogen, (1-3)alkyl or (1-3)alkoxy, each of the two last-mentioned radicals being unsubstituted or substituted by one or more radicals selected from the group consisting of halogen, (1-3)alkoxy and (1-3)alkylthio, and
the other of the radicals X and Y is hydrogen, halogen, (1-3)alkyl, (1-3)alkoxy or (1-3)alkylthio, each of the three last-mentioned alkyl-containing radicals being unsubstituted or substituted by one or more radicals selected from the group consisting of halogen, (1-3)alkoxy and (1-3)alkylthio, or is a radical of the formula NR^{a}R^{b}, (3-6)cycloalkyl, (2-4)alkenyl, (2-4)alkynyl, (3-4)alkenyloxy or (3-4)alkynyloxy,
Z is CH or N,
R^{a} and R^{b} independently of one another are H, (1-4)alkyl or (2-4)alkenyl,
X¹ is CH₃, OCH₃, OC₂H₅ or OCHF₂,
Y¹ is -O-or-CH₂-,
X² is CH₃, C₂H₅ or CH₂CF₃,
Y² is OCH₃, OC₂H₅, SCH₃, SCH₂CH₃, CH₃ or C₂H₅,
X³ is CH₃ or OCH₃,
Y³ is H or CH₃,
X⁴ is CH₃, OCH₃, OC₂H₅, CH₂OCH₃ or Cl,
Y⁴ is CH₃, OCH₃, OC₂H₅ or Cl and
Y⁵ is CH₃, C₂H₅, OCH₃ or Cl.

2. A compound or salt thereof as claimed in claim 1, wherein
R¹ is S(O)ₙ-R¹⁰ or CO-OR¹¹,
n is the number 0, 1 or 2, with the exception of the case R¹⁰ = NR¹⁷R¹⁸, in which case n = 2,
R⁶ is H or (1-4)alkyl which is unsubstituted or substituted by one or more halogen atoms or by one or more radicals selected from the group (1-4)alkoxy and (1-4)alkylthio,
R⁷ is formyl, [(1-6)alkyl]carbonyl, [(2-4)alkenyl]carbonyl, [(2-4)alkynyl]-carbonyl, [(3-6)cycloalkyl]carbonyl or (1-6)alkylsulfonyl, each of the 5 last-mentioned radicals being unsubstituted or substituted by one or more radicals selected from the group consisting of halogen, (1-4)alkoxy, (1-4)alkylthio, (1-4)alkylsulfonyl, [(1-4)alkyl]carbonyl, [(1-4)alkoxy]carbonyl, [(1-4)alkyl]carbonyloxy and CN and, in the case of cyclic radicals, also (1-4)alkyl and (1-4)haloalkyl, or phenylcarbonyl or phenylsulfonyl, each of the two last-mentioned radicals being unsubstituted or substituted by one or more radicals selected from the group consisting of halogen, CN, SO₂, (1-4)alkyl, (1-4)haloalkyl, (1-4)alkoxy and (1-4)haloalkoxy, or
mono- or di[(1-4)-alkyl]aminosulfonyl, or a group of the formula -CO-CO-R' in which R' is (1-4)alkoxy, or
a group of the formula -CW^{o}-R¹², -CW^{o}-NR¹³R¹⁴ or -CW^{o}-N(R¹⁵)₂ or
R⁶, R⁷ together are a chain of the formula (-CH₂)ₘ₁B¹- or -B¹-(CH₂)ₘ₂B²-, m1 being 3, 4 or 5 and m2 being 2, 3 or 4, and
W, W^{o} in each case independently are an oxygen or sulfur atom,
T^{o} is an oxygen or sulfur atom,
B¹ is SO₂ or CO,
B² is SO₂ or CO,
Q is O, S or NR¹⁶,
R⁸ is a hydrogen atom,
R⁹ is H or CH₃,
R¹⁰ is NR¹⁷R¹⁸, (1-6)alkyl or (3-6)cycloalkyl,
R¹¹ is H, (1-6)alkyl, (3-6)cycloalkyl or a radical of the formulae A-1 to A-6, 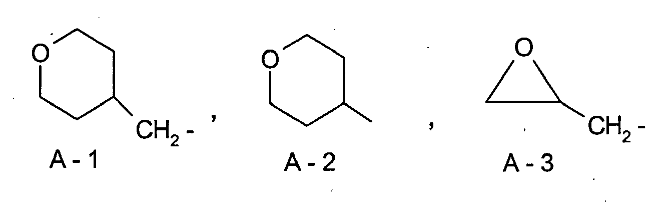 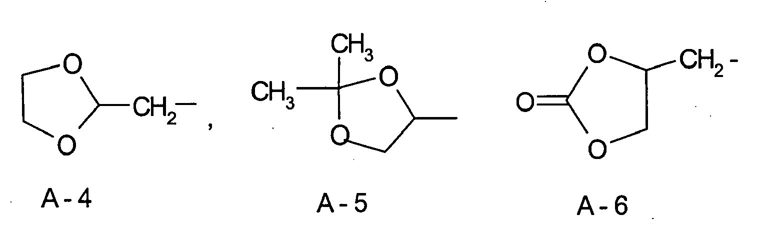
R¹² is (1-4)alkyl or (1-4)haloalkyl,
R¹³, R¹⁴ independently of one another are H or (1-4)alkyl, the radicals R¹⁵ together are a divalent chain of the formula -(CH₂)ₘ₃- in which
m3 is 3, 4 or 5, or of the formula -CH₂CH₂-O-CH₂CH₂-,
R¹⁶ is H or (1-4)alkyl,
R¹⁷ is H or (1-4)alkyl and
R¹⁸ is H or (1-4)alkyl.

3. A compound or salt thereof as claimed in either of claims 1 or 2, wherein
R⁶ is H or (1-4)alkyl,
R⁷ is CHO, [(1-6)alkyl]carbonyl, [(1-4)haloalkyl]carbonyl, [(1-4)alkoxy-(1-4)alkyl]carbonyl, [(3-6)cycloalkyl]carbonyl, phenylcarbonyl which is unsubstituted or substituted by one or more radicals selected from the group consisting of halogen, CN, NO₂, (1-4)alkyl, (1-4)haloalkyl, (1-4)alkoxy and (1-4)haloalkoxy, or is phenylsulfonyl which is unsubstituted or substituted by one or more radicals selected from the group consisting of (1-4)alkyl and (1-4)alkoxy, or is mono- or di[(1-4)alkyl]aminosulfonyl, (1-6)alkylsulfonyl, (1-4)haloalkylsulfonyl or a group of the formula
-CW^{o}-T-R¹² or -CW^{o}-NR¹³R¹⁴
W, W^{o} independently of one another are in each case O or S,
T^{o} is O or S,
Q is O, S or NR¹⁶,
R¹⁰ is NR¹⁷R¹⁸, (1-4)alkyl or (3-6)cycloalkyl,
R¹¹ is H or (1-4)alkyl,
R¹² is (1-4)alkyl,
R¹³, R¹⁴ independently of one another are H or (1-4)alkyl,
R¹⁶ is H or (1-3)alkyl,
R¹⁷ is (1-4)alkyl and
R¹⁸ is H or (1-4)alkyl.

4. A process for the preparation of a compound of the formula (I) or a salt thereof as defined in any of claims 1 to 3, which comprises
a) reacting a compound of the formula (II) 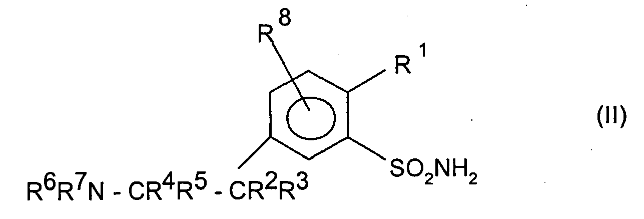 with a heterocyclic carbamate of the formula (III)
R*-O-CO-NR⁹-A (III)
in which R* is optionally substituted phenyl or (C₁-C₄)alkyl, or
b) reacting an arylsulfonylcarbamate of the formula (IV) 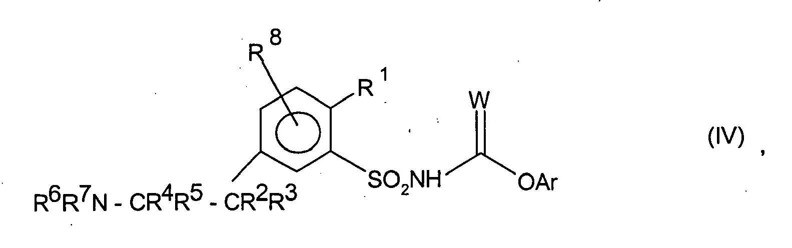 in which Ar is an aryl radical, with an amino heterocycle of the formula (V)
H - NR⁹ - A (V),
or
c) reacting a sulfonyl isocyanate of the formula (VI) 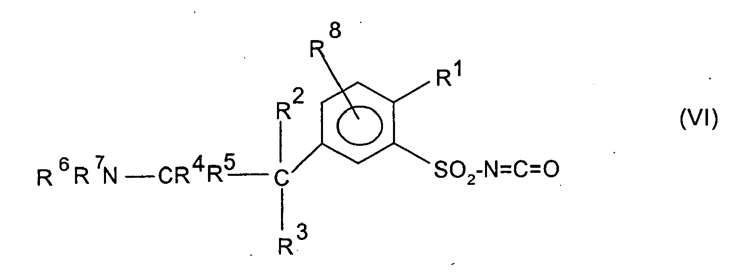 with an amino heterocycle of the formula H-NR⁹-A (V), or
d) first reacting, in a one-pot reaction, an amino heterocycle of the formula
H-NR⁹-A (V) with phosgene in the presence of a base and reacting the intermediate formed with a phenylsulfonamide of the formula (II), or
e) reacting a sulfonyl chloride of the formula (VII) 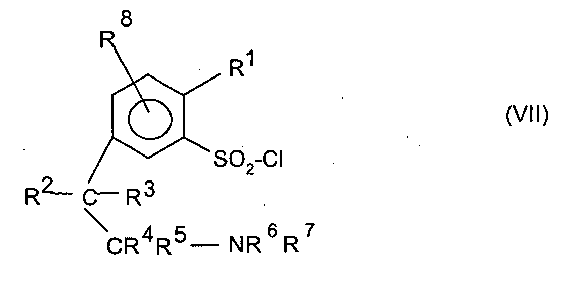 with a cyanate M-OCN in which M = a cation and with an amino heterocycle of the formula H-NR⁹-A (V) in the presence of a base, or
f) reacting a sulfonamide of the abovementioned formula (II) with a (thio)isocyanate of the formula (V')
W=C=N-A (V')
in the presence of a base,
where, in formulae (II)-(VII) and (V'), the radicals or symbols R¹-R⁹, A, W and n are as defined in formula (I) as claimed in claim 1 and where, in variants a) and c)-e), the initial products are compounds of the formula (I) where W = O.

5. A herbicidal or plant-growth-regulating composition which comprises one or more compounds of the formula (I) or salts thereof as claimed in any of claims 1 to 3 and formulation auxiliaries conventionally used in crop protection.

6. A method of controlling harmful plants or for regulating the growth of plants, which comprises applying an effective amount of one or more compounds of the formula (I) or salts thereof as claimed in any of claims 1 to 3 to the plants, seeds of the plants or the area under cultivation.

7. The use of a compound of the formula (I) or a salt thereof as claimed in any of claims 1 to 3 as a herbicide and plant growth regulator.

8. Compounds of the formulae (II) and (IV), (VI) and (VII), as they are defined in claim 4.

## Revendications

1. Composés, de formule (I) ou leurs sels, 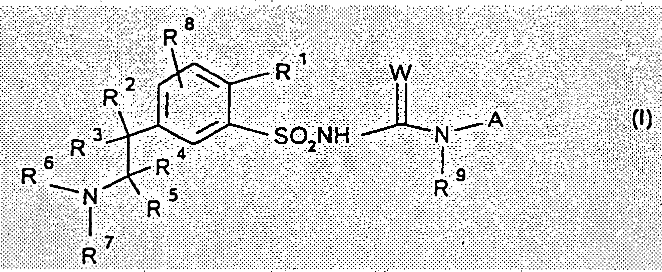 dans laquelle
R¹ représente S(O)ₙ-R¹⁰ ou COQR¹¹,
R², R³, R⁴, R⁵ représentent, indépendamment les uns des autres, un atome d'hydrogène ou un groupe alkyle (en C₁ à C₄),
R6 représente un atome d'hydrogène, un groupe OH, formyle, alkyle (en C₁ à C₆), alcényle (en C₂ à C₆), alcynyle (en C₂ à C₄), alcoxy (en C₁ à C₆), alcényle (en C₂ à C₆)-oxy, alcynyle (en C₂ à C₆)-oxy, [alkyle (en C₁ à C₆)]-carbonyle, [alcényle (en C₂ à C₆)]-carbonyle, [alcynyle (en C₂ à C₆)]-carbonyle, alkyle(en C₁ à C₄)-sulfonyle, alcényle(en C₂ à C₆)sulfonyle, alcynyle (en C₂ à C₆)sulfonyle, cycloalkyle(en C₃ à C₆), cycloalcényle (en C₅ à C₆), [cycloalkyle(en C₃ à C₆)]-carbonyle, [cycloalcényle(en C₅ à C₆)]-carbonyle, [cycloalkyle(en C₃ à C₆)]-sulfonyle, [cycloalcényle (en C₅ à C₆)]-sulfonyle, où chacun des 18 derniers groupes mentionnés est non substitué ou substitué par un ou plusieurs groupes choisis dans le groupe constitué par un atome d'halogène, un groupe alcoxy(en C₁ à C₄), alkyle(en C₁ à C₄)thio, alkyle(en C₁ à C₄)sulfinyle, alkyle(en C₁ à C₄)sulfonyle, [alcoxy(en C₁ à C₄)]carbonyle, [alkyle(en C₁ à C₄)]carbonyle, [alkyle(en C₁ à C₄)]carbonyloxy et CN et, dans le cas de groupes cycliques, également par un groupe alkyle(en C₁ à C₄) et halogénoalkyle(en C₁ à C₄),
ou
phénylcarbonyle ou phénylsulfonyle, où chacun des deux derniers groupes mentionnés est, dans le noyau phényle, non substitué ou substitué par un ou plusieurs groupes choisis dans le groupe constitué par un atome d'halogène, un groupe CN, NO₂, alkyle(en C₁ à C₄), halogénoalkyle(en C₁ à C₄), alcoxy(en C₁ à C₄) et halogénoalcoxy(en C₁ à C₄),
R⁷ représente un groupe CHO, [alkyle(en C₁ à C₆)]-carbonyle, [alcényle(en C₂ à C₆)]-carbonyle, [alcynyle(en C₂ à C₆)]-carbonyle, alkyle(en C₁ à C₆)sulfonyle, alcényle(en C₂ à C₆)sulfonyle, alcynyle (en C₂ à C₆) sulfonyle, [cycloalkyle (en C₃ à C₆)]-carbonyle, [cycloalcényle(en C₅ à C₆)]-carbonyle, [cycloalkyle(en C₃ à C₆)]sulfonyle, cycloalcényle(en C₅ à C₆)sulfonyle, où chacun des 10 derniers groupes mentionnés est non substitué ou substitué par un ou plusieurs groupes choisis dans le groupe constitué par un atome d'halogène, un groupe alcoxy(en C₁ à C₄), alkyle(en C₁ à C₄)thio, alkyle (en C₁ à C₄)sulfonyle, alkyle (en C₁ à C₄)sulfinyle, alkyle(en C₁ à C₄)carbonyle, [alcoxy(en C₁ à C₄)]-carbonyle, [alkyle(en C₁ à C₄)]-carbonyloxy et CN et, dans le cas de groupes cycliques, également, par un groupe alkyle(en C₁ à C₄) et halogénoalkyle(en C₁ à C₄),
ou
phénylcarbonyle ou phénylsulfonyle, où chacun des deux derniers groupes mentionnés est, dans le noyau phényle, non substitué ou substitué par un ou plusieurs groupes choisis dans le groupe constitué par un atome d'halogène, un groupe CN, NO₂, alkyle (en C₁ à C₄), halogénoalkyle (en C₁ à C₄), alcoxy (en C₁ à C₄) et halogénoalcoxy (en C₁ à C₄),
ou
mono- ou di-[alkyle (en C₁ à C₄)]aminosulfonyle qui, dans la fraction alkyle, est non substitué ou substitué par un ou plusieurs groupes choisis dans le groupe constitué par un atome d'halogène, un groupe alcoxy (en C₁ à C₄), alkyle(en C₁ à C₄)thio, alkyle (en C₁ à C₄)sulfinyle, alkyle (en C₁ à C₄) sulfonyle, [alkyle(en C₁ à C₄)]-carbonyle, [alkyle(en C₁ à C₄)]-carbonyloxy, [alcoxy(en C₁ à C₄)]-carbonyle et CN, ou un groupe de formule COCOR', dans laquelle
R' représente un atome d'hydrogène, un groupe OH, alcoxy(en C₁ à C₄) ou alkyle(en C₁ à C₄), ou un groupe de formule 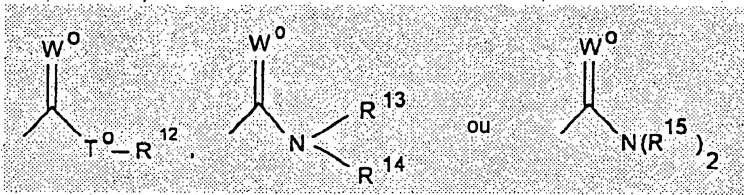 ou
R⁶ et R⁷ forment ensemble une chaîne de.formule (-CH₂)ₘ₁ B¹- ou -B¹-(CH₂)ₘ₂B²-, ou la chaîne est non substituée ou substituée par un ou plusieurs groupes alkyle (en C₁ à C₃) ou par un atome d'halogène, et m1 = 3, 4 ou 5 ou m2= 2, 3 ou 4, et
W, W^{o} représentent un atome d'oxygène ou un atome de soufre,
B¹, B² représentent, indépendamment l'un, de l'autre, un groupe SO₂ ou CO,
Q représente un atome d'oxygène, de soufre ou un groupe NR¹⁶,
T^{o} représente un atome d'oxygène ou de soufre,
R⁸ représente un atome d'hydrogène, un groupe alkyle(en C₁ à C₄), alcoxy(en C₁ à C₄), alkyle(en C₁ à C₄)thio, [alkyle(en C₁ à C₄)]carbonyle ou [alcoxy(en C₁ à C₄)]carbonyle, où chacun des 5 derniers groupes mentionnés est non substitué ou substitué dans la fraction alkyle par un ou plusieurs atomes d'halogène, ou un atome d'halogène, un groupe NO₂, CN ou mono- ou di-alkyle(en C₁ à C₄)amino,
R⁹ représente un atome d'hydrogène ou un groupe CH₃,
R¹⁰ représente un groupe NR¹⁷R¹⁸, alkyle(en C₁ à C₆), alcényle(en C₂ à C₆), alcynyle(en C₂ à C₆), cycloalkyle(en C₃ à C₆), cycloalcényle(en C₅ à C₆) ou phényle, où chacun des 6 derniers groupes mentionnés est non substitué ou substitué par un ou plusieurs groupes choisis dans le groupe constitué par un atome d'halogène, un groupe CN, alcoxy(en C₁ à C₄), alkyle(en C₁ à C₄)thio, alkyle(en C₁ à C₄)sulfinyle, alkyle(en C₁ à C₄) sulfonyle, [alkyle(en C₁ à C₄)]-carbonyle, [alcoxy(en C₁ à C₄)]-carbonyle et [alkyle(en C₁ à C₄)]-carbonyloxy,
n vaut 0, 1 ou 2, sauf si R¹⁰ = NR¹⁷R¹⁸, auquel cas n = 2, et
R¹¹ représente un atome d'hydrogène, un groupe alkyle(en C₁ à C₆), alcényle (en C₂ à C₆), alcynyle (en C₂ à C₆), cycloalkyle (en C₃ à C₆), cycloalcényle (en C₅ à C₆) ou phényle, où chacun des 6 derniers groupes mentionnés est non substitué ou substitué par un ou plusieurs groupes choisis dans le groupe constitué par un atome d'halogène, un groupe CN, alcoxy(en C₁ à C₄), alkyle (en C₁ à C₄)thio, alkyle (en C₁ à C₄)sulfinyle, alkyle (en C₁ à C₄) sulfonyle, [alkyle (en C₁ à C₄)] carbonyle, [alcoxy(en C₁ à C₄)] carbonyle et [alkyle (en C₁ à C₄)]carbonyloxy et, dans le cas de groupes cycliques, également par un groupe alkyle(en C₁ à C₄) et halogénoalkyle(en C₁ à C₄), ou un groupe du type hétérocyclyle, ou hétérocyclyl[alkyle (en C₁ à C₄)] avec 3 à 7, atomes cycliques et 1 à 3 hétéroatomes cycliques du groupe constitué par un atome d'azote, d'oxygène et de soufre,
R¹² représente un groupe alkyle(en C₁ à C₄), alcényle(en C₃ à C₄) ou alcynyle (en C₃ à C₄), où chacun des trois derniers groupes mentionnés est non substitué ou substitué par un ou plusieurs groupes choisis dans le groupe constitué par un atome d'halogène, un groupe alcoxy (en C₁ à C₄), alkyle(en C₁ à C₄)thio, [alkyle(en C₁ à C₄)]-carbonyle et [alcoxy (en C₁ à C₄)]-carbonyle,
R¹³, R¹⁴ représentent, indépendamment l'un de l'autre, un atome d'hydrogène, un groupe alkyle (en C₁ à C₄), alcényle (en C₃ à C₄) ou alcynyle (en C₃ à C₄), où chacun des trois derniers groupes mentionnés est non substitué ou substitué par un ou plusieurs des groupes choisis dans le groupe constitué par un atome d'halogène ; un groupe alcoxy (en C₁ à C₄), alkyle(en C₁ à C₄)thio, [alkyle(en C₁ à C₄)]-carbonyle et [alcoxy(en C₁ à C₄)]-carbonyle,
les groupes R¹⁵ représentent, conjointement avec l'atome d'azote, un groupe hétérocyclique de 5 ou 6 éléments, qui peut contenir d'autres hétéroatomes du groupe constitué par un atome d'azote, un atome d'oxygène et un atome de soufre aux états d'oxydation possibles, et qui est non substitué ou substitué par un groupe alkyle (en C₁ à C₄) ou le groupe oxo, ou benzocondensé,
R¹⁶ représente un atome d'hydrogène, un groupe alkyle(en C₁ à C₄), alcényle (en C₃ à C₄) ou alcynyle (en C₃ à C₄), où chacun des trois derniers groupes mentionnés est non substitué ou substitué par un ou plusieurs groupes choisis dans le groupe constitué par un atome d'halogène, un groupe alcoxy(en C₁ à C₄) et alkyle(en C₁ à C₄)thio,
R¹⁷ représente un atome d'hydrogène, un groupe alkyle(en C₁ à C₄) ou alcoxy(en C₁ à C₄),
R¹⁸ représente un atome d'hydrogène ou un groupe alkyle (en C₁ à C₄),
A représente un groupe de formule 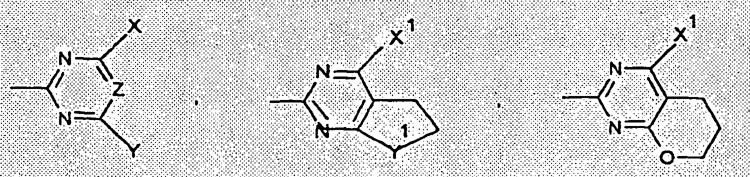 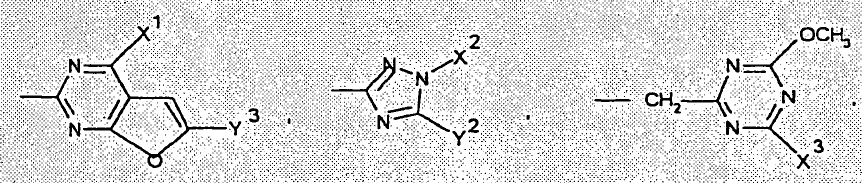 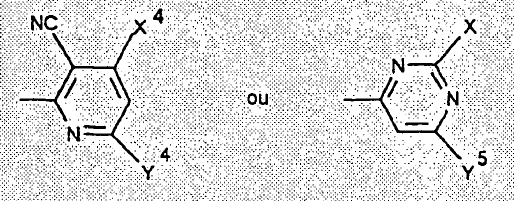
l'un des groupes X et Y représente un atome d'hydrogène, d'halogène, un groupe alkyle (en C₁ à C₃) ou alcoxy(en C₁ à C₃), où chacun des deux derniers groupes mentionnés est non substitué ou substitué par un ou plusieurs groupes choisis dans le groupe constitué par un atome d'halogène, un groupe alcoxy(en C₁ à C₃) et alkyle(en C₁ à C₃)thio, et
l'autre des groupes X et Y représente un atome d'hydrogène, d'halogène, un groupe alkyle(én C₁ à C₃), alcoxy(en C₁ à C₃) ou alkyle(en C₁ à C₃)thio, où chacun des trois derniers groupes contenant une fraction alkyle mentionnés est non substitué ou substitué par un ou plusieurs groupes choisis dans le groupé constitué par un atome d'halogène, un groupe alcoxy (en C₁ à C₃) et alkyle (en C₁ à C₃)thio, ou un groupe de formule NR^{a}R^{b} cycloalkyle(en C₃ à C₆), alcényle(en C₂ à C₄), alcynyle(en. C₂ à C₄), alcényle(en C₃ à C₄)oxy ou alcynyle (en C₃ à C₄)oxy,
Z représente CH ou un atome d'azote,
R^{a} et R^{b} représentent, indépendamment l'un de l'autre, un atome d'hydrogène, un groupe alkyle(en C₁ à C₄) ou alcényle(en C₂ à C₄),
X¹ représente un groupe CH₃, OCH₃, OC₂H₅ ou OCHF₂,
Y¹ représente un groupe -O- ou -CH₂-,
X² représente un groupe CH₃, C₂H₅ ou CH₂CF₃,
Y² représente un groupe OCH₃, OC₂H₅, SCH₃, SCH₂CH₃, CH₃ ou C₂H₅,
X³ représente un groupe CH₃ ou OCH₃,
Y³ représente un atome d'hydrogène ou un groupe CH₃,
X⁴ représente un groupe CH₃, OCH₃, OC₂H₅, CH₂OCH₃ ou un atome de chlore,
Y⁴ représente un groupe CH₃, OCH₃, OC₂H₅ ou un atome de chlore et
Y⁵ représente un groupe CH₃, C₂H₅, OCH₃ ou un atome de chlore.

2. Composés ou leurs sels selon la revendication 1, **caractérisés en ce que**
R¹ représente S(O)ₙ⁻R¹⁰ ou CO-OR¹¹,
n vaut 0, 1 ou 2, sauf si R¹⁰ = NR¹⁷R¹⁸, auquel cas n = 2,
R⁶ représente un atome d'hydrogène ou un groupe alkyle(en C₁ à C₄), qui est non substitué ou substitué par un ou plusieurs atomes d'halogène ou par un ou plusieurs groupes choisis dans le groupe constitué par un groupe alcoxy(en C₁ à C₄) et alkyle (en C₁ à C₄)thio,
R7 représente un groupe formyle, [alkyle (en C₁ à C₆)]-carbonyle, [alcényle (en C₂ à C₄)]-carbonyle, [alcynyle (en C₂ à C₄)]-carbonyle, [cycloalkyle(en C₃ à C₆)]-carbonyle ou alkyle(en C₁ à C₆)sulfonyle, ou chacun des cinq derniers groupes mentionnés est non substitué ou substitué par un ou plusieurs groupes choisis dans le groupe constitué par un atome d'halogène, un groupe alcoxy(en C₁ à C₄), alkyle(en C₁ à C₄)thio, alkyle(en C₁ à C₄)sulfonyle, [alkyle(en C₁ à C₄)]-carbonyle, [alcoxy (en C₁ à C₄)]-carbonyle, [alkyle(en C₁ à C₄)]-carbonyloxy et CN et, dans le cas de groupes cycliques, également par un groupe alkyle (en C₁ à C₄) et halogénoalkyle(en C₁ à C₄), ou
phénylcarbonyle ou phénylsulfonyle, où chacun des deux derniers groupes mentionnés est non substitué ou substitué par un ou plusieurs groupes choisis dans le groupe constitué par un atome d'halogène, un groupe CN, SO₂, alkyle(en C₁ à C₄), halogénoalkyle(en C₁ à C₄), alcoxy(en C₁ à C₄) et halogénoalcoxy(en C₁ à C₄), ou
mono- ou di-[alkyle(en C₁ à C₄)]-aminosulfonyle, ou un groupe de formule -CO-CO-R', dans laquelle R représente un groupe alcoxy(en C₁ à C₄), ou
un groupe de formule -CW^{o}-R¹², =CW^{o}-NR¹³R¹⁴ ou -CW^{o}-N(R¹⁵)₂ ou
R⁶, R⁷ forment ensemble une chaîne de formule (-CH₂)ₘ₁B¹- ou -B¹-(CH₂)ₘ₂B2-, où m1 = 3, 4 ou 5 et m2 = 2, 3 ou 4, et
W, W^{o} représentent chacun, indépendamment l'un de l'autre, un atome d'oxygène ou un atome de soufre,
T^{o} représente un atome d'oxygène ou un atome de soufre,
B¹ représente un groupe SO₂ ou CO,
B² représente un groupe SO₂ ou CO,
Q représente un atome d'oxygène, de soufre ou un groupe NR¹⁶,
R⁸ représente un atome d'hydrogène,
R⁹ représente un atome d'hydrogène ou un groupe CH₃,
R¹⁰ représente un groupe NR¹⁷R¹⁸, alkyle(en C₁ à C₆) ou cycloalkyle(en C₃ à C₆),
R¹¹, représente un atome d'hydrogène, un groupe alkyle(en C₁ à C₆), cycloalkyle(en C₃ à C₆) ou un groupe de formules A-1 à A-6, 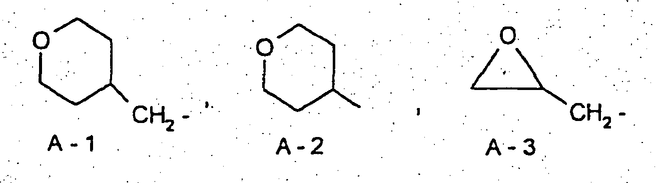 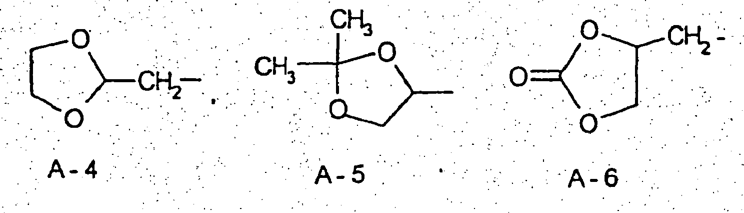
R¹² représente un groupe alkyle (en C₁ à C₄) ou halogénoalkyle (en C₁ à C₄),
R¹³, R¹⁴ représentent, indépendamment l'un de l'autre, un atome d'hydrogène ou un groupe alkyle(en C₁ à C₄),
les groupes R¹⁵ forment ensemble une chaîne divalente de formule -(CH₂)m3-, où m3 = 3, 4 ou 5, ou de formule-CH₂CH₂-O-CH₂CH2-,
R¹⁶ représente un atome d'hydrogène ou un groupe alkyle(en C₁ à C₄),
R¹⁷ représente un atome d'hydrogène ou un groupe alkyle(en C₁ à C₄), et
R¹⁸ représente un atome d'hydrogène ou un groupe alkyle (en C₁ à C₄).

3. Composés ou leurs sels selon l'une quelconque des revendications 1 ou 2, **caractérisés en ce que**
R⁶ représente un atome d'hydrogène ou un groupe alkyle (en C₁ à C₄),
R⁷ représente un groupe CHO, [alkyle(en C₁ à C₆)]-carbonyle, [halogénoalkyle(en C₁ à C₄)]-carbonyle, [alcoxy (en C₁ à C₄) -alkyle (en C₁ à C₄)]-carbonyle, [cycloalkyle (en C₃ à C₆)]-carbonyle, phénylcarbonyle, qui est non substitué ou substitué par un ou plusieurs groupes choisis dans le groupe constitué par un atome d'halogène, un groupe CN, NO₂, alkyle (en C₁ à C₄), halogénoalkyle (en C₁ à C₄), alcoxy(en C₁ à C₄) et halogénoalcoxy(en C₁ à C₄), ou phénylsulfonyle, qui est non substitué ou substitué par un ou plusieurs groupes choisis dans le groupe constitué par un groupe alkyle(en C₁ à C₄) et alcoxy (en C₁ à C₄), ou un groupe mono- ou di-(alkyle (en C₁ à C₄))-aminosulfonyle, alkyle (en C₁ à C₆) sulfonyle, halogénoalkyle (en C₁ à C₄) sulfonyle ou un groupe de formule -CW^{o}-T-R¹² ou -CW^{o}-NR¹³R¹⁴
W, W^{o} représentent chacun, indépendamment l'un de l'autre, un atome d'oxygène ou un atome de soufre,
T^{o} représente un.atome d'oxygène ou de soufre,
Q représente un atome d'oxygène, de soufre ou un groupe NR¹⁶,
R¹⁰ représente un groupe NR¹⁷R¹⁸, alkyle (en C₁ à C₄) ou cycloalkyle(en C₃ à C₆),
R¹¹ représente un atome d'hydrogène ou un groupe alkyle(en C₁ à C₄),
R¹² représente un groupe alkyle(en C₁ à C₄),
R¹³, R¹⁴ représentent, indépendamment l'un de l'autre, un atome d'hydrogène ou un groupe alkyle(en C₁ à C₄),
R¹⁶ représente un atome d'hydrogène ou un groupe alkyle (en C₁ à C₃),
R¹⁷ représente un groupe alkyle (en C₁ à C₄), et
R¹⁸ représente un atome d'hydrogène ou un groupe alkyle (en C₁ à C₄)

4. Procédé de préparation de composés de formule (I) ou de leurs sels, tels que définis selon l'une quelconque des revendications 1 à 3, **caractérisé en ce que**
a) l'on fait réagir un composé de formule (II) 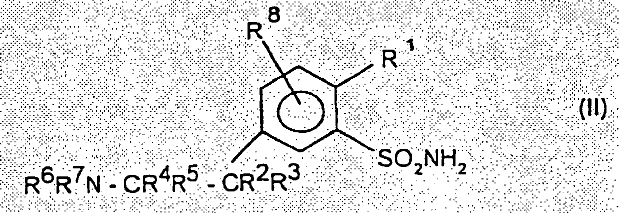 avec un carbamate hétérocyclique de formule (III),
R* -O-CO-NR⁹-A (III)
dans laquelle R* représente un groupe phényle éventuellement substitué ou un groupe alkyle (en C₁ à C₄), ou
b) l'on fait réagir un arylsulfonylcarbamate de formule (IV) 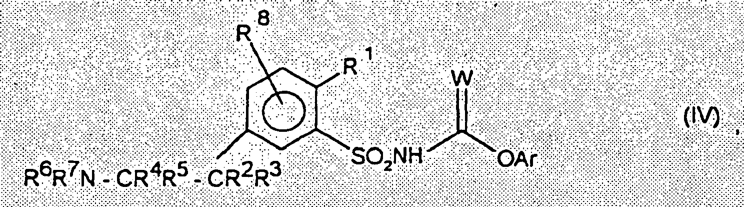 dans laquelle Ar représente un groupe aryle, avec un aminohétérocycle de formule (V)
H-NR⁹-A (V),
ou
c) l'on fait réagir un isocyanate de sulfonyle de formule (VI) 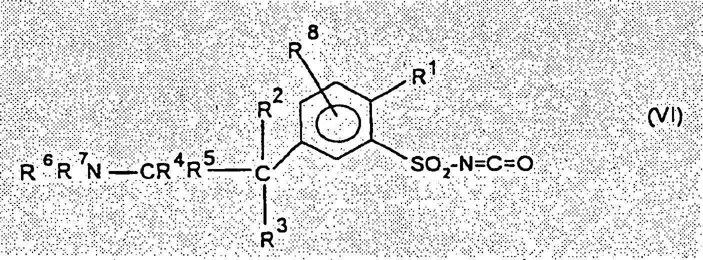 avec un aminohétérocycle de formule H-NR⁹-A (V), ou
d) l'on fait réagir, dans une réaction en récipient unique, d'abord un aminohétérocycle de formule H-NR⁹-A (V) en présence d'une base avec un phosgène, puis l'intermédiaire formé avec un phénylsulfonamide de formule (II), ou
e) l'on fait réagir un chlorure de sulfonyle de formule (VII) 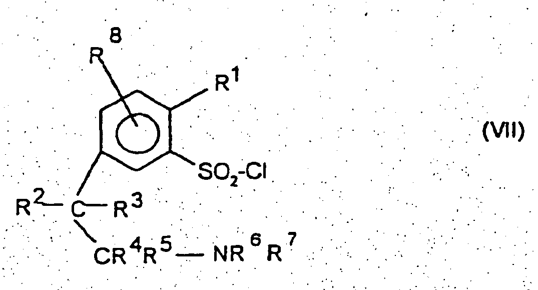 avec un cyanate M-OCN, où M représente un cation, et avec un aminohétérocycle de formule H-NR⁹-A (V) en présence d'une base, ou
f) l'on fait réagir un sulfonamide de formule (II) mentionnée avec un (thio-)isocyanate de formule (V')
W=C=N-A (V')
en présence d'une base,
où, dans les formules (II) à (VII) et (V'), les groupes ou symboles R¹ à R⁹, A, W et n sont tels que définis dans la formule (I) selon la revendication 1 et, dans les variantes a) et c) à e), on obtient d'abord des composés de formule (I) avec W = 0.

5. Agent herbicide ou régulateur de croissance des plantes, **caractérisé en ce qu'**il contient un ou plusieurs composés de formule (I) ou leurs sels selon l'une quelconque des revendications 1 à 3 et des adjuvants de formulation habituels pour la protection phytosanitaire.

6. Procédé pour lutter contre les plantes adventices ou pour réguler la croissance des plantes, **caractérisé en ce que** l'on applique une quantité efficace d'un ou plusieurs composés de formule (I) ou de leurs sels selon l'une quelconque des revendications 1 à 3 sur les plantes, les graines ou les terres cultivées.

7. Utilisation de composés de formule (I) ou de leurs sels selon l'une quelconque des revendications 1 à 3 en tant qu'herbicides et régulateurs de croissance des plantes.

8. Composés de formules (II) et (IV), (VI) et (VII) tels que définis dans la revendication (4).
